# EUROPEAN PATENT SPECIFICATION

(11) **EP 2 640 432 B1**
(45) Date of publication and mention of the grant of the patent: **07.06.2017**
(21) Application number: 11793589.0
(22) Date of filing: 17.11.2011
(51) Int. Cl.: A61L 31/02, A61L 31/18

(54) **RADIOPAQUE INTRALUMINAL STENTS COMPRISING COBALT-BASED ALLOYS CONTAINING ONE OR MORE PLATINUM GROUP METALS, REFRACTORY METALS, OR COMBINATIONS THEREOF**
RÖNTGENDICHTE INTRALUMINALE STENTS MIT LEGIERUNGEN AUF KOBALTBASIS MIT EINEM ODER MEHREREN PLATINGRUPPENMETALLEN, REFRAKTÄRMETALLEN, ODER KOMBINATIONEN DARAUS
ENDOPROTHÈSES INTRALUMINALES RADIO-OPAQUES COMPORTANT DES ALLIAGES À BASE DE COBALT CONTENANT UN OU PLUSIEURS MÉTAUX DU GROUPE DU PLATINE, DES MÉTAUX RÉFRACTAIRES OU DES COMBINAISONS DE CEUX-CI

(30) Priority: 16.11.2011 US 201113298070; 17.11.2010 US 414566 P
(43) Date of publication of application: 25.09.2013
(73) Proprietor: Abbott Cardiovascular Systems, Inc., Santa Clara, CA 95054 (US)
(72) Inventor: KRAMER-BROWN, Pamela, A., San Jose CA 95135 (US); LEACH, Austin, San Francisco CA (US); VON OEPEN, Randolf, Aptos CA 95003 (US)
(74) Representative: Peters, Hajo
(86) International application number: PCT/US2011/061161
(87) International publication number: WO 2012/068358

(56) References cited:
- EP-A1- 1 632 584
- EP-A1- 1 829 982
- US-A1- 2004 129 347

## Description

### BACKGROUND

Intraluminal stents implanted with percutaneous methods have become a standard adjunct to procedures such as balloon angioplasty in the treatment of atherosclerotic disease of the arterial system. Stents, by preventing acute vessel recoil, improve long term patient outcome and have other benefits such as securing vessel dissections.

Intraluminal stents comprise generally tubular-shaped devices which are constructed to hold open a segment of a blood vessel or other anatomical lumen. Intraluminal stents are used in treatment of diseases such as atherosclerotic stenosis as well as diseases of the stomach and esophagus, and for urinary tract applications. Adequate stent function requires a precise placement of the stent over a lesion or site of plaque or other lumen site in need of treatment. Typically, the stent is delivered to a treatment site by a delivery catheter that comprises an expandable portion for expanding the stent within the lumen.

The delivery catheter onto which the stent is mounted may be a balloon delivery catheter similar to those used for balloon angioplasty procedures. In order for the stent to remain in place on the balloon during delivery to the site of damage within a lumen, the stent may be compressed onto the balloon. The catheter and stent assembly is introduced within a patient's vasculature using a guide wire. The guide wire is disposed across the damaged arterial section and then the catheter-stent assembly is advanced over the guide wire within the artery until the stent is directly within the lesion or the damaged section.

The balloon of the catheter is expanded, expanding the stent against the artery wall. The artery is preferably slightly expanded by the expansion of the stent to seat or otherwise fix the stent to prevent movement. In some circumstances during treatment of stenotic portions of the artery, the artery may have to be expanded considerably in order to facilitate passage of blood or other fluid therethrough. In the case of a self expanding stent, the stent is expanded by retraction of a sheath or actuation of a release mechanism. Self expanding stents may expand to the vessel wall automatically without the aid of a dilation balloon, although such a dilation balloon may be used for another purpose.

These manipulations are performed within the body of a patient by a practitioner who may rely upon both placement markers on the stent catheter and on the radiopacity of the stent itself. The stent radiopacity arises from a combination of stent material and stent pattern, including stent strut or wall thickness. After deployment within the vessel, the stent radiopacity should allow adequate visibility of both the stent and the underlying vessel and/or lesion morphology under fluoroscopic visualization.

### SUMMARY

Embodiments of the present invention are directed to radiopaque stent, comprising:a cylindrical main body comprising a cobalt-based alloy including cobalt, chromium and one or more platinum group metals selected from the group consisting of platinum, palladium, rhodium, iridium, osmium, ruthenium, silver and gold the cobalt-based alloy being substantially free of nickel and comprising no more than about 20 percent by weight iron;wherein the cobalt-based alloy comprises from about 18 weight percent to about 39 weight percent cobalt, from about 10 weight percent to about 25 weight percent chromium, from about 40 weight percent to about 65 weight percent platinum, and wherein the alloy is substantially free of molybdenum. The expression "nickel free" implies e.g. that the alloy includes no more than about 2% nickel by weight. In other embodiments, the amount of iron may be further limited (e.g., no more than about 10% by weight, no more than about 8% by weight). In some embodiments, the alloys are substantially iron free (*e.g.,* no more than about 4% iron by weight). In another embodiment, iron is entirely absent.

In an embodiment, the cobalt-based alloy is entirely free of nickel and comprising no more than about 20 percent by weight iron.

In an embodiment, the cobalt-based alloy being substantially free of nickel and comprising no more than about 16 percent by weight iron.

In an embodiment, the cobalt-based alloy being substantially free of nickel and comprising no more than about 10 percent by weight iron.

In an embodiment, the cobalt-based alloy being substantially free of nickel and comprising no more than about 8 percent by weight iron.

In an embodiment, the cobalt-based alloy being substantially free of nickel and comprising no more than about 4 percent by weight iron.

In a disclosed embodiment, the cobalt-based alloy comprises from about 18 weight percent to about 50 weight percent cobalt, from about 10 weight percent to about 25 weight percent chromium, from about 10 weight percent to about 15 weight percent tungsten, from about 0 weight percent to about 2 weight percent manganese, from about 0 weight percent to about 3 weight percent iron, and from about 10 weight percent to about 65 weight percent of the one or more platinum group metals.

In a disclosed embodiment, the cobalt-based alloy comprises from about 40 weight percent to about 50 weight percent cobalt, from about 15 weight percent to about 25 weight percent chromium, from about 10 weight percent to about 15 weight percent tungsten, from about 0 weight percent to about 2 weight percent manganese, from about 0 weight percent to about 3 weight percent iron, and from about 10 weight percent to about 35 weight percent of the one or more platinum group metals.

In a disclosed embodiment, the cobalt-based alloy comprises from about 18 weight percent to about 50 weight percent cobalt, from about 10 weight percent to about 25 weight percent chromium, from about 10 weight percent to about 15 weight percent tungsten, from about 0 weight percent to about 2 weight percent manganese, from about 0 weight percent to about 3 weight percent iron, and from about 10 weight percent to about 65 weight percent of the one or more platinum group metals, wherein the one or more platinum group metals are selected from the group consisting of platinum and palladium.

In a disclosed embodiment, the cobalt-based alloy comprises from about 18 weight percent to about 50 weight percent cobalt, from about 10 weight percent to about 25 weight percent chromium, from about 10 weight percent to about 15 weight percent tungsten, from about 0 weight percent to about 2 weight percent manganese, from about 0 weight percent to about 3 weight percent iron, and from about 10 weight percent to about 65 weight percent of the one or more platinum group metals, wherein the one or more platinum group metals comprise from about 10 atomic percent to about 12 atomic percent of the cobalt-based alloy.

In a disclosed embodiment, the cobalt-based alloy comprises from about 22 weight percent to about 40 weight percent cobalt, from about 15 weight percent to about 25 weight percent chromium, from about 4 weight percent to about 7 weight percent molybdenum, from about 0 weight percent to about 2 weight percent manganese, from about 0 weight percent to about 18 weight percent iron, and from about 10 weight percent to about 65 weight percent of the one or more platinum group metals.

In a disclosed embodiment, the cobalt-based alloy comprises from about 22 weight percent to about 40 weight percent cobalt, from about 15 weight percent to about 25 weight percent chromium, from about 4 weight percent to about 7 weight percent molybdenum, from about 0 weight percent to about 2 weight percent manganese, from about 0 weight percent to about 18 weight percent iron, and from about 10 weight percent to about 65 weight percent of the one or more platinum group metals, wherein the one or more platinum group metals are selected from the group consisting of platinum and palladium.

In a disclosed embodiment, the cobalt-based alloy comprises from about 22 weight percent to about 40 weight percent cobalt, from about 15 weight percent to about 25 weight percent chromium, from about 4 weight percent to about 7 weight percent molybdenum, from about 0 weight percent to about 2 weight percent manganese, from about 0 weight percent to about 18 weight percent iron, and from about 10 weight percent to about 65 weight percent of the one or more platinum group metals, wherein the one or more platinum group metals comprise from about 14 atomic percent to about 16 atomic percent of the cobalt-based alloy.

In a disclosed embodiment, the cobalt-based alloy comprises from about 22 weight percent to about 40 weight percent cobalt, from about 15 weight percent to about 25 weight percent chromium, from about 4 weight percent to about 7 weight percent molybdenum, from about 0 weight percent to about 2 weight percent manganese, from about 0 weight percent to about 18 weight percent iron, and from about 10 weight percent to about 65 weight percent of the one or more platinum group metals, wherein the one or more platinum group metals comprise from about 33 atomic percent to about 35 atomic percent of the cobalt-based alloy.

In a disclosed embodiment, the cobalt-based alloy comprises from about 18 weight percent to about 39 weight percent cobalt, from about 10 weight percent to about 25 weight percent chromium, from about 5 weight percent to about 10 weight percent molybdenum, and from about 10 weight percent to about 65 weight percent of the one or more platinum group metals.

In a disclosed embodiment, the cobalt-based alloy comprises from about 18 weight percent to about 35 weight percent cobalt, from about 15 weight percent to about 25 weight percent chromium, from about 5 weight percent to about 10 weight percent molybdenum, and from about 40 weight percent to about 65 weight percent of the one or more platinum group metals.

In a disclosed embodiment, the cobalt-based alloy comprises from about 18 weight percent to about 35 weight percent cobalt, from about 15 weight percent to about 25 weight percent chromium, from about 5 weight percent to about 10 weight percent molybdenum, and from about 40 weight percent to about 65 weight percent of the one or more platinum group metals, wherein the one or more platinum group metals are selected from the group consisting of platinum and palladium.

In a disclosed embodiment, the cobalt-based alloy comprises from about 18 weight percent to about 35 weight percent cobalt, from about 15 weight percent to about 25 weight percent chromium, from about 5 weight percent to about 10 weight percent molybdenum, and from about 40 weight percent to about 65 weight percent of the one or more platinum group metals, wherein the one or more platinum group metals comprise from about 35 atomic percent to about 37 atomic percent of the cobalt-based alloy.

In a disclosed embodiment, the cobalt-based alloy is formed by providing an initial alloy comprising nickel and substituting the nickel with the one or more platinum group metals.

In a disclosed embodiment, the cobalt-based alloy is formed by providing an initial alloy comprising nickel, manganese, and iron and substituting the nickel, manganese, and iron with the one or more platinum group metals.

In an embodiment, the cobalt-based alloy is formed by providing each constituent metal in powder form, mixing the powders together, and compacting and sintering the mixture of constituent metals in powder form so as to form the cobalt-based alloy.

In an embodiment, the cobalt-based alloy is formed by providing each constituent metal in solid form or the powder form of each constituent metal and then melting the pieces or parts by arc melting, electron beam melting, induction melting, radiant heat melting, microwave melting, or so forth.

In a disclosed embodiment, the one or more platinum group metals consists of iridium.

In a disclosed embodiment, the one or more platinum group metals consists of iridium, and the cobalt-based alloy is a ternary Co-Cr-Ir alloy consisting essentially of cobalt, chromium, and iridium in which the chromium is present from about 10 weight percent to about 25 atomic percent, and the ratio of iridium to cobalt is greater than about 1:1 on an atomic basis.

In a disclosed embodiment, the cobalt-based alloy is formed by providing an initial alloy comprising nickel and cobalt and substituting the nickel and at least a portion of the cobalt with the one or more platinum group metals.

In a disclosed embodiment, the cobalt-based alloy is formed by providing an initial alloy comprising nickel and cobalt and substituting the nickel and at least a portion of the cobalt with the one or more platinum group metals, wherein the one or more platinum group metals are selected from the group consisting of platinum and palladium.

According to another disclosed embodiment the main body includes a cobalt-based alloy that includes cobalt, chromium, and one or more so-called refractory metals (*i.e.*, zirconium, niobium, molybdenum, hafnium, tantalum, tungsten, rhenium, silver, or gold). Silver and gold are included within this broad classification of refractory metals for sake of simplicity, as they can be used, even though their melting temperatures are significantly lower than the other members of the class. These two metals could alternatively be termed "precious metals". In one embodiment, the one or more included refractory metals substitute for nickel, such that the alloy is substantially nickel free (*e.g.*, includes no more than about 2% nickel by weight). Another embodiment is entirely free of nickel. In addition, the alloys may include iron, although the amount of iron is limited to no more than about 20% by weight, no more than about 10% by weight, or no more than about 8% by weight. In other embodiments, the alloys are substantially iron free (*e.g.*, no more than about 4% iron by weight). In another embodiment, iron is entirely absent.

Another embodiment of the present invention includes a method for positioning a stent in a lumen of a living being. The method comprises providing a radiopaque stent comprising a cylindrical main body that includes a cobalt-based alloy as described above. The cobalt-based alloy is deformable in a ductile manner, rendering the radiopaque stent balloon expandable on a delivery system. The stent is initially unexpanded. The stent is transported to a lesion site in the lumen wherein the stent is optionally imaged during transport. The stent is expanded to contact the lesion. The radiopaque stent is imaged during or after expanding the stent.

In a disclosed embodiment, the cobalt-based alloy may be formed beginning with a cobalt-based alloy that does not contain the platinum group metal or refractory metal, but contains another component to be partially or completely substituted (*e.g.*, nickel) with a platinum group metal or refractory metal. All ingredients would then be melted together (e.g., arc melting, electron beam melting, induction melting, radiant heat melting, microwave melting, or so forth) to produce an ingot which is then processed by conventional metalworking means to produce tubing or other desired forms. Additional elements such as iron, silicon, titanium, manganese and cobalt may also be substituted either partially or completely in addition to the nickel. For example, the substitution may be made by arc melting the alloy in the presence of the substituting element(s). For example, nickel initially present within such a cobalt-chromium alloy may thus be partially or completely substituted with a platinum group metal or refractory metal. In some embodiments, a portion of the cobalt may also be substituted.

In another embodiment, powdered elements of the various constituents of the revised cobalt-based alloy composition may be mixed together and then compacted and sintered so as to form the desired alloy by means of conventional powder metallurgy processing techniques.

### BRIEF DESCRIPTION OF THE DRAWINGS

To further clarify the above and other advantages and features of the present disclosure, a more particular description will be rendered by references to specific embodiments thereof, which are illustrated in the appended drawings. It is appreciated that these drawings depict only typical embodiments of the invention and are therefore not to be considered limiting of its scope. The present disclosure will be described and explained with additional specificity and detail through the use of the accompanying drawings in which:
**FIG. 1** is an elevational view, partially in section, of a radiopaque stent according to an embodiment of the present invention mounted on a delivery catheter and disposed within a damaged lumen;
**FIG. 2** is an elevational view, partially in section, showing the radiopaque stent of **FIG. 1** within a damaged lumen;
**FIG. 3** is an elevational view, partially in section, showing the radiopaque stent of **FIG. 1** expanded within the lumen after withdrawal of the delivery catheter;
**FIGS. 4A** and **4B** show a phase diagram for cobalt-chromium;
**FIGS. 5A** and **5B** show a phase diagram for cobalt-nickel;
**FIGS. 6A** and **6B** show a phase diagram for nickel-chromium;
**FIGS. 7A** and **7B** show a phase diagram for cobalt-tungsten;
**FIGS. 8A** and **8B** show a phase diagram for chromium-tungsten;
**FIGS. 9A** and **9B** show a phase diagram for nickel-tungsten;
**FIGS. 10A** and **10B** show a phase diagram for molybdenum-cobalt;
**FIGS. 11A** and **11B** show a phase diagram for chromium-molybdenum;
**FIGS. 12A** and **12B** show a phase diagram for nickel-molybdenum;
**FIGS. 13A** and **13B** show a phase diagram for silver-cobalt;
**FIGS. 14A** and **14B** show a phase diagram for chromium-silver;
**FIGS. 15A** and **15B** show a phase diagram for silver-molybdenum;
**FIGS. 16A** and **16B** show a phase diagram for gold-cobalt;
**FIGS. 17A** and **17B** show a phase diagram for gold-chromium;
**FIGS. 18A** and **18B** show a phase diagram for gold-molybdenum;
**FIGS. 19A** and **19B** show a phase diagram for gold-tungsten;
**FIGS. 20A** and **20B** show a phase diagram for cobalt-hafnium;
**FIGS. 21A** and **21B** show a phase diagram for chromium-hafnium;
**FIGS. 22A** and **22B** show a phase diagram for molybdenum-hafnium;
**FIGS. 23A** and **23B** show a phase diagram for hafnium-tungsten;
**FIGS. 24A** and **24B** show a phase diagram for molybdenum-cobalt;
**FIGS. 25A** and **25B** show a phase diagram for chromium-molybdenum;
**FIGS. 26A** and **26B** show a phase diagram for molybdenum-tungsten;
**FIGS. 27A** and **27B** show a phase diagram for niobium-cobalt;
**FIGS. 28A** and **28B** show a phase diagram for chromium-niobium;
**FIGS. 29A** and **29B** show a phase diagram for molybdenum-niobium;
**FIGS. 30A** and **30B** show a phase diagram for niobium-tungsten;
**FIGS. 31A** and **31B** show a phase diagram for cobalt-rhenium;
**FIGS. 32A** and **32B** show a phase diagram for chromium-rhenium;
**FIGS. 33A** and **33B** show a phase diagram for molybdenum-rhenium;
**FIGS. 34A** and **34B** show a phase diagram for rhenium-tungsten;
**FIGS. 35A** and **35B** show a phase diagram for cobalt-tantalum;
**FIGS. 36A** and **36B** show a phase diagram for chromium-tantalum;
**FIGS. 37A** and **37B** show a phase diagram for molybdenum-tantalum;
**FIGS. 38A** and **38B** show a phase diagram for tantalum-tungsten;
**FIGS. 39A** and **39B** show a phase diagram for cobalt-tungsten;
**FIGS. 40A** and **40B** show a phase diagram for chromium-tungsten;
**FIGS. 41A** and **41B** show a phase diagram for molybdenum-tungsten;
**FIGS. 42A** and **42B** show a phase diagram for cobalt-zirconium;
**FIGS. 43A** and **43B** show a phase diagram for zirconium-chromium;
**FIGS. 44A** and **44B** show a phase diagram for molybdenum-zirconium;
**FIGS. 45A** and **45B** show a phase diagram for tungsten-zirconium;
**FIGS. 46A** and **46B** show a phase diagram for cobalt-iron;
**FIGS. 47A** and **47B** show a phase diagram for cobalt-palladium;
**FIGS. 48A** and **48B** show a phase diagram for cobalt-platinum;
**FIGS. 49A** and **49B** show a phase diagram for chromium-iron;
**FIGS. 50A** and **50B** show a phase diagram for chromium-palladium;
**FIGS. 51A** and **51B** show a phase diagram for chromium-platinum;
**FIGS. 52A** and **52B** show a phase diagram for iron-palladium;
**FIGS. 53A** and **53B** show a phase diagram for iron-platinum; and
**FIGS. 54A** and **54B** show a phase diagram for palladium-platinum.

### DETAILED DESCRIPTION

### I. Introduction

Embodiments of the present invention are directed to radiopaque stents of claim 1 and related methods of manufacture and use. The alloys of the cylindrical main body are substantially nickel free (*e.g.,* include no more than about 2% by weight nickel, more typically no more than 1% nickel by weight). In one embodiment, the alloy is entirely free of nickel. By entirely free of nickel, it will be understood that minute trace fractions of nickel may still be present in some embodiments based on the fact that the alloy includes cobalt, and it can be very difficult, if not a practical impossibility to entirely separate trace amounts of nickel from the cobalt. In any event, where the alloy is "entirely" free of nickel, no nickel is intentionally added to the alloy.

Some patients exhibit an allergic reaction to nickel, and so providing a nickel-free alloy is advantageous as it increases the biocompatibility of the alloy. In addition, the inclusion of the nickel limits the degree of radiopacity that may be achieved. In addition, the alloy preferably does not include a large fraction of iron. The alloy includes no more than about 20% by weight iron, more preferably no more than about 16% by weight iron, no more than about 10% by weight iron, or no more than about 8% by weight iron. In another embodiment, iron is present, if at all, in an amount not greater than about 4% by weight, more typically not greater than about 3% by weight. In another embodiment, the alloy is iron-free. Similar to the meaning of nickel-free, the term iron-free means that no iron is intentionally added to the alloy, although trace amounts may be present as impurities carried in the other elements. Limiting the amount of iron within the alloy allows other elements to be present that provide for better radiopacity and corrosion resistance than does iron. In addition, it may reduce any magnetic characteristics of the resulting metal alloy, which may be helpful in conjunction with MRI and/or other imaging techniques.

### II. Radiopaque Stent and Cobalt-Based Alloy Embodiments

According to various embodiments, the radiopaque stent is imagable during fluoroscopy. Due to the enhanced radiopacity, the entire stent is better observed by the practitioner placing the stent. The image observed by the practitioner is thus less likely to be too faint due to insufficient radiopacity or "washed out" from excessive radiopacity. Because of the improved image, the stent is more easily and accurately positioned and manipulated within a lumen of a patient. Because the radiopaque composition from which the stent is formed has greater radiopacity per unit thickness than alternative alloys, the stent may also be formed at thinner thickness all while providing a desired level of radiopacity performance. An additional advantage to the better radiopacity is the visualization of the stent and the underlying vessel during follow-up examinations by the practitioner. Because the entire stent is radiopaque, the diameter and length of the stent are readily discerned by the practitioner. Also, because the stent itself is made of the radiopaque alloy, the stent does not have problems associated with radiopaque coatings, such as cracking, separation, or corrosion. Also, because the entire stent is radiopaque, the stent does not require extra markers with their attendant issues.

The strength of the alloy material is sufficient that the stent may be manufactured with a low profile. The low profile of the disclosed cobalt-based alloy stents, coupled with its enhanced radiopacity renders the stent easily deliverable with easier observation and detection throughout its therapeutic use than stents heretofore available. A stent constructed of the specific contemplated cobalt-based alloys can be made thinner than one of stainless steel without sacrificing fluoroscopic visibility. The low profile of the disclosed cobalt-based stents renders the stent more easily deliverable with greater flexibility.

Furthermore, improved radiopacity of the low profile stent increases deliverability of the stent and offers additional performance advantages in the form of decreased fluid mechanics disturbances to blood flow. Improved radiopacity assists the practitioner in placing the device precisely. Inflation of the stent is better monitored because the stent is more readily visible to the practitioner. This visibility reduces the incidence and probability of an under-deployed stent. Further, in-stent restenosis may be more accurately monitored as the stent and an injected contrast agent are able to be imaged simultaneously. Unlike some stents, the disclosed stents do not produce an image which is too bright, thereby obscuring imaging of the underlying vessel morphology.

Many cobalt-based alloys, although very strong, have insufficient ductility for use in a stent. The cobalt-based alloys described herein preferably have at least 20% or greater elongation and thereby achieve adequate stent expansion. Some of the disclosed cobalt-based alloys also include tungsten not only strengthens, but also contributes to the overall excellent radiopacity of the cobalt-based alloy. Tungsten may also improve corrosion resistance and a resistance to oxidation at high temperatures of the cobalt-based alloy.

For example, disclosed cobalt-chromium alloy L-605, covered by ASTM standard F90, includes about 15% by weight tungsten. Alloy L-605 has a minimum ultimate tensile strength of 125 ksi, a minimum yield strength of 45 ksi and a minimum total elongation of 30%. According to a disclosed embodiment of the invention, the alloy is similar to L-605, in which substantially all of the nickel of L-605 has been replaced with a platinum group metal, a refractory metal, or combinations thereof. For example, alloy L-605 contains about 10% by weight nickel. By substituting the nickel with a platinum group metal or refractory metal, the relative radiopacity of the resulting alloy is increased relative to alloy L-605, and the resulting alloy is advantageously nickel free or substantially nickel free.

Another disclosed alloy which may be similarly modified is Elgiloy, covered by ASTM standard F1058 Grade 1. Phynox is a disclosed alloy composition similar to that of Elgiloy. Phynox is covered by ASTM standard F1058 Grade 2. Elgiloy is a cobalt-chromium alloy containing about 40% by weight cobalt, about 20% by weight chromium, about 16% by weight iron, about 15% by weight nickel, about 7% by weight molybdenum, and about 2% by weight manganese. Phynox is similar, but the manganese is replaced with iron. The nickel may be substituted with a platinum group metal or refractory metal so as to result in an alloy having increased relative radiopacity and that is nickel free or substantially nickel free. In another disclosed a embodiment, the platinum group metal or refractory metal may also replace all or a part of the iron and/or manganese.

Another disclosed alloy which may be similarly modified is MP-35N, covered by ASTM standard F562. MP-35N is a cobalt-chromium alloy containing about 35% by weight cobalt, about 20% by weight chromium, about 35% by weight nickel, about 10% by weight molybdenum, and about 1% maximum iron. The nickel may be substituted with a platinum group metal or refractory metal so as to result in an alloy having increased relative radiopacity and that is nickel free or substantially nickel free. In a disclosed embodiment, the platinum group metal or refractory metal may replace all or a part of the iron as well.

In additional disclosed embodiments, upon completely replacing the nickel, a portion of the cobalt may also be replaced by the platinum group metal(s) or refractory metal(s), based on the overall increase in radiopacity that is desired. This substitution may be performed for any cobalt-based alloy, including those described above. To maintain a single-phase microstructure, it is suggested that the cobalt be substituted with a platinum group element or refractory metal that has substantially complete mutual solid solubility with cobalt. Furthermore, in some disclosed embodiments, some of the chromium or another element in the known alloy may also be replaced.

Atomic substitution takes an "atom for atom" approach in alloy modification by employing atomic weight. Atomic weight is commonly understood to be weight per mole of atoms. Atomic substitution maintains the stoichiometry of the original alloy when substituting, which may be an important approach when working with ordered alloys and when maintaining a particular phase structure. Atomic substitution may be more commonly understood in the art than volumetric substitution.

Volumetric substitution accounts for both the atomic radii and the crystal structure that the element naturally takes in the solid form, by employing atomic volume. Atomic volume is commonly understood to be volume per mole of atoms in the solid phase. This approach provides insight into effects on the host lattice by the substituting atom(s). This approach may allow for better retaining and understanding of the workability and mechanical strength of the modified alloy.

Substitution on a weight percent basis results in alloys that have comparatively less of the platinum group elements (or refractory elements), as all of them have greater atomic weight and density than the majority of elements being substituted for in the original cobalt-based alloys. Utilizing weight percent for substitution may be more typically employed for cobalt-based alloys where the elements have similar atomic weights, such as stainless steels, which are comprised primarily of iron, chromium and nickel.

Due to the low atomic weight of nickel compared to that of the platinum group metals (and somewhat less so as compared to the refractory metals), it will readily be realized that substituting for nickel would produce significantly different overall alloy compositions if the substitution were based on weight percentage as compared to atomic percentage. This is an important consideration with regard to radiopacity, because the attenuation of x-rays by a given material is largely dictated by the electric fields surrounding its atoms and their nuclei. Elements with more massive nuclei, and correspondingly more orbiting electrons, attenuate x-rays to a much greater extent than those with lighter nuclei, which explains why metals like tantalum, tungsten, platinum, and gold are inherently more radiopaque than lighter metals like chromium, iron, cobalt, and nickel. Thus, when substituting a platinum group metal (or a refractory metal) for nickel in a given alloy, the resulting impact on radiopacity is considerably greater when the nickel is replaced on an atom-for-atom basis rather than gram-for-gram. The impact of a given alloying element on many of an alloy's chemical properties, such as corrosion resistance, also depends on the atomic percentage present.

While nickel is the preferred element to be substituted by a platinum group or refractory metal for biocompatibility reasons, in some embodiments other elements in commercial cobalt-based alloys may be replaced (*i.e.*, substituted), particularly if greater radiopacity is desired. Note that iron and manganese are minor alloying elements in L-605, iron and silicon are minor alloying elements in Elgiloy, and iron and titanium are minor alloying elements in MP-35N. These elements are not considered essential, with regard to corrosion behavior and room-temperature mechanical properties, especially if other impurities such as carbon and sulfur are held to a minimum, and therefore could be replaced by more of the platinum group metal(s) (or refractory metal(s)) and thereby simplify the overall composition while further increasing radiopacity. Should radiopacity still be insufficient after nickel and these minor alloying elements have been fully substituted, a portion of the cobalt could also be replaced by the platinum group metal(s). This strategy would more likely be applied to cobalt-based alloys that contain lesser amounts of nickel, such as L-605.

Nickel plays an important role in commercial cobalt-based alloys. As in iron-based stainless steels, nickel serves as an "austenite stabilizer" in cobalt-based alloys. That is, nickel suppresses cobalt's allotropic transformation from a face-centered-cubic ("FCC") crystal structure at high temperatures to a hexagonal-close-packed ("HCP") structure at low temperatures. In pure cobalt, this transformation naturally occurs at around 422°C. The addition of nickel significantly reduces cobalt's transformation temperature, thereby favoring the FCC structure which, in general, is a more ductile and more creep-resistant crystal structure than HCP. Therefore, when substituting nickel with another element, it is important that the replacement also serve as an austenite stabilizer.

By way of example, palladium immediately suppresses cobalt's FCC-to-HCP transformation temperature at relatively small addition levels, whereas platinum, rhodium and iridium initially raise and then ultimately lower the transformation temperature as alloying levels rise, while ruthenium and osmium continuously raise cobalt's transformation temperature as their levels rise. Similar considerations apply in the potential substitution of refractory metals for nickel. Thus, the particular platinum group and/or refractory metal(s) selected and their substitution levels are important considerations with regard to the final crystal structure(s) that will be obtained at ambient temperatures.

Chromium also plays an important role in commercial cobalt-based alloys. As in iron-based stainless steels, chromium is also a powerful corrosion inhibitor in cobalt-based alloys. Corrosion and elevated-temperature oxidation behavior are substantially improved by the stable, tightly adhering chromium oxide layer that spontaneously forms when chromium containing cobalt-based alloys are exposed to air or other oxidizing environments. This layer serves to protect these alloys in a variety of otherwise corrosive environments, including saline and blood. For this reason, it is beneficial when substituting nickel and possibly other elements with platinum group and/or refractory metals that the resulting alloy composition contain sufficient chromium that adequate corrosion resistance is maintained. Iron-based austenitic stainless steels like types 304 and 316 typically contain approximately 18% by weight chromium, whereas L-605, Elgiloy, and MP-35N each contain about 20% by weight. Thus, it is not recommended that platinum group metals and/or refractory metals replace significant amounts of the chromium present in commercial cobalt-based alloys. Where additional corrosion resistance is warranted, the chromium level may be increased (e.g., to about 25% by weight).

In any case, chromium is present in sufficient amount to inhibit corrosion. Some alloy embodiments may include chromium fractions well below 20% by weight while still achieving this purpose (e.g., at least about 10% by weight, or between about 10% and about 15% by weight). Specific examples of such alloys are found in Table 5 and Examples 78-89. This may be possible particularly where the atomic percentage of chromium remains relatively high (e.g., at least about 20 atomic percent, or at least about 25 atomic percent). The weight percentages are lower in Examples 78-89 because the very dense platinum is included in high fractions.

Generally, alloys based on an L-605 alloy in which the nickel has been replaced on either an atomic or volumetric basis with a platinum group metal may include about 18 weight percent to about 50 weight percent cobalt *(e.g.,* in one embodiment about 40 to about 50 weight percent cobalt), about 10 weight percent to about 25 weight percent chromium (*e.g.*, in one embodiment about 15 weight percent to about 25 weight percent chromium, about 15 to about 20 weight percent chromium, or about 20 weight percent chromium), about 10 weight percent to about 15 weight percent tungsten, about 0 weight percent to about 2 weight percent manganese, about 0 weight percent to about 3 weight percent iron, and about 10 weight percent to about 65 weight percent of a platinum group metal. A disclosed embodiment may include about 10 weight percent to about 35 weight percent of a platinum group metal (*i.e.*, platinum, palladium, ruthenium, rhodium, osmium, iridium, or combinations thereof). Reference examples of such materials are further described below in conjunction with Table 1 and Examples 1-12. Trace elements included within this alloy may not be shown unless they are called out for substitutional purposes.

Generally, disclosed alloys based on an ASTM F1058 alloy (*e.g.*, Elgiloy or Phynox) in which at least the nickel has been replaced (*e.g.*, iron and/or manganese may also be replaced) on an atomic basis with a platinum group metal may include about 22 weight percent to about 40 weight percent cobalt, about 10 weight percent to about 25 weight percent chromium (*e.g*., in one embodiment about 15 weight percent to about 25 weight percent chromium, about 15 to about 20 weight percent chromium, or about 20 weight percent chromium), about 4 weight percent to about 7 weight percent molybdenum, about 0 weight percent to about 2 weight percent manganese, about 0 weight percent to about 18 weight percent iron, and about 10 weight percent to about 65 weight percent of a platinum group metal (*i.e.,* platinum, palladium, ruthenium, rhodium, osmium, iridium, or combinations thereof). One example may include about 15 weight percent to about 65 weight percent of a platinum group metal. Reference examples of such materials are further described below in conjunction with Table 2 and Examples 13-20. Trace elements included within this alloy may not be shown unless they are called out for substitutional purposes.

Another disclosed alloy based on an ASTM F1058 alloy could replace the nickel with a refractory metal (e.g., silver, gold, hafnium, niobium, rhenium, tantalum, molybdenum, zirconium, or combinations thereof), and include weight fractions as described above, but in which the refractory metal (rather than a platinum group metal) is included from about 10 weight percent to about 65 weight percent, or from about 15 weight percent to about 65 weight percent.

Generally, disclosed alloys based on an MP-35N alloy in which the nickel has been replaced on an atomic percentage basis by a platinum group metal may include about 18 weight percent to about 39 weight percent cobalt (*e.g.*, one embodiment may include about 18 to about 35 weight percent cobalt), about 10 weight percent to about 25 weight percent chromium (*e.g*., in one embodiment about 15 weight percent to about 25 weight percent chromium, about 15 to about 20 weight percent chromium, about 10 to about 21 weight percent chromium, or about 20 weight percent chromium), about 5 weight percent to about 10 weight percent molybdenum, and about 10 weight percent to about 65 weight percent of a platinum group metal (*i.e.*, platinum, palladium, ruthenium, rhodium, osmium, iridium, or combinations thereof). One embodiment may include about 40 to about 65 weight percent of a platinum group metal. Reference examples of such materials are further described below in conjunction with Table 3 and Examples 21-24. Trace elements included within this alloy may not be shown unless they are called out for substitutional purposes.

Generally, disclosed alloys based on an L-605 alloy in which the nickel has been replaced on a weight percentage basis with a refractory metal may include about 18 weight percent to about 55 weight percent cobalt (*e.g.*, in one embodiment about 20 to about 55 weight percent cobalt), about 15 weight percent to about 25 weight percent chromium (*e.g.*, in one embodiment about 20 weight percent chromium), about 0 weight percent to about 15 weight percent tungsten, and about 10 weight percent to about 60 weight percent of a substituting refractory metal selected from the group consisting of silver, gold, hafnium, niobium, rhenium, tantalum, molybdenum, zirconium, or combinations thereof. One embodiment may include about 10 weight percent to about 45 weight percent of the substituting refractory metal (*i.e.*, silver, gold, hafnium, niobium, rhenium, tantalum, molybdenum, zirconium, or combinations thereof). Reference examples of such alloys are further described below in conjunction with Table 1 and Examples 25-28, 31-33, 36-38, 41-43, 46-47, 50-52, 55-57, 60, 65-66, and 69. Other trace elements (e.g., manganese, iron, etc.) may be present in small amounts of about 0 to about 3 percent by weight. Trace elements included within this alloy may not be shown unless they are called out for substitutional purposes.

L-605 alloy already contains about 15 weight percent tungsten. A disclosed alloy based on an L-605 alloy in which the nickel has been replaced on a weight percentage basis with the refractory metal tungsten may include about 18 weight percent to about 55 weight percent cobalt (*e.g.*, in one embodiment about 20 to about 55 weight percent cobalt), about 15 weight percent to about 25 weight percent chromium (*e.g*., in one embodiment about 20 weight percent chromium), and about 25 weight percent to about 60 weight percent tungsten. One embodiment may include about 25 weight percent to about 45 weight percent of the substituting refractory metal tungsten. Reference examples of such tungsten alloys are further described below in conjunction with Table 1 and Examples 61-62. Other trace elements (e.g., manganese, iron, etc.) may be present in small amounts of about 0 to about 3 percent by weight.

Generally, disclosed alloys based on an MP-35N alloy in which the nickel has been replaced on a weight percentage basis by a refractory metal may include about 18 weight percent to about 39 weight percent cobalt *(e.g.,* one embodiment may include about 20 to about 35 weight percent cobalt), about 15 weight percent to about 25 weight percent chromium (*e.g.*, one embodiment may include about 20 weight percent chromium), about 0 weight percent to about 10 weight percent molybdenum, and about 35 weight percent to about 60 weight percent of a substituting refractory metal (*i.e.*, silver, gold, hafnium, niobium, rhenium, tantalum, tungsten, zirconium, or combinations thereof). One embodiment may include about 35 to about 50 weight percent of a substituting refractory metal. Examples of such materials are further described below in conjunction with Table 3 and Examples 29-31, 34-36, 39-41, 44-45, 48-50, 53-55, 58-60, 63-64, and 67-69. Trace elements included within this alloy may not be shown unless they are called out for substitutional purposes.

MP-35N alloy already contains about 10 weight percent molybdenum. A disclosed alloy based on an MP-35N alloy in which the nickel has been replaced on a weight percentage basis with the refractory metal molybdenum may include about 18 weight percent to about 39 weight percent cobalt (*e.g.,* in one embodiment about 20 to about 35 weight percent cobalt), about 15 weight percent to about 25 weight percent chromium (*e.g.,* in one embodiment about 20 weight percent chromium), and about 45 weight percent to about 60 weight percent molybdenum. Reference examples of such molybdenum alloys are further described below in conjunction with Table 1 and Examples 44-45. Other trace elements (e.g., manganese, iron, etc.) may be present in small amounts of about 0 to about 3 percent by weight.

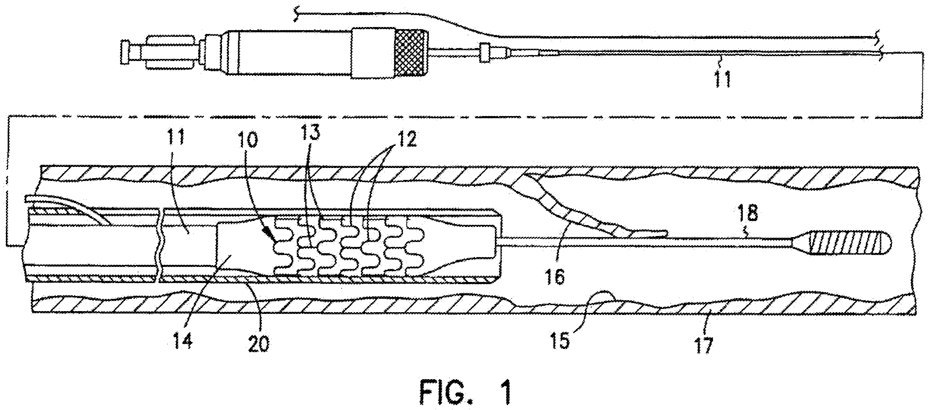

One type of radiopaque stent design embodiment is a high precision patterned cylindrical device. This device is illustrated generally at 10 in **FIG. 1****.** The stent 10 typically comprises a plurality of radially expanded cylindrical elements 12 disposed generally coaxially and interconnected by elements 13 disposed between adjacent cylindrical elements.

For some embodiments, the stent 10 is expanded by a delivery catheter 11. The delivery catheter 11 has an expandable portion or a balloon 14 for expanding of the stent 10 within an artery 15. The delivery catheter 11 onto which the stent 10 is mounted may be similar to a conventional balloon dilation catheter used for angioplasty procedures. The artery 15, as shown in **FIG. 1****,** has a dissected lining 16 which has occluded a portion of the arterial passageway.

Each radially expandable cylindrical element 12 of the radiopaque stent 10 may be independently expandable. Therefore, the balloon 14 may be provided with an inflated shape other than cylindrical, *e.g.*, tapered, to facilitate implantation of the stent 10 in a variety of body lumen shapes.

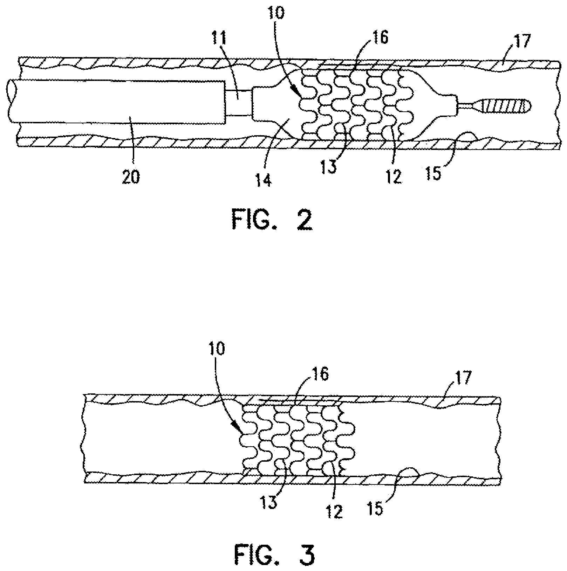

The delivery of the radiopaque stent 10 is accomplished by mounting the stent 10 onto the inflatable balloon 14 on the distal extremity of the delivery catheter 11. The catheter-stent assembly is introduced within the patient's vasculature using conventional techniques through a guiding catheter which is not shown. A guidewire 18 is disposed across the damaged arterial section and then the catheter-stent assembly is advanced over a guidewire 18 within the artery 15 until the stent 10 is directly under detached lining 16 of the damaged arterial section. The balloon 14 of the catheter is expanded, expanding the stent 10 against the artery 15, which is illustrated in **FIG. 2****.** While not shown in the drawing, the artery 15 may preferably be expanded slightly by the expansion of the stent 10 to seat or otherwise fix the stent 10 to prevent movement. In some circumstances during the treatment of a stenotic portion of an artery, the artery may have to be expanded considerably in order to facilitate passage of blood or other fluid therethrough. This expansion is easily observable by the practitioner with the disclosed radiopaque stents.

The stent 10 serves to hold open the artery 15 after the catheter 11 is withdrawn, as illustrated in **FIG. 3****.** Due to the formation of the stent 10 from the elongated tubular member, the undulating component of the cylindrical elements of the stent 10 is relatively flat in transverse cross section so that when the stent is expanded, the cylindrical elements are pressed into the wall of the artery 15 and as a result do not interfere with the blood flow through the artery 15. The cylindrical elements 12 of the stent 10 which are pressed into the wall of the artery 15 are eventually covered with endothelial cell growth which further minimizes blood flow interference. The undulating pattern of the cylindrical sections 12 provides good characteristics to prevent stent movement within the artery. Furthermore, the closely spaced cylindrical elements at regular intervals provide uniform support for the wall of the artery 15, and consequently are well adapted to tack up and hold in place small flaps or dissections in the wall of the artery 15 as illustrated in **FIGS. 2-3****.** The undulating pattern of the radiopaque stent is readily discernable to the practitioner performing the procedure.

Additional details of exemplary stents are disclosed in U.S. Patent No. 7,250,058.

**Table 1 - ASTM F90 L-605 Disclosed Alloy**

| Element | Weight Percent | Atomic Percent | Volume Percent |
|---|---|---|---|
| Cobalt | 50 | 53.9 | 51.6 |
| Chromium | 20 | 24.4 | 25.2 |
| Tungsten | 15 | 5.2 | 7.1 |
| Nickel | 10 | 10.8 | 10.2 |
| Manganese (maximum) | 2 | 2.3 | 2.4 |
| Iron (maximum) | 3 | 3.4 | 3.5 |

Reference examples 1-12 below are based on the nominal compositions of the ASTM F90 L-605 Alloy of Table 1 in which only the nickel has been replaced on an atomic substitution basis or a volumetric substitution basis with a platinum group metal. Trace elements such as beryllium, boron, carbon, phosphorus, silicon, and sulfur are not listed.

### Reference Example 1 - ASTM F90 L-605 Alloy With Atomic Substitution of Nickel With Platinum

| Element | Atomic Percent | Weight Percent |
|---|---|---|
| Cobalt | 53.9 | 40.6 |
| Chromium | 24.4 | 16.2 |
| Tungsten | 5.2 | 12.2 |
| Platinum | 10.8 | 27.0 |
| Manganese (maximum) | 2.3 | 1.6 |
| Iron (maximum) | 3.4 | 2.4 |

### Reference Example 2 - ASTM F90 L-605 Alloy With Volumetric Substitution of Nickel With Platinum

| Element | Weight Percent | Volume Percent |
|---|---|---|
| Cobalt | 43.8 | 51.6 |
| Chromium | 17.5 | 25.2 |
| Tungsten | 13.2 | 7.1 |
| Platinum | 21.1 | 10.2 |
| Manganese (maximum) | 1.8 | 2.4 |
| Iron (maximum) | 2.6 | 3.5 |

### Reference Example 3 - ASTM F90 L-605 Alloy With Atomic Substitution of Nickel With Palladium

| Element | Atomic Percent | Weight Percent |
|---|---|---|
| Cobalt | 53.9 | 46.2 |
| Chromium | 24.4 | 18.5 |
| Tungsten | 5.2 | 13.9 |
| Palladium | 10.8 | 16.8 |
| Manganese (maximum) | 2.3 | 1.8 |
| Iron (maximum) | 3.4 | 2.8 |

### Reference Example 4 - ASTM F90 L-605 Alloy With Volumetric Substitution of Nickel With Palladium

| Element | Volume Percent | Weight Percent |
|---|---|---|
| Cobalt | 51.6 | 48.3 |
| Chromium | 25.2 | 19.3 |
| Tungsten | 7.1 | 14.4 |
| Palladium | 10.2 | 13.1 |
| Manganese (maximum) | 2.4 | 2 |
| Iron (maximum) | 3.5 | 2.9 |

### Reference Example 5 - ASTM F90 L-605 Alloy With Atomic Substitution of Nickel With Rhodium

| Element | Atomic Percent | Weight Percent |
|---|---|---|
| Cobalt | 53.9 | 46.5 |
| Chromium | 24.4 | 18.6 |
| Tungsten | 5.2 | 13.9 |
| Rhodium | 10.8 | 16.3 |
| Manganese (maximum) | 2.3 | 1.9 |
| Iron (maximum) | 3.4 | 2.8 |

### Reference Example 6 - ASTM F90 L-605 Alloy With Volumetric Substitution of Nickel With Rhodium

| Element | Volume Percent | Weight Percent |
|---|---|---|
| Cobalt | 51.6 | 48.1 |
| Chromium | 25.2 | 19.2 |
| Tungsten | 7.1 | 14.4 |
| Rhodium | 10.2 | 13.4 |
| Manganese (maximum) | 2.4 | 2 |
| Iron (maximum) | 3.5 | 2.9 |

### Reference Example 7 - ASTM F90 L-605 Alloy With Atomic Substitution of Nickel With Iridium

| Element | Atomic Percent | Weight Percent |
|---|---|---|
| Cobalt | 53.9 | 40.7 |
| Chromium | 24.4 | 16.3 |
| Tungsten | 5.2 | 12.3 |
| Iridium | 10.8 | 26.7 |
| Manganese (maximum) | 2.3 | 1.6 |
| Iron (maximum) | 3.4 | 2.4 |

### Reference Example 8 - ASTM F90 L-605 Alloy With Volumetric Substitution of Nickel With Iridium

| Element | Volume Percent | Weight Percent |
|---|---|---|
| Cobalt | 51.6 | 43.4 |
| Chromium | 25.2 | 17.4 |
| Tungsten | 7.1 | 13 |
| Iridium | 10.2 | 21.9 |
| Manganese (maximum) | 2.4 | 1.7 |
| Iron (maximum) | 3.5 | 2.6 |

### Reference Example 9 - ASTM F90 L-605 Alloy With Atomic Substitution of Nickel With Ruthenium

| Element | Atomic Percent | Weight Percent |
|---|---|---|
| Cobalt | 53.9 | 46.6 |
| Chromium | 24.4 | 18.7 |
| Tungsten | 5.2 | 14.0 |
| Ruthenium | 10.8 | 16.1 |
| Manganese (maximum) | 2.3 | 1.9 |
| Iron (maximum) | 3.4 | 2.8 |

### Reference Example 10 - ASTM F90 L-605 Alloy With Volumetric Substitution of Nickel With Ruthenium

| Element | Volume Percent | Weight Percent |
|---|---|---|
| Cobalt | 51.6 | 48.2 |
| Chromium | 25.2 | 19.3 |
| Tungsten | 7.1 | 14.5 |
| Ruthenium | 10.2 | 13.2 |
| Manganese (maximum) | 2.4 | 1.9 |
| Iron (maximum) | 3.5 | 2.9 |

### Reference Example 11 - ASTM F90 L-605 Alloy With Atomic Substitution of Nickel With Osmium

| Element | Atomic Percent | Weight Percent |
|---|---|---|
| Cobalt | 53.9 | 40.8 |
| Chromium | 24.4 | 16.3 |
| Tungsten | 5.2 | 12.3 |
| Osmium | 10.8 | 26.5 |
| Manganese (maximum) | 2.3 | 1.6 |
| Iron (maximum) | 3.4 | 2.5 |

### Reference Example 12 - ASTM F90 L-605 Alloy With Volumetric Substitution of Nickel With Osmium

| Element | Volume Percent | Weight Percent |
|---|---|---|
| Cobalt | 51.6 | 43.3 |
| Chromium | 25.2 | 17.3 |
| Tungsten | 7.1 | 13.0 |
| Osmium | 10.2 | 22.0 |
| Manganese (maximum) | 2.4 | 1.7 |
| Iron (maximum) | 3.5 | 2.6 |

Although Examples 1-12 illustrate complete substitution of the nickel with a platinum group metal, it will be understood that in other disclosed embodiments, a small fraction (*e.g.*, about 2% by weight or less) of nickel may remain within the modified alloy. Platinum or palladium substitution is disclosed, as these are Group 10 elements, as is nickel. As such, these substitutions would be expected to be the most metallurgically neutral and compatible so as to maintain the strength, ductility, and microstructural integrity (*e.g.*, avoiding phase separations) of the resulting alloy. Group 9 elements (*i.e.*, rhodium or iridium) and Group 8 elements (ruthenium or osmium) may be less likely to substitute metallurgically neutrally for nickel. For example, ruthenium and osmium may decrease the ductility of the alloy. Thus, the Group 9 elements may be more preferred and the Group 10 elements may be most preferred. In another disclosed embodiment, one or more Group 11 elements (*i.e.*, silver or gold) may be substituted for the nickel. In addition, although described as substituting a single platinum group metal for the nickel, it will be understood that combinations of two or more platinum group metals (*e.g.*, platinum and palladium) may be used.

Further disclosed examples may be envisioned with the ASTM F90 L-605 alloy where the nickel, iron, and manganese have been replaced either completely or partially with palladium, platinum, and/or other Group 8, 9, 10, or 11 elements prior to melting, either singly or in combination with one another. Further disclosed examples may be envisioned where at least a portion of the cobalt is replaced by one or more elements from groups 8, 9, 10, or 11 of the periodic table.

**Table 2 - ASTM F1058 Disclosed Alloy**

| Element | Weight Percent | Atomic Percent | Volume Percent |
|---|---|---|---|
| Cobalt | 40 | 39.6 | 37.9 |
| Chromium | 20 | 22.4 | 23.2 |
| Iron | 16 | 16.7 | 16.9 |
| Manganese | 2 | 2.1 | 2.2 |
| Molybdenum | 7 | 4.3 | 5.7 |
| Nickel | 15 | 14.9 | 14.1 |

As with reference Examples 1-12, reference Examples 13-16 below based on the F1058 Alloy of Table 2 are reference examples in which the nickel has been replaced either volumetrically-based or atomically-based with palladium or platinum. As with the ASTM F90 L-605 alloy, substitutions may occur from Group 8, 9, 10, and/or 11 elements. Trace metal elements such as beryllium, boron, carbon, phosphorus, silicon, and sulfur are not listed.

### Reference Example 13 - ASTM F1058 Alloy With Atomic Substitution of Nickel With Platinum

| Element | Weight Percent | Atomic Percent |
|---|---|---|
| Cobalt | 29.7 | 39.6 |
| Chromium | 14.8 | 22.4 |
| Iron | 11.9 | 16.7 |
| Manganese | 1.5 | 2.1 |
| Molybdenum | 5.2 | 4.3 |
| Platinum | 37.0 | 14.9 |

### Reference Example 14 - ASTM F1058 Alloy With Volumetric Substitution of Nickel With Platinum

| Element | Weight Percent | Volume Percent |
|---|---|---|
| Cobalt | 33 | 37.9 |
| Chromium | 16.5 | 23.2 |
| Iron | 13.2 | 16.9 |
| Manganese | 1.7 | 2.2 |
| Molybdenum | 5.8 | 5.7 |
| Platinum | 29.8 | 14.1 |

### Reference Example 15 - ASTM F1058 Alloy With Atomic Substitution of Nickel With Palladium

| Element | Weight Percent | Atomic Percent |
|---|---|---|
| Cobalt | 35.7 | 39.6 |
| Chromium | 17.8 | 22.4 |
| Iron | 14.2 | 16.7 |
| Manganese | 1.8 | 2.1 |
| Molybdenum | 6.3 | 4.3 |
| Palladium | 24.2 | 14.9 |

### Reference Example 16 - ASTM F1058 Alloy With Volumetric Substitution of Nickel With Palladium

| Element | Weight Percent | Volume Percent |
|---|---|---|
| Cobalt | 38.0 | 37.9 |
| Chromium | 19.0 | 23.2 |
| Iron | 15.3 | 16.9 |
| Manganese | 1.9 | 2.2 |
| Molybdenum | 6.7 | 5.7 |
| Palladium | 19.1 | 14.1 |

Reference 17-20 below are based on the ASTM F1058 Alloy of Table 2 in which the nickel, iron, and manganese have been replaced with palladium or platinum on either an atomic or volumetric basis. These reference examples may be further extended to substitution with Group 8, 9, 10, and/or 11 elements. Even further reference examples may be envisioned where at least part of the cobalt is substituted by one or more elements from groups 8, 9, 10, and/or 11 of the periodic table.

### Reference Example 17 - ASTM F1058 Alloy With Atomic Substitution of Nickel, Iron, and Manganese With Platinum

| Element | Weight Percent | Atomic Percent |
|---|---|---|
| Cobalt | 22.3 | 39.6 |
| Chromium | 11.1 | 22.4 |
| Molybdenum | 3.9 | 4.3 |
| Platinum | 62.7 | 33.7 |

### Reference Example 18 - ASTM F1058 Alloy With Volumetric Substitution of Nickel, Iron, and Manganese With Platinum

| Element | Weight Percent | Volume Percent |
|---|---|---|
| Cobalt | 26.2 | 37.9 |
| Chromium | 13.1 | 23.2 |
| Molybdenum | 4.6 | 5.7 |
| Platinum | 56.1 | 33.2 |

### Reference Example 19 - ASTM F1058 Alloy With Atomic Substitution of Nickel, Iron, and Manganese With Palladium

| Element | Weight Percent | Atomic Percent |
|---|---|---|
| Cobalt | 31.1 | 39.6 |
| Chromium | 15.5 | 22.4 |
| Molybdenum | 5.5 | 4.3 |
| Palladium | 47.9 | 33.7 |

### Reference Example 20 - ASTM F1058 Alloy With Volumetric Substitution of Nickel, Iron, and Manganese With Palladium

| Element | Weight Percent | Volume Percent |
|---|---|---|
| Cobalt | 34.9 | 37.9 |
| Chromium | 17.4 | 23.2 |
| Molybdenum | 6.1 | 5.7 |
| Palladium | 41.6 | 33.2 |

**Table 3 - ASTM F562 MP-35N Disclosed Alloy**

| Element | Weight Percent | Atomic Percent | Volume Percent |
|---|---|---|---|
| Chromium | 20 | 22.9 | 23.8 |
| Nickel | 35 | 35.5 | 33.7 |
| Molybdenum | 10 | 6.2 | 8.4 |
| Cobalt | 35 | 35.4 | 34.1 |

Reference Examples 21-24 below are based on the MP-35N Alloy of Table 3 in which the nickel, as well as iron and titanium (present in MP-35N in small amounts) have been replaced with palladium or platinum on an atomic or volumetric basis. Trace elements such as beryllium, boron, carbon, iron, manganese, phosphorus, silicon, sulfur, and titanium are not listed. Further reference examples are envisioned where elements from groups 8, 9, 10, and/or 11 of the periodic table may be substituted for only the nickel, or also for at least some of the cobalt.

### Reference Example 21 - ASTM F562 Alloy With Atomic Substitution of Nickel, Iron, and Titanium With Platinum

| Element | Weight Percent | Atomic Percent |
|---|---|---|
| Chromium | 11 | 22.9 |
| Platinum | 64.2 | 35.5 |
| Molybdenum | 5.5 | 6.2 |
| Cobalt | 19.3 | 35.4 |

### Reference Example 22 - ASTM F562 Alloy With Volumetric Substitution of Nickel, Iron, and Titanium With Platinum

| Element | Weight Percent | Volume Percent |
|---|---|---|
| Chromium | 13.4 | 23.8 |
| Platinum | 56.5 | 33.7 |
| Molybdenum | 6.7 | 8.4 |
| Cobalt | 23.4 | 34.1 |

### Reference Example 23 - ASTM F562 Alloy With Atomic Substitution of Nickel, Iron, and Titanium With Palladium

| Element | Weight Percent | Atomic Percent |
|---|---|---|
| Chromium | 15.6 | 22.9 |
| Palladium | 49.4 | 35.5 |
| Molybdenum | 7.8 | 6.2 |
| Cobalt | 27.2 | 35.4 |

### Reference Example 24 - ASTM F562 Alloy With Volumetric Substitution of Nickel, Iron, and Titanium With Palladium

| Element | Weight Percent | Volume Percent |
|---|---|---|
| Chromium | 17.8 | 23.8 |
| Palladium | 42.1 | 33.7 |
| Molybdenum | 8.9 | 8.4 |
| Cobalt | 31.2 | 34.1 |

Relative radiopacity (RR) is a comparative measurement useful in comparing the relative radiopacity of various alloy materials. The higher the RR value, the greater the material's radiopacity. For example, 316L stainless steel has a RR value of about 2.5 barnes/cc. The RR values for Reference Examples 1-24, as well as the materials in Tables 1-3 are shown in Table 4 below. As can be seen, the presently disclosed alloys exhibit significantly higher relative radiopacity values than L-605, Elgiloy, or MP-35N alloys. For further comparison purposes, ASTM F138 316L stainless steel has a relative radiopacity of 2.5 barnes/cc.

**Table 4 - Relative Radiopacities**

| Reference Example | Relative Radiopacity (barnes/cc) |
|---|---|
| Table 1 (ASTM F90 L-605 Alloy) | 3.6 |
| Table 2 (ASTM F1058 Elgiloy Alloy) | 3.1 |
| Table 3 (ASTM F562 MP-35N Alloy) | 3.5 |
| Example 1 | 6.2 |
| Example 2 | 5.5 |
| Example 3 | 5.3 |
| Example 4 | 4.9 |
| Example 5 | 5.2 |
| Example 6 | 4.9 |
| Example 7 | 6.1 |
| Example 8 | 5.6 |
| Example 9 | 5.0 |
| Example 10 | 4.8 |
| Example 11 | 5.9 |
| Example 12 | 5.4 |
| Example 13 | 6.5 |
| Example 14 | 5.7 |
| Example 15 | 5.4 |
| Example 16 | 4.9 |
| Example 17 | 10.7 |
| Example 18 | 9.5 |
| Example 19 | 8.3 |
| Example 20 | 7.5 |
| Example 21 | 11.1 |
| Example 22 | 9.8 |
| Example 23 | 8.7 |
| Example 24 | 7.8 |

In a disclosed embodiment, any of the contemplated cobalt-based alloys may be formed by beginning with a cobalt-based alloy that does not contain the platinum group metal (*e.g.,* L-605, Elgiloy, Phynox, or MP-35N), but contains another component (*e.g.,* nickel) to be partially or completely substituted with the platinum group metal. The substitution may be made by arc or otherwise melting the alloy in the presence of the substituting element(s) or by combining material constituents and then melting. The material constituents may be provided as solid pieces of constituent elements, powder compacts, loose powders, etc. Nickel initially present within such a cobalt-based alloy may thus be partially or completely substituted with a platinum group metal prior to melting. All ingredients would then be melted together to produce an ingot which is then processed by conventional metalworking techniques to produce tubing or other desired forms. The above disclosed embodiments illustrate final compositions for complete substitution of the nickel, but should not be construed as embodiments of the invention.

In another embodiment, powdered elements of the various constituents of the alloy may be mixed together and then compacted and sintered so as to form the desired alloy by means of conventional powder metallurgy processing techniques. Other suitable alloy forming techniques may be apparent to those of skill in the art in light of the present disclosure.

As is apparent from the various reference examples, the alloys may be patterned after an existing alloy (*e.g.*, ASTM F90 L-605, ASTM F1058 Elgiloy or Phynox, or ASTM F562 MP-35N) in which at least the nickel and optionally the iron, manganese and/or other constituents have been substituted with a platinum group metal. The substitution may proceed so as to maintain the atomic percentage of the original composition, or alternatively, the volume percentage of the original composition. Another disclosed embodiment may include weight percentage substitution with the platinum group metal.

As described above, according to a disclosed embodiment, the alloys may be similar to those described above in which at least the nickel has been substituted with a platinum group metal, but in which the substituting metal is a refractory metal selected from the group consisting of silver, gold, hafnium, niobium, rhenium, tantalum, molybdenum, tungsten, zirconium, or combinations thereof. It is also conceivable that the substituting elements may be a combination of platinum group metals and refractory metals.

As described above, substitution may be on an atomic basis, a volumetric basis, or a weight basis. Reference Examples 25-69 below are based on weight percentage substitution of at least the nickel of an L-605 alloy and an MP-35N alloy. It will be understood that additional examples may substitute at least the nickel (and preferably at least some of the iron) of other cobalt-chromium alloys such as Elgiloy or Phynox. Additionally, it will be understood that substitution may alternatively proceed on an atomic or volumetric basis.

In order to better understand Reference Examples 25-69, the various binary phase diagrams of the included elements will be discussed.

When considering the cobalt-chromium alloy systems from the perspective of elimination of nickel from the alloys, substitution of another element for the nickel in the alloy is a natural approach. When considering which elements to utilize for substitution, the desired end goals come into play. The goal of such substitution is to create an alloy that has a greater radiopacity than the unmodified alloy (e.g., L-605 or MP-35N). In order to create an alloy with greater radiopacity, one or more elements of higher atomic weight and greater relative radiopacity (RR) need to be utilized.

In reviewing the periodic table of the elements, and the binary phase diagrams for the heavier transition metal and other precious metal elements in the periodic table, a number of elements stand out as appropriate for the substitution of nickel. Further substitution for some of the cobalt in the alloy(s) is possible with the desire for even better radiopacity of the alloy compared to L-605 and MP-35N. These elements include refractory metal elements such as Zr, Nb, Mo, Hf, Ta, W, and Re, and precious metal elements such as Ag and Au (which are herein grouped with the refractory elements for sake of simplicity).

To understand the reasoning behind these selections, the phase diagrams for the L-605 CoCr (Co-20Cr-15W-10Ni) and MP-35N (35Co-35Ni-20Cr-10Mo) alloys are first discussed below. When reviewing these phase diagrams they appear complex. The following points, however, work in favor of having alloys that have properties that allow them to be mechanically worked into a variety of useful forms, including stent tubing. For ease of understanding, elements less than 5 weight percent (e.g., manganese, iron, etc.) are ignored in this analysis, and presumed to be part of the cobalt concentration.

For the most part, the discussion will be limited to phases that are predicted to exist at the temperatures of interest, which include body temperature and room temperature. Also, when discussing disclosed compositions, all are considered "relative" compositions to relate them to the binary phase diagram unless otherwise indicated. For example, an alloy including 55 weight percent Co and 20 weight percent Cr ("55Co-20Cr") would translate to a relative composition of 73Co-27Cr in weight percent. All substituted alloy compositions below are discussed weight percent, unless otherwise indicated. Phase structures discussed include body centered cubic (BCC), face centered cubic (FCC), hexagonal close packed (HCP), orthorhombic, rhombohedral, tetragonal, and body centered tetragonal (BCT).

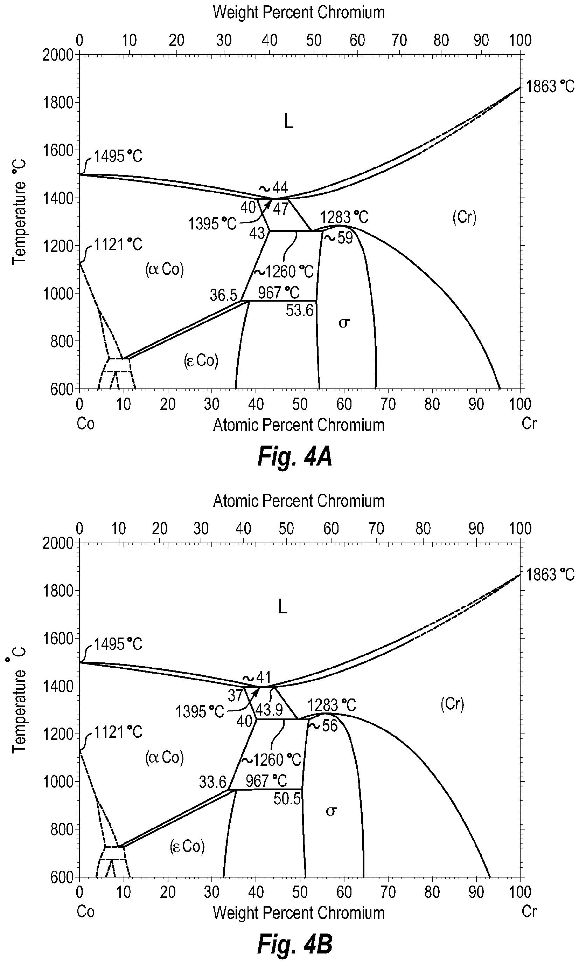

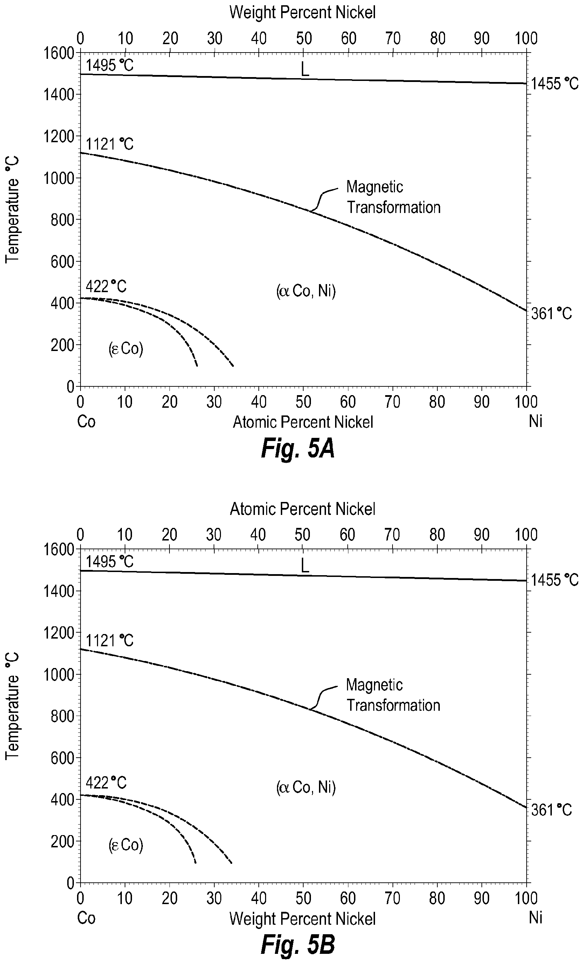

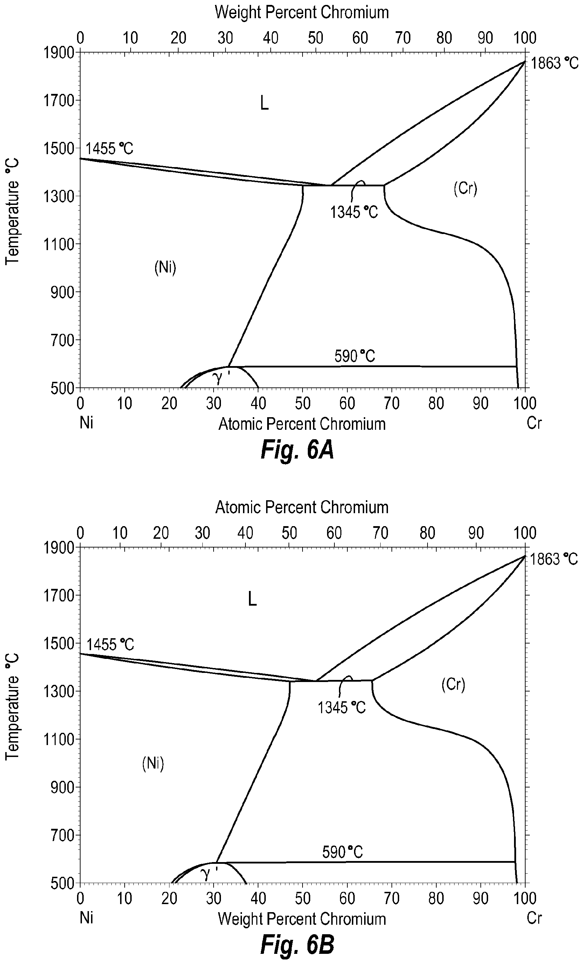

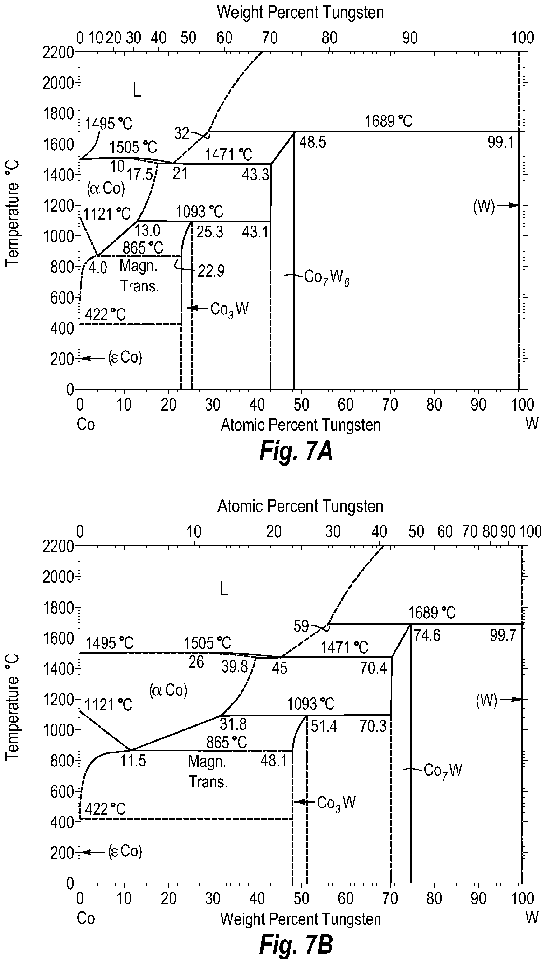

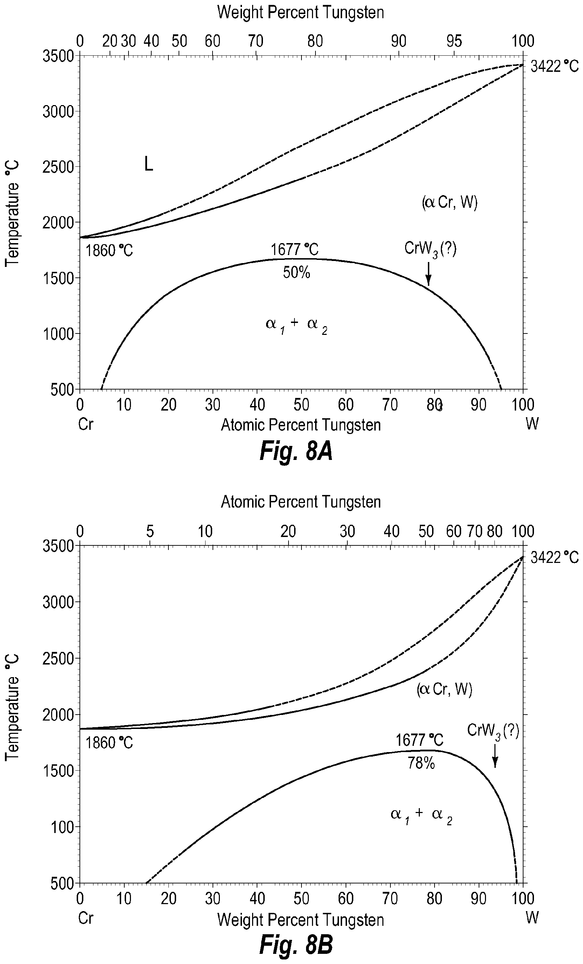

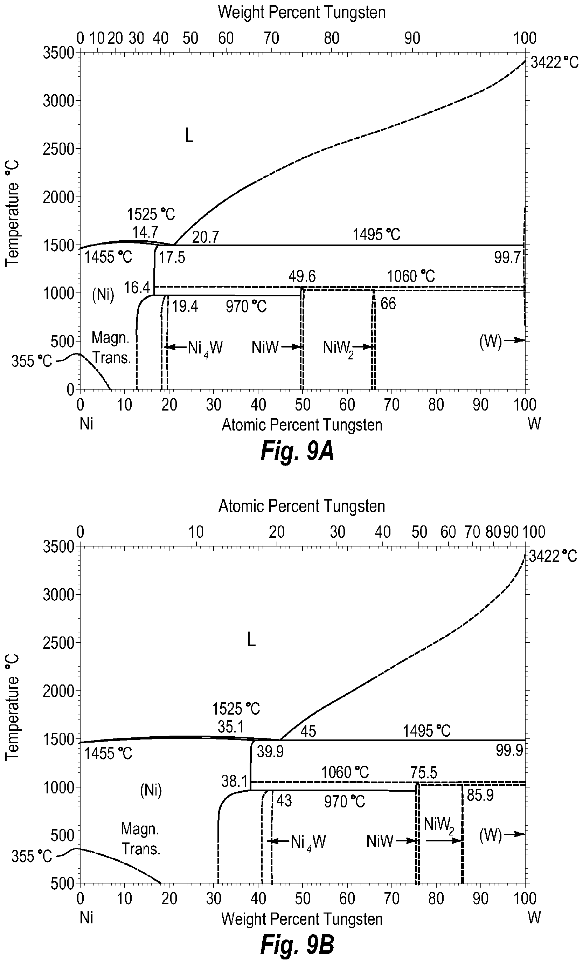

For L-605 CoCr:
- Co-Cr is a complex phase diagram with eutectic, eutectoid, peritectic, peritectoid, and congruent features. It includes at least three distinct phases and two eutectoid compositions. The tetragonal σ intermetallic peritectoid phase exists from about 51 to about 64Cr. L-605 falls into the HCP (εCo) phase area of the diagram (**FIGS. 4A-4B**).
- Co-Ni is a solid solution across the entire phase diagram, with an atomic structure change from HCP to FCC that occurs around 25Ni. L-605 falls into the (εCo) HCP phase area of the diagram (**FIGS. 5A-5B**).
- Cr-Ni is a phase diagram with eutectic, peritectoid, and allotropic transformation features. L-605 falls in the eutectoid body centered cubic (BCC)-orthorhombic area of the phase diagram (**FIGS. 6A-6B**).
- Co-W is an extremely complex phase diagram with eutectic, eutectoid, peritectoid, and allotropic transformation features. L-605 falls within the eutectoid HCP (εCo) - BCC (W) portion of the phase diagram (**FIGS. 7A-7B**).
- Cr-W is a phase diagram with a large miscibility gap starting around 15 weight percent W. L-605 resides in the miscibility gap (α1+α2) with two BCC phases (**FIGS. 8A-8B**).
- Ni-W is a complex phase diagram with eutectic and multiple peritectoid reactions. L-605 falls in the eutectoid BCT Ni4W - orthorhombic NiW phase combination (**FIGS. 9A-9B**).

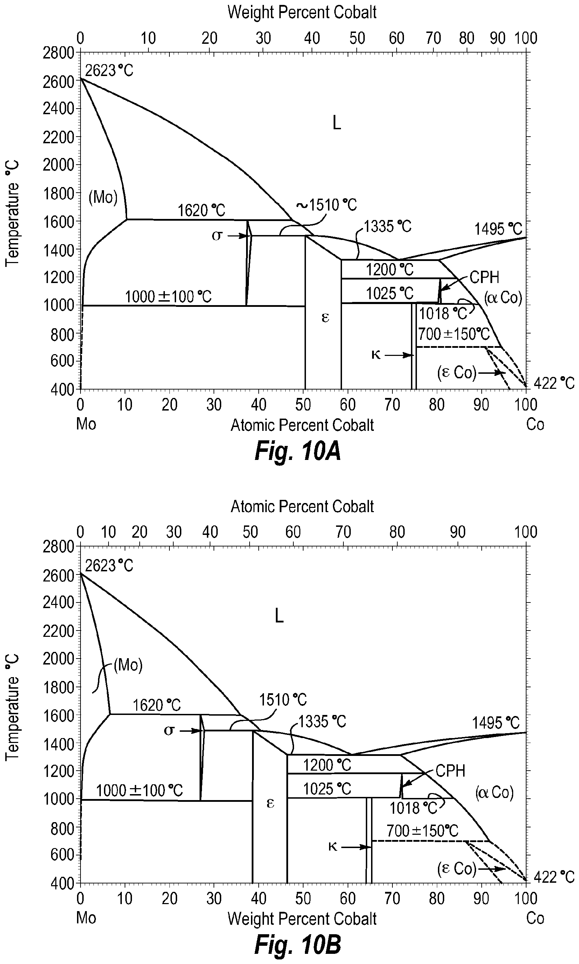

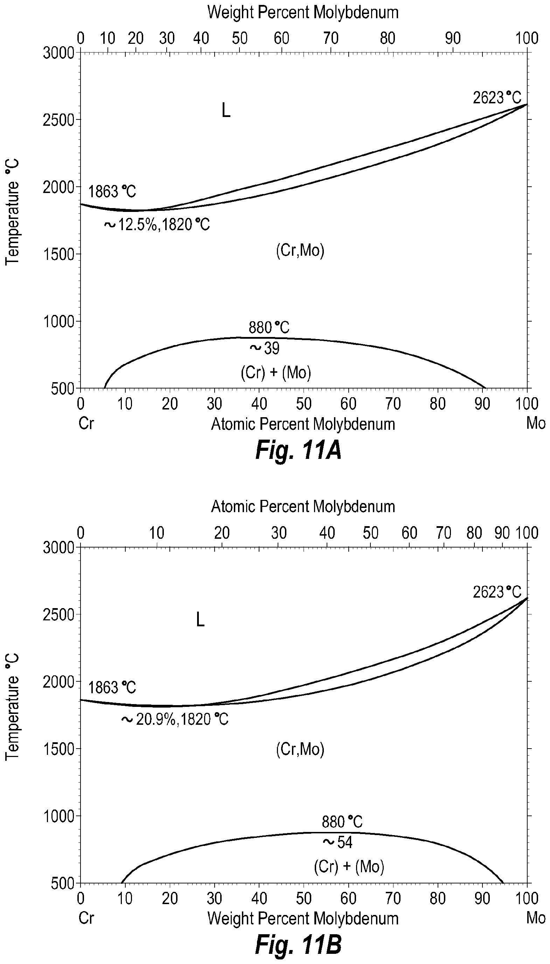

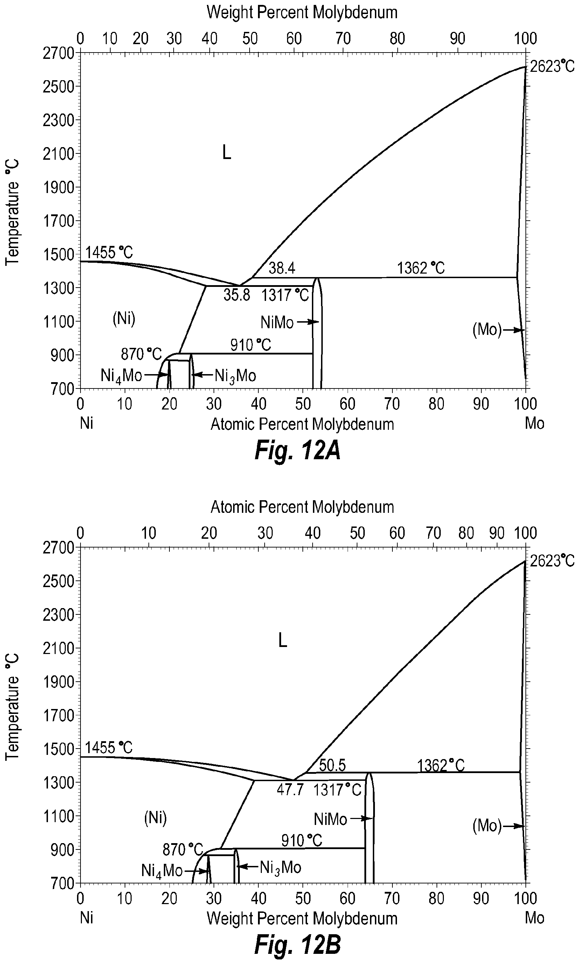

For MP-35N CoCr:
- Co-Cr - MP-35N falls into a eutectoid structure comprised of HCP (εCo) and tetragonal σ phases (**FIGS. 4A-4B**).
- Co-Ni - MP-35N falls in the FCC (αCo,Ni) portion of the phase diagram **(****FIGS. 5A-5B****).**
- Cr-Ni - MP-35N falls in the orthorhombic ordered phase regime of Ni₂Cr. It is possible that the presence of the other elements suppresses the formation of this ordered phase (**FIGS. 6A-6B**).
- Co-Mo is a complex phase diagram with multiple eutectic, peritectoid, and allotropic transformations. Similar to Co-W, two distinct ordered phases are formed, from about 33 to about 35Mo and from about 53 to about 61Mo. MP-35N falls in the region combining HCP κ and HCP (εCo) phases (**FIGS 10A-10B**).
- Cr-Mo exhibits a non-mixing region, called a miscibility gap, across most of the phase diagram. This indicates that distinct BCC phases of Cr and of Mo may exist without full mixing of the atoms among each other. MP-35N falls within this miscibility gap (Cr)+(Mo) region (**FIGS. 11A-11B**).
- Ni-Mo is a phase diagram that exhibits eutectic, peritectoid, and allotropic transformations. MP-35N falls in the FCC solid solution (Ni) regime of the phase diagram (**FIGS. 12A-12B**).

As can be seen from the complex phase diagrams described above, the equilibrium microstructures need not be single phase (i.e., fully solid solution) for an alloy composition to be selected that works well (as it is already established that L-605 and MP-35N work from a workability perspective, although with relatively lower radiopacity than desired).

The next discussion covers the elements listed above from the perspective of substituting for part or all of the Ni and possibly part of the Co in L-605 and MP-35N alloys. Other Co-Cr alloys (e.g., Elgiloy, Phynox) could similarly be substituted. In addition, an entirely new alloy composition may be developed independent of basing the initial substitution on existing alloys.

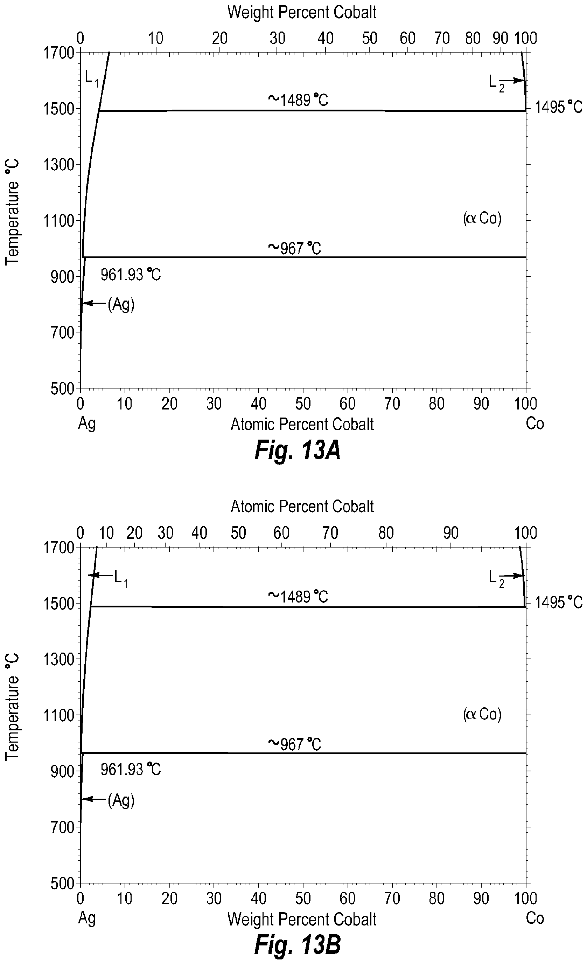

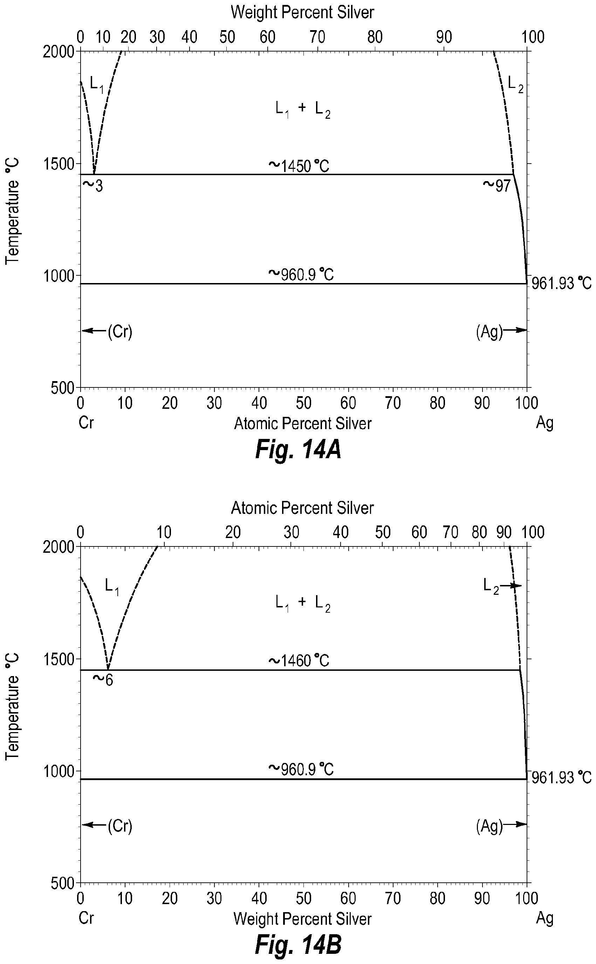

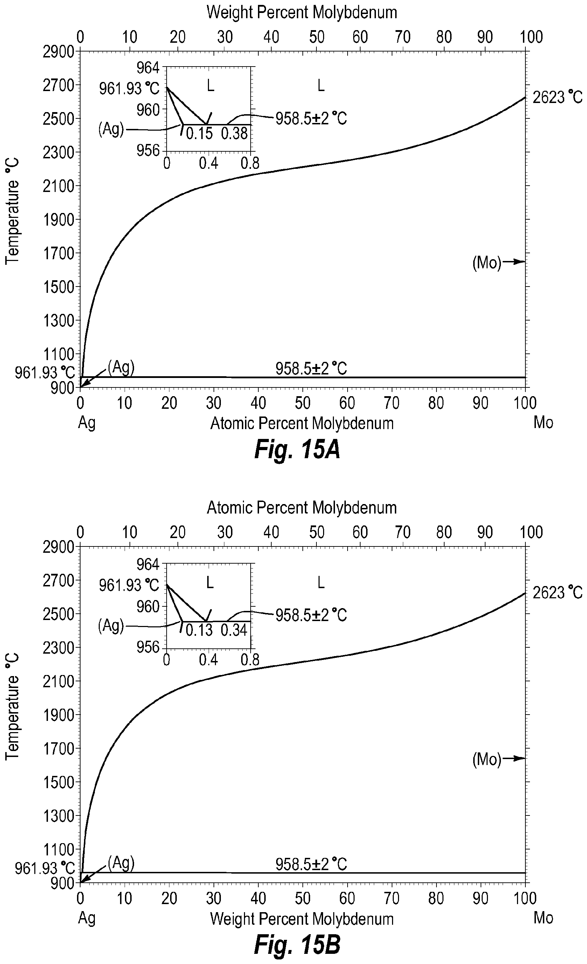

For an alloy containing Ag, considering different disclosed compositions that may include Mo and/or W:
- Ag-Co phase diagram shows a complete miscibility gap with two allotropic transformations. Any mixture at the temperatures of interest will simply include a mixture of FCC (Ag) and HCP (εCo) phases (**FIGS. 13A-13B**).
- Ag-Cr phase diagram shows a eutectic and a miscibility gap with two allotropic transformations. At the temperatures of interest, any mixture will simply include FCC (Ag) and BCC (Cr) phases regardless of composition (**FIGS. 14A-14B**).
- Ag-Mo is a phase diagram with a eutectic at the Ag side and an allotropic transformation. With the two elements being immiscible at the temperatures of interest, the material will simply include a mixture of FCC (Ag) and BCC (Mo) phases regardless of composition (**FIGS. 15A-****15B**).
- Ag-W is completely immiscible across all compositions. This indicates that the material will simply include a mixture of FCC (Ag) and BCC (W) phases.

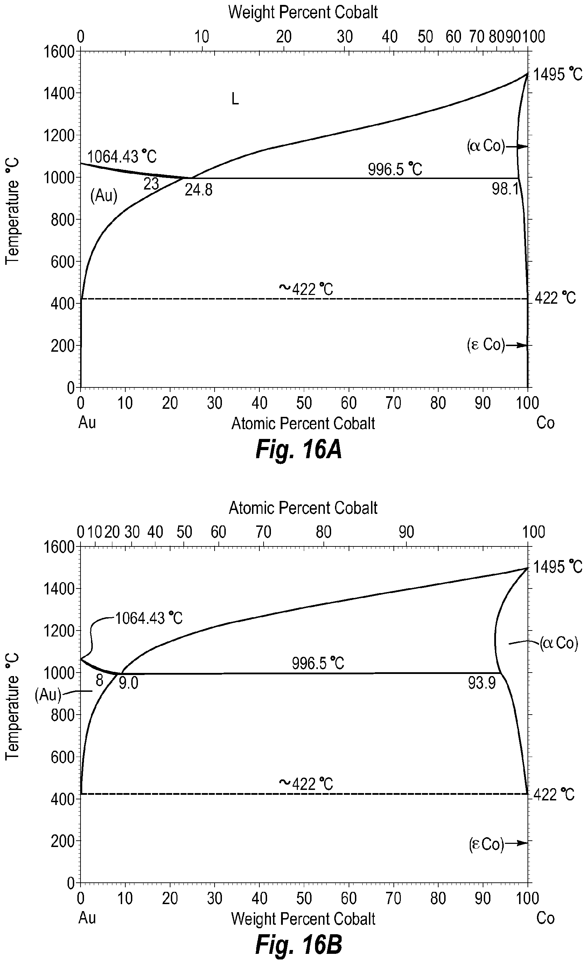

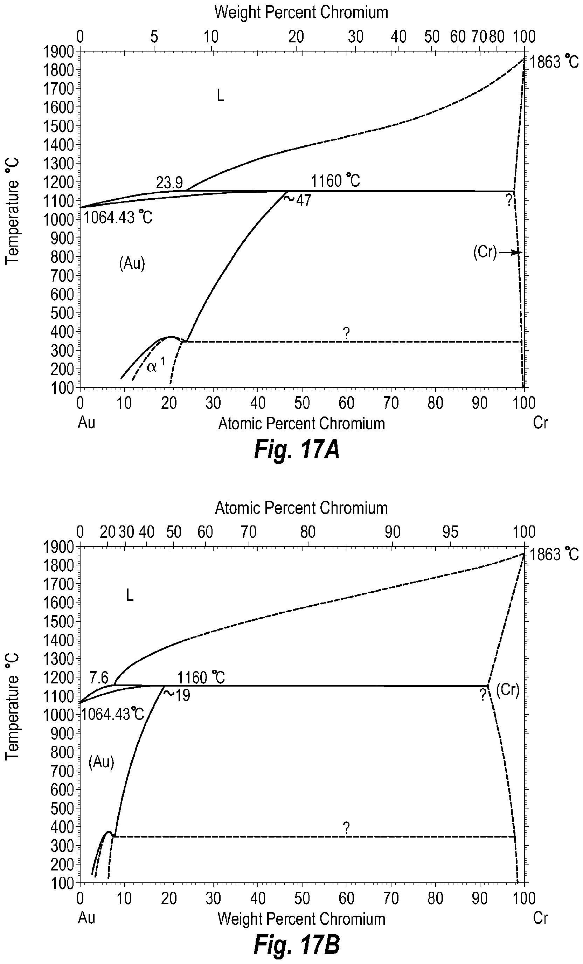

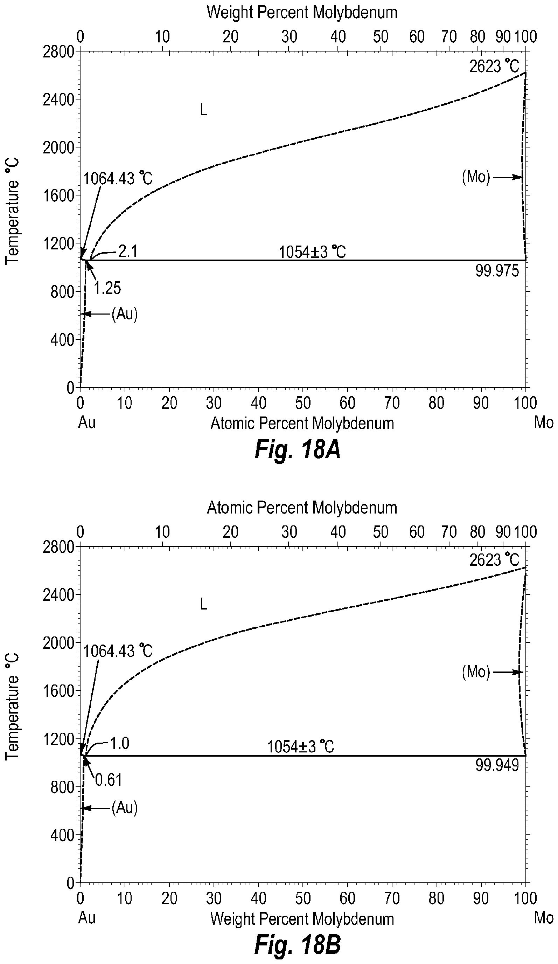

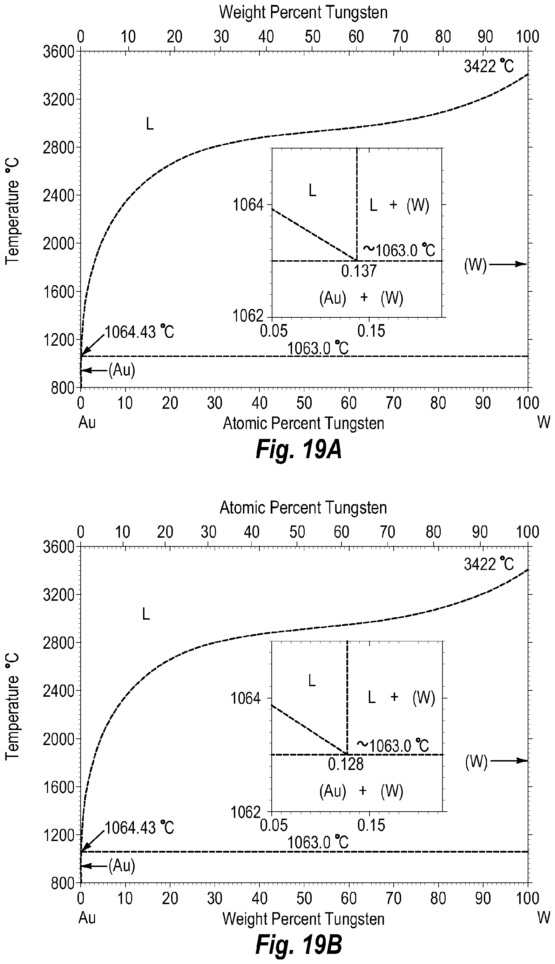

For an alloy containing Au, considering different disclosed compositions that may include Mo and/or W:
- Au-Co is a phase diagram with eutectic, eutectoid, and allotropic transformation features. The material will simply include a mixture of FCC (Au) and HCP (εCo) phases regardless of composition (**FIGS. 16A-****16B**).
- Au-Cr displays eutectic and ordering in the phase diagram. From about 1 to 6Au, an ordered Au₄Cr (α') BCT phase forms. Above this region the material will include a mixture of BCT α' and BCC (Cr) phases (**FIGS. 17A-17B**).
- Au-Mo is a eutectic across the entire phase diagram. This indicates that the material will include a mixture of FCC (Au) and BCC (Mo) phases regardless of composition (**FIGS. 18A-18B**).
- Au-W is a eutectic across the entire phase diagram. This indicates that the material will include a mixture of FCC (Au) and BCC (W) phases regardless of composition (**FIGS. 19A-19B**).

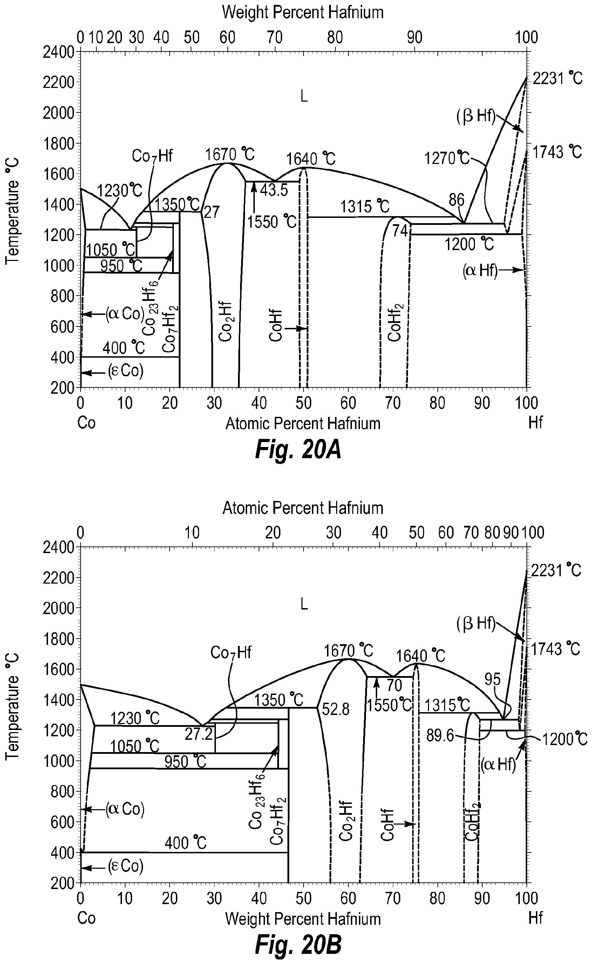

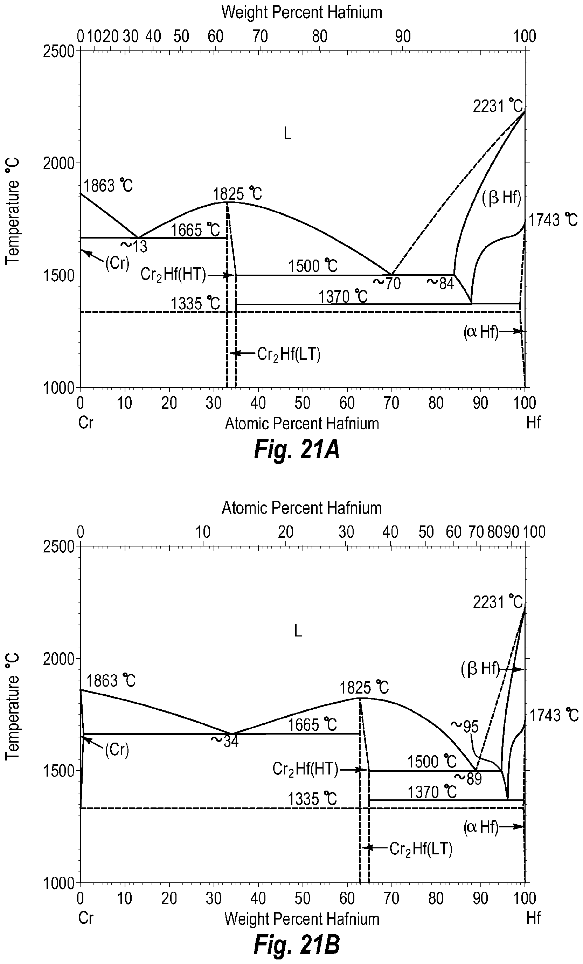

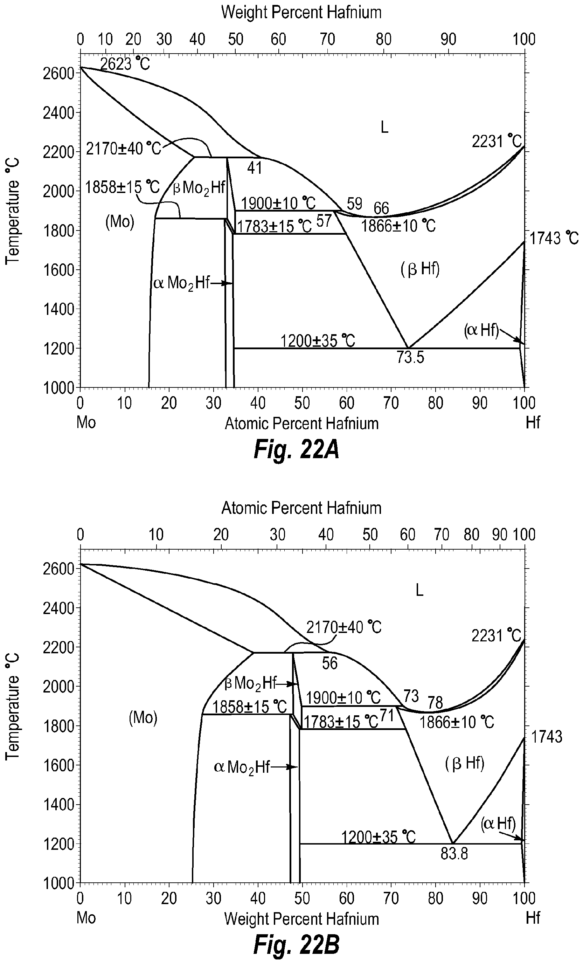

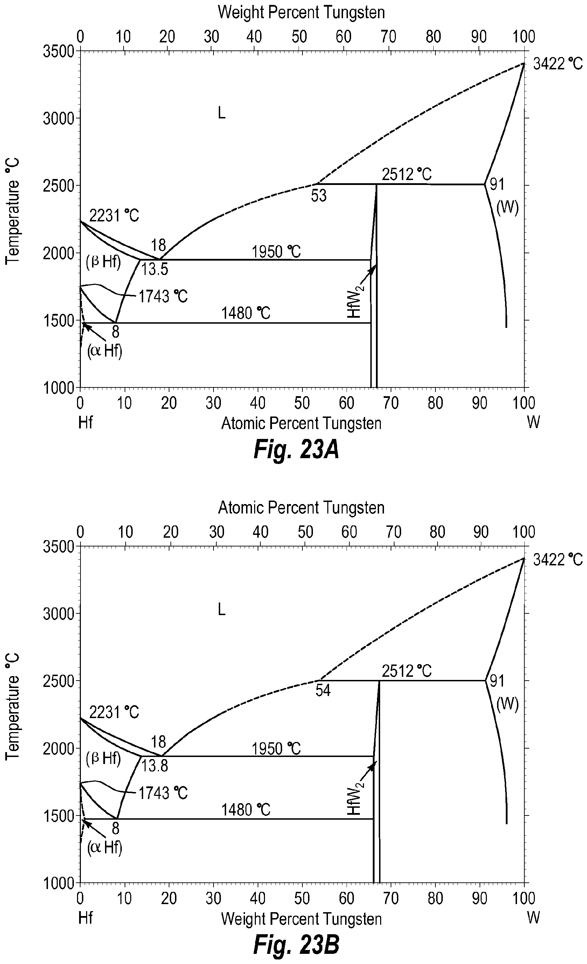

For an alloy containing Hf, considering different disclosed compositions that may include Mo and/or W:
- Co-Hf is a complex phase diagram with multiple eutectics, eutectoids, peritectics, congruent, and allotropic transformations. It is possible that this mixture becomes paramagnetic as early as about 47Hf. Up to this point, (e.g., about 46.5Hf), the microstructure will be a combination of HCP (εCo) and tetragonal Co₇Hf₂. From about 46.5 to about 56Hf, the microstructure is a mix of tetragonal Co₇Hf₂ and FCC Co₂Hf. From about 56 to about 62.5Hf is the ordered FCC Co₂Hf phase. From about 62.5 to about 74.5Hf the microstructure is a mixture of FCC Co₂Hf and cubic CoHf phases. The cubic CoHf phase sits from about 74.5 to about 75.5Hf. From about 75.5 to about 86Hf, the microstructure is a mix of the cubic CoHf and FCC CoHf₂ phases. The FCC CoHf₂ phase sits from about 86 to about 89Hf. Above about 89Hf is a mixture of FCC CoHf₂ and HCP (αHf) phases (**FIGS. 20A-20B**).
- Cr-Hf phase diagram shows two eutectics, one eutectoid, and one intermediate FCC Cr₂Hf phase from about 62 to about 65Hf. Below this intermediate phase the microstructure should include FCC Cr₂Hf plus (Cr) BCC phases. Above the Cr₂Hf phase, the eutectic should be a mix of Cr₂Hf and HCP (αHf) phases (**FIGS. 21A-21B**).
- Hf-Mo is a complex phase diagram with eutectic, eutectoid, peritectic, and allotropic phase transformations. Only one ordered FCC αMo₂Hf phase exists at the temperatures of interest, from about 47 to about 50 relative weight percent Hf. Up to about 25Hf, a BCC (Mo) solid solution exists. From about 25 to about 47Hf the microstructure would be a mix of BCC (Mo) solid solution and FCC αMo₂Hf. Above about 50Hf is a mix of this phase (i.e., FCC αMo₂Hf) and HCP (αHf) (**FIGS. 22A-22B**).
- Hf-W, similar to Hf-Mo, is a phase diagram with a eutectic, eutectoid, peritectic/ordered phase, with allotropic transformations. The one ordered FCC HfW₂ phase exists from about 66 to about 68Hf. Below this phase the microstructure is a mix of BCC (W) and FCC HfW₂. Above this phase is a mix of FCC HfW₂ and HCP (αHf) (**FIGS. 23A-23B**).

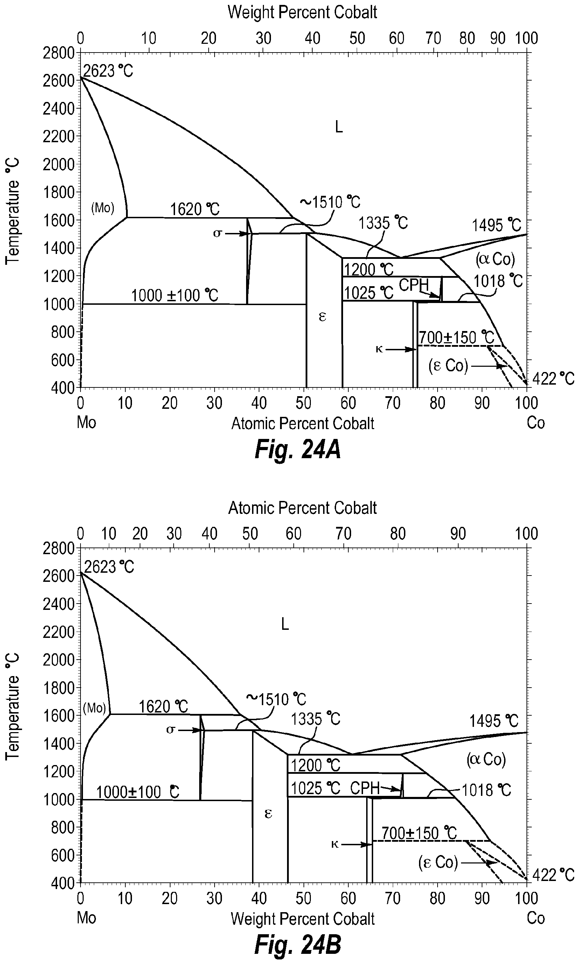

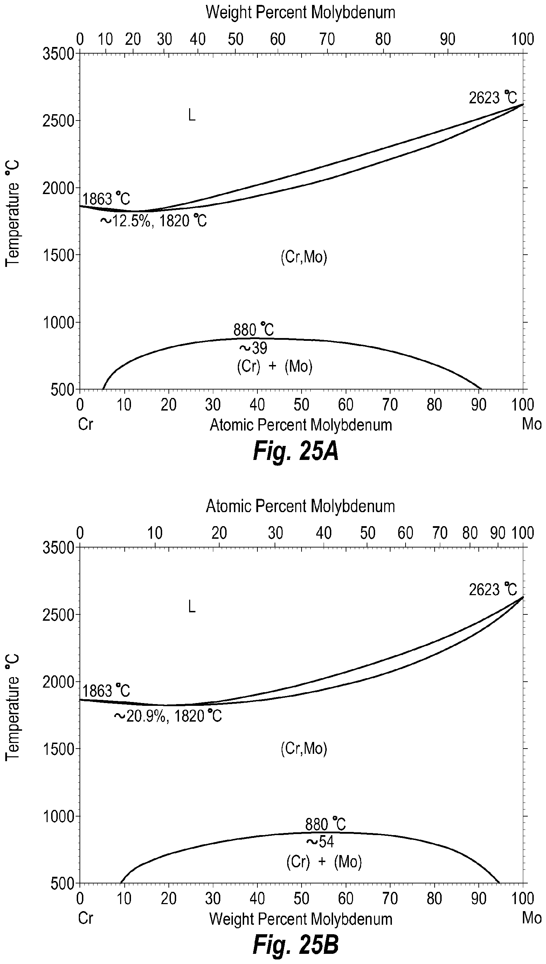

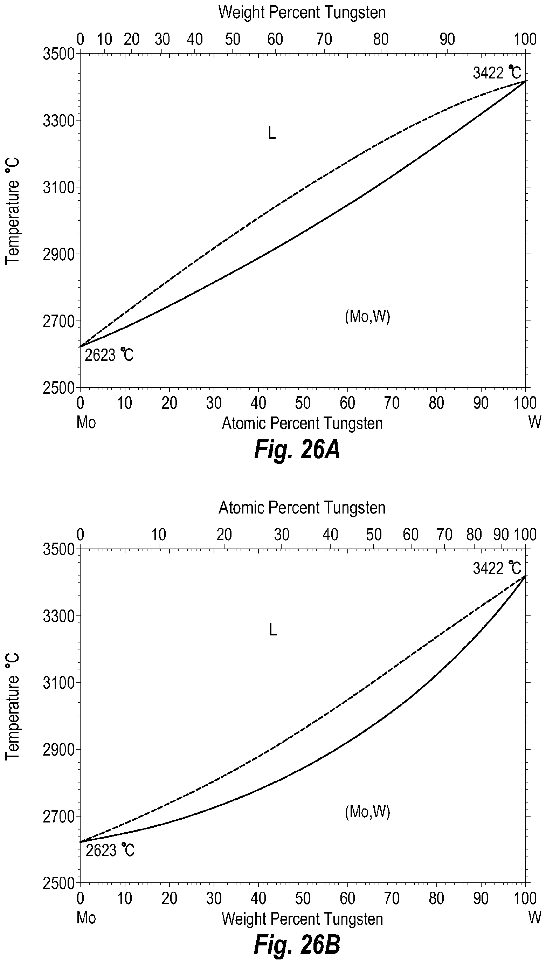

For a disclosed alloy containing Mo, the following may be observed:
- Co-Mo is a complex phase diagram with two eutectics, a peritectic, peritectoids/ordered phases, and allotropic transformations. Two distinct ordered phases exist at the temperature of interest. The HCP κ phase falls from about 34 to about 36Mo while the rhombohedral ε phase falls from about 53 to about 61Mo. Up to about 6Mo, HCP (εCo) appears to be the primary phase. From about 6 to about 34Mo is an HCP (εCo) and HCP κ mixture. From about 36 to about 53Mo a mixture of HCP κ and rhombohedral ε exist. Above about 61Mo is a mixture of rhombohedral ε and BCC (Mo) (**FIGS. 24A-24B**).
- Cr-Mo phase diagram exhibits a miscibility gap from about 9Mo through about 95Mo. Based on the shape of the miscibility curve at 500°C, it is likely that this extends to close to 2Mo through 99+Mo at room/body temperature. This indicates that the microstructure will be a mixture of (Cr) + (Mo) BCC phases (**FIGS. 25A-25B**).
- Mo-W phase diagram exhibits a continuous BCC solid solution (Mo,W) regardless of composition (**FIGS. 26A-26B**).

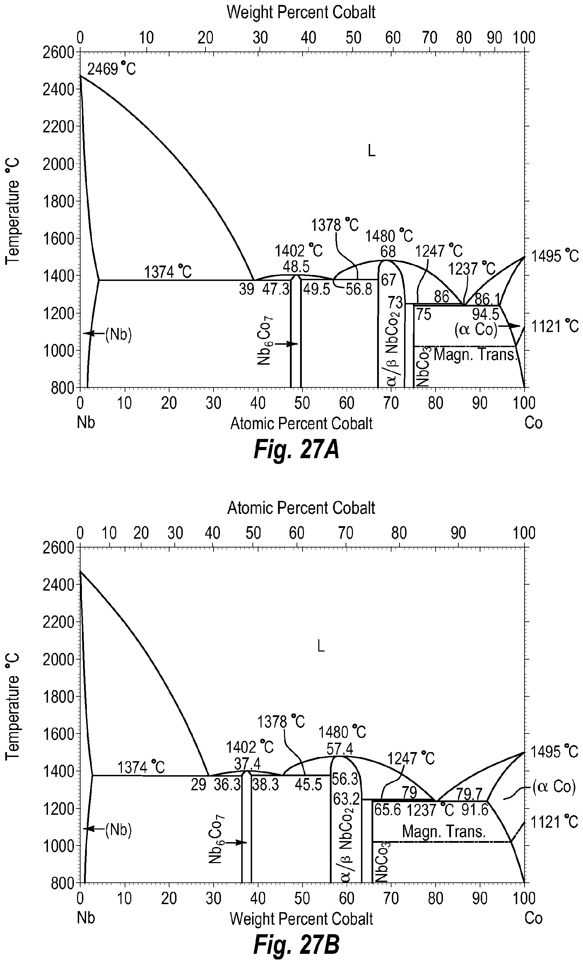

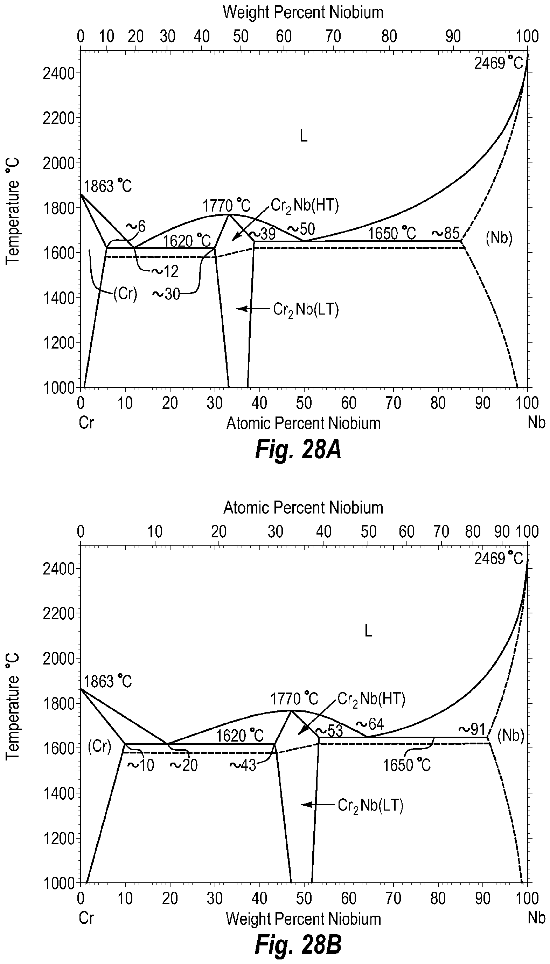

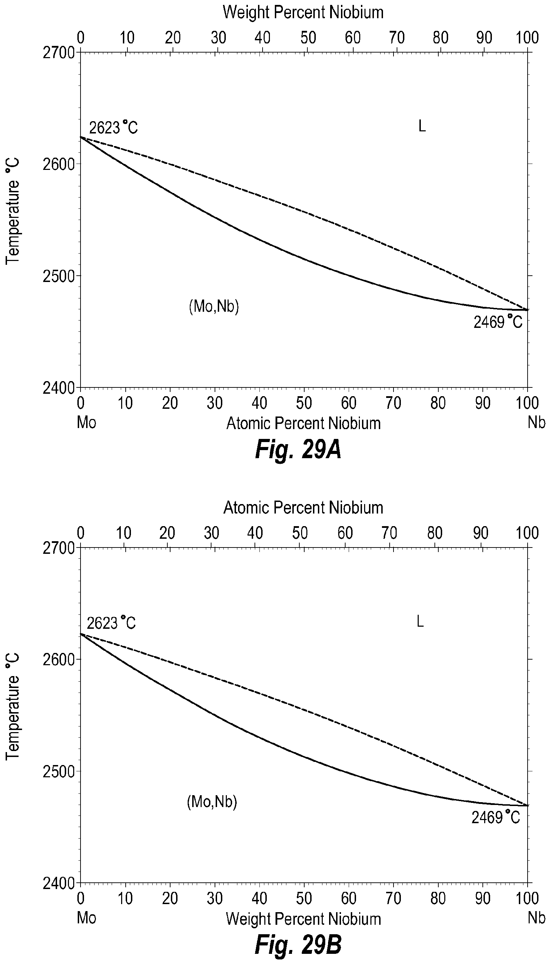

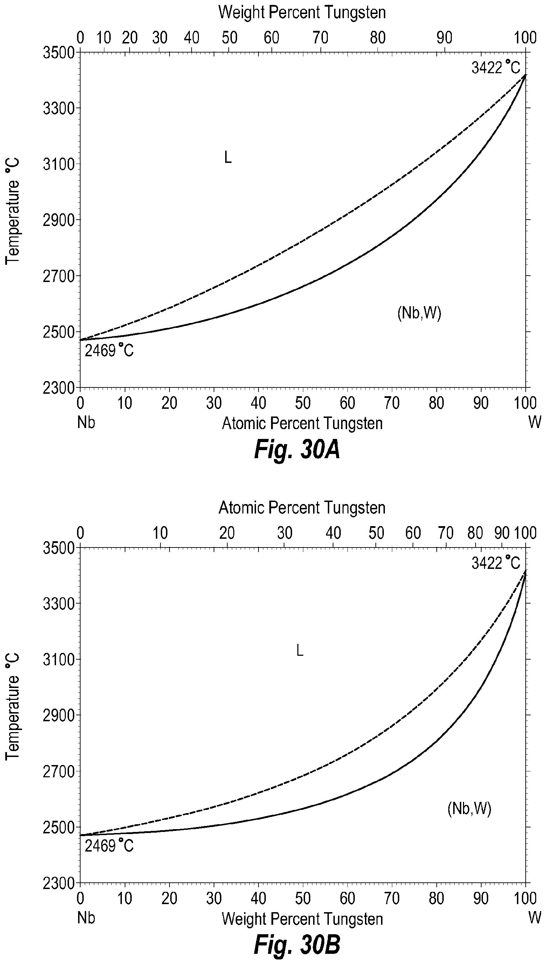

For a disclosed alloy containing Nb, considering different compositions that may include Mo and/or W':
- Co-Nb is a complex phase diagram with multiple eutectics and congruent ordered phases, including one phase specific to a single composition. These phases include an HCP NbCo₃ phase at about 34.5Nb, an FCC αNbCo₂ phase from about 37 to about 44Nb, and rhombohedral Nb₆Co₇ from about 61 to about 64Nb. Up to about 34.5Nb most likely is a mixture of HCP (εCo) and HCP NbCo₃. From about 34.5 to about 37Nb is a mixture of HCP NbCo₃ and FCC αNbCo₂ phases. From about 44 to about 61Nb is a mixture of FCC αNbCo₂ and rhombohedral Nb₆Co₇. Above about 64Nb is a mixture of rhombohedral Nb₆Co₇ and BCC (Nb) phases (**FIGS. 27A-27B**).
- Cr-Nb shows two eutectics and a congruent ordered phase around the middle of the phase diagram. The ordered FCC Cr₂Nb phase falls from about 47 to about 52Nb. Up to about 47Nb exists a mix of BCC (Cr) and FCC Cr₂Nb phases. Above about 52Nb exists a mix of FCC Cr₂Nb and BCC (Nb) phases (**FIGS. 28A-28B**).
- Mo-Nb phase diagram shows a single solid solution BCC (Mo,Nb) phase across the entire range of compositions (**FIGS. 29A-29B**).
- Nb-W phase diagram shows a single solid solution BCC (Nb,W) phase across the entire range of compositions (**FIGS. 30A-30B**).

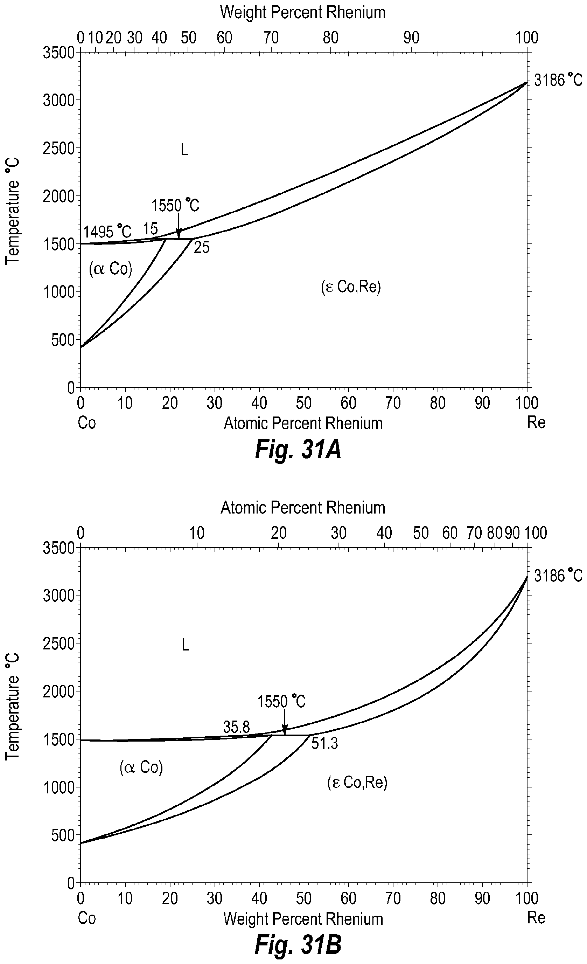

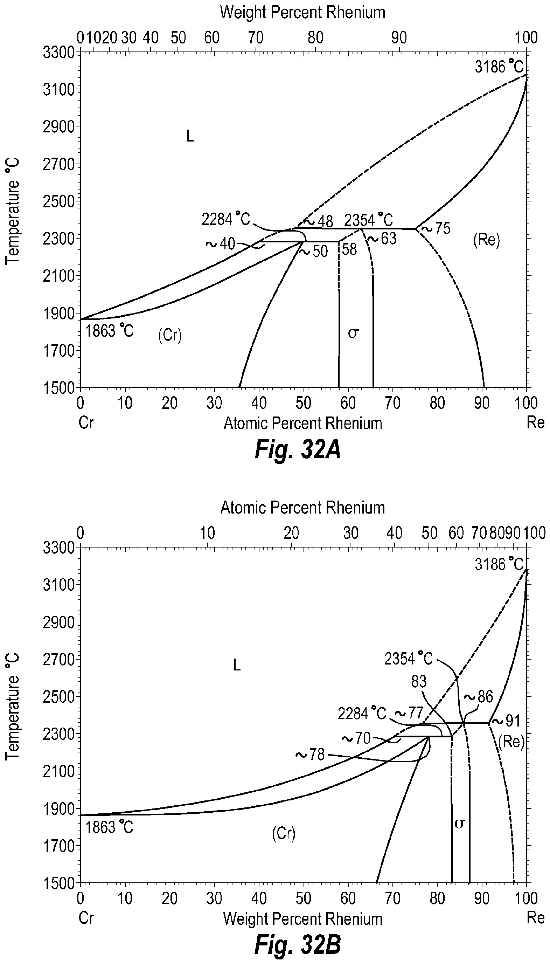

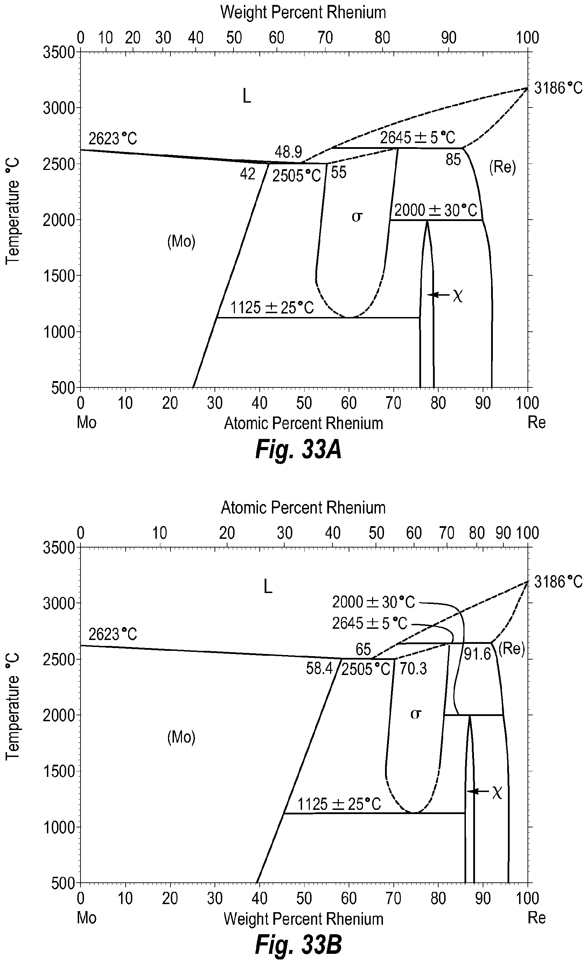

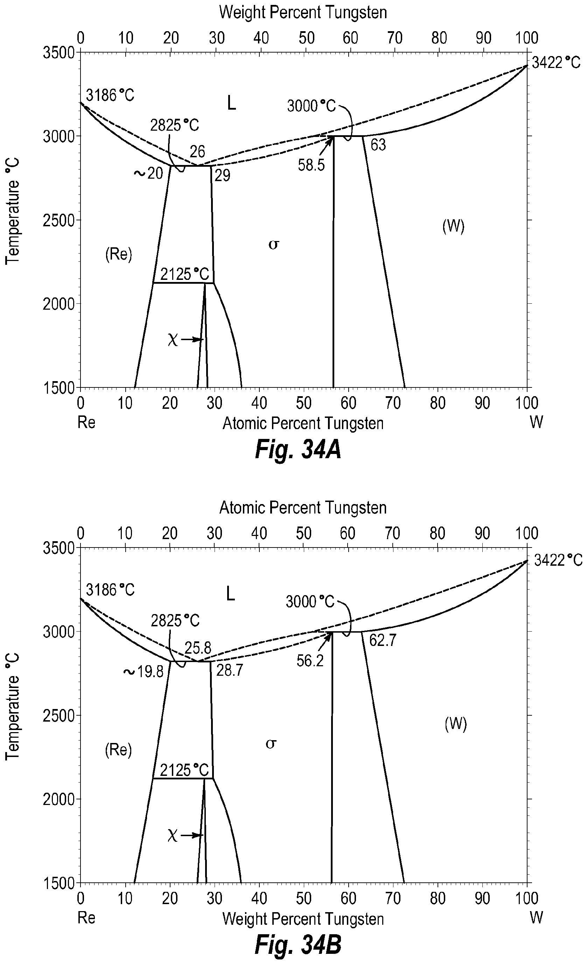

For a disclosed alloy containing Re, considering different compositions that may include Mo and/or W:
- Co-Re forms an HCP solid solution phase of (εCo, Re) across the entire phase diagram at the temperatures of interest (**FIGS. 31A-31B**).
- Cr-Re is a phase diagram with eutectic and peritectic reactions. Up to about 66Re the microstructure is primarily BCC (Cr). From about 66 to about 83Re is a mixture of BCC (Cr) and σ phases. A tetragonal ordered phase (the σ phase, also written as Cr₂Re₃) forms from about 83 to about 88Re. Above about 88Re through about 97Re, a mixture of σ and HCP (Re) phases exist. Above about 97Re, the dominant microstructure is HCP (Re) (**FIGS. 32A-32B**).
- Mo-Re phase diagram is a combination of a eutectic, congruent, peritectoid, and allotropic phase transformation. Up to about 38Re a BCC (Mo) solid solution phase exists. From about 28Re to about 86Re is a mix of BCC (Mo) and BCC χ phases. From about 86 to about 88Re is the ordered BCC χ phase. From about 88 to about 95.5Re a combination of BCC χ and HCP (Re) phases exist. Above about 96 relative weight percent the structure is a solid solution HCP (Re) phase (**FIGS. 33A-****33B**).
- Re-W is a complex phase diagram with eutectic, peritectoid, and congruent features. Up to about 28Re is a solid solution BCC (W) phase. From about 28 to about 44Re is a combination of BCC (W) and tetragonal σ phases. From about 44 to about 64Re is the ordered tetragonal σ phase. From about 64 to about 72Re is a combination of tetragonal σ and BCC χ phases. From about 72 to about 74Re is the ordered BCC χ phase. From about 74 to about 88Re is a combination of BCC χ and HCP (Re) phases. Above about 88Re, a HCP solid solution phase with W exists (**FIGS. 34A-34B**).

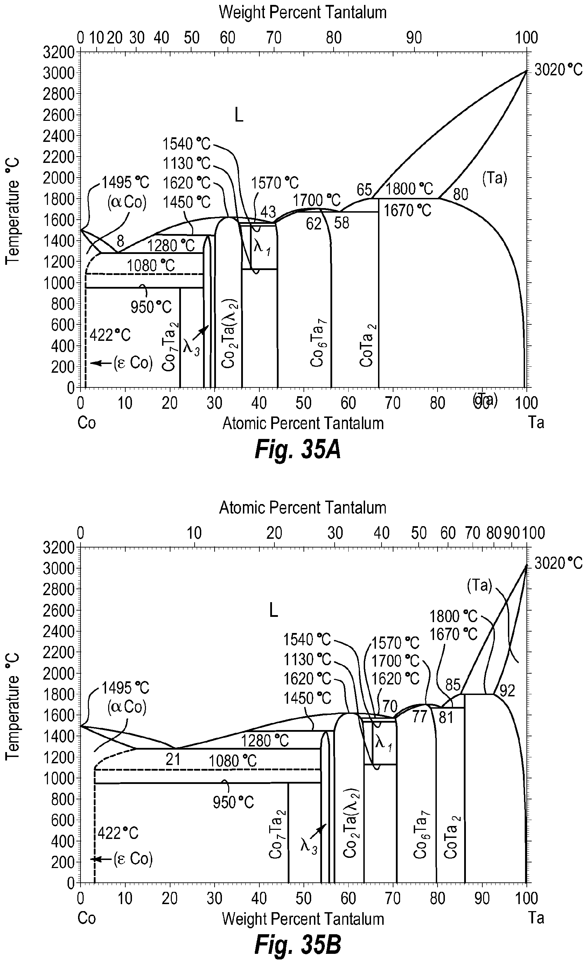

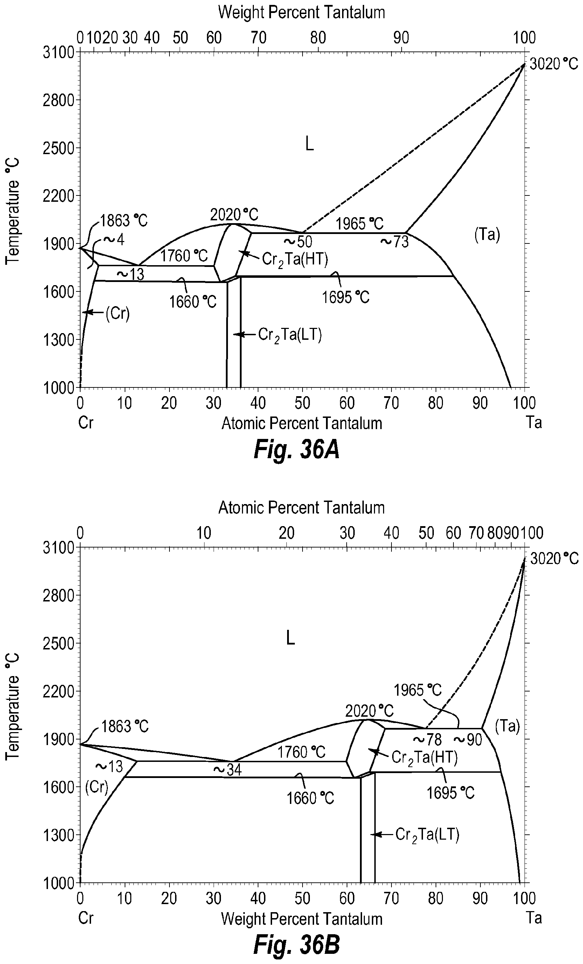

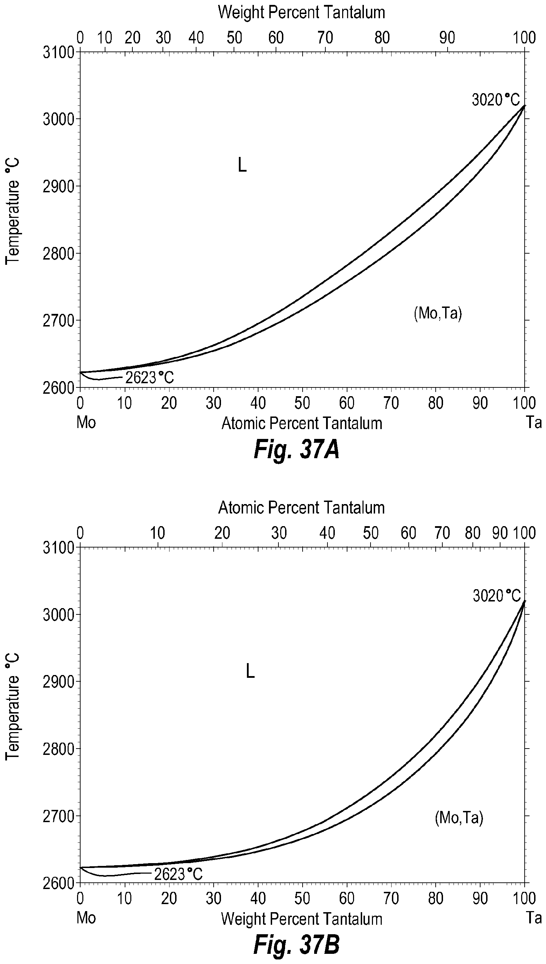

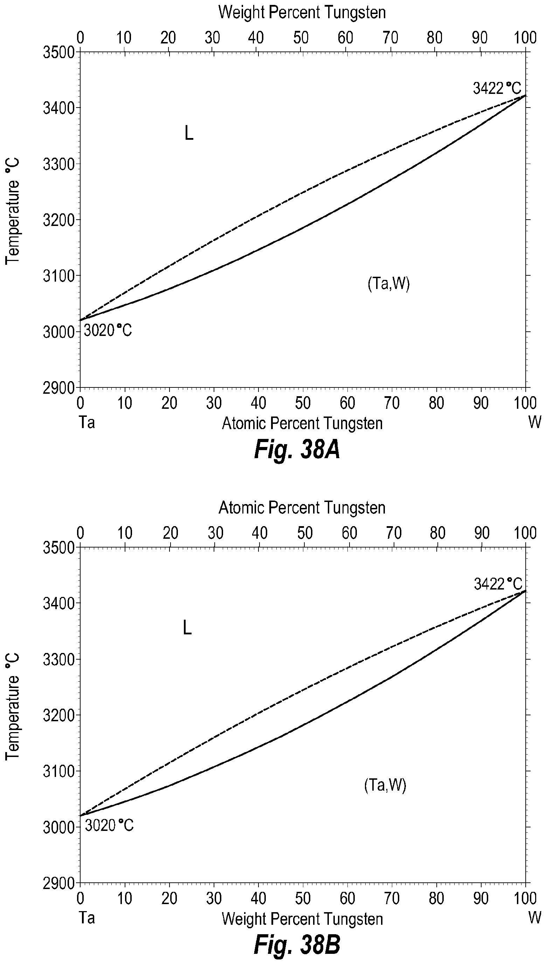

For a disclosed alloy containing Ta, considering different compositions that may include Mo and/or W:
- Co-Ta is a fairly complex phase diagram that exhibits five ordered phases, three of which are peritectic. Up to about 3Ta is HCP (εCo). From about 3 to about 46.5Ta is a mixture of HCP (εCo) and Co₇Ta₂ (unknown microstructure). The Co₇Ta₂ phase sits at about 46.5Ta. From about 46.5 to about 54Ta is a combination of Co₇Ta₂ and HCP λ₃ phases. The HCP λ₃phase sits from about 54 to about 55.5Ta. From about 55.5 to 57Ta is a mixture of HCP λ₃ and FCC Co₂Ta (λ₂) phases. From about 57 to about 63Ta is the FCC Co₂Ta (λ₂) phase. From about 63 to about 70.5Ta is mixture of FCC Co₂Ta (λ₂) and rhombohedral Co₆Ta₇ phases. From about 70.5 to 79.5Ta is the Co₆Ta₇ rhombohedral phase. From about 79.5 to about 86Ta is a mixture of rhombohedral Co₆Ta₇ and BCT CoTa₂ phases. A BCT CoTa₂ phase sits at around 86Ta. Above about 85Ta is a mixture of BCT CoTa₂ and BCC (Ta) phases (**FIGS. 35A-35B**).
- Cr-Ta is a phase diagram with a single intermediate phase that forms a eutectic with each of the (Cr) and (Ta) solid solutions. The intermediate Laves phase formation of FCC Cr₂Ta sits from about 63 to about 66.5Ta. Up to about 63Ta, the microstructure is a mixture of BCC (Cr) and FCC Cr₂Ta. Above about 66.5Ta is a mixture of FCC Cr₂Ta and BCC (Ta) **(****FIGS. 36A-36B****).**
- Mo-Ta forms a continuous BCC (Mo,Ta) solid solution at all compositions (**FIGS. 37A-37B**).
- Ta-W forms a continuous BCC (Ta,W) solid solution at all compositions (**FIGS. 38A-38B**).

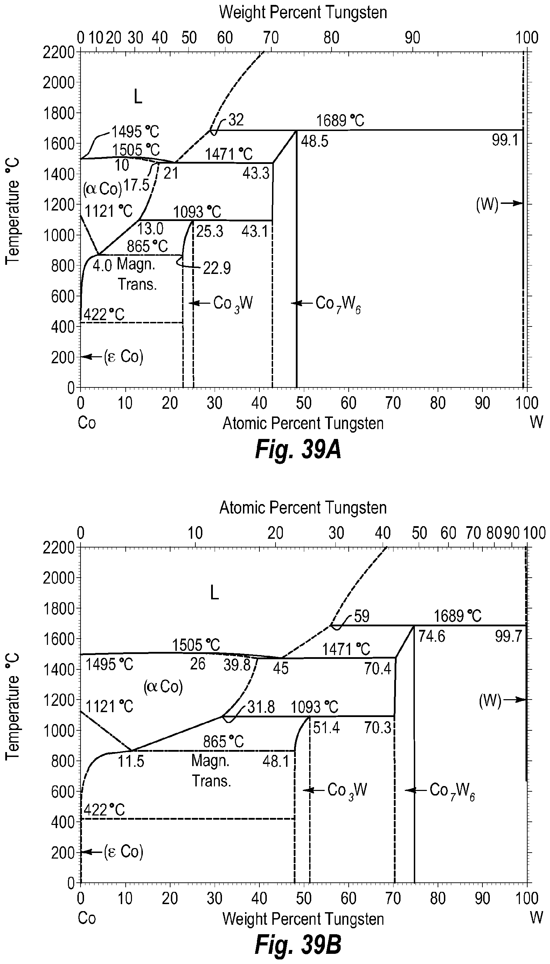

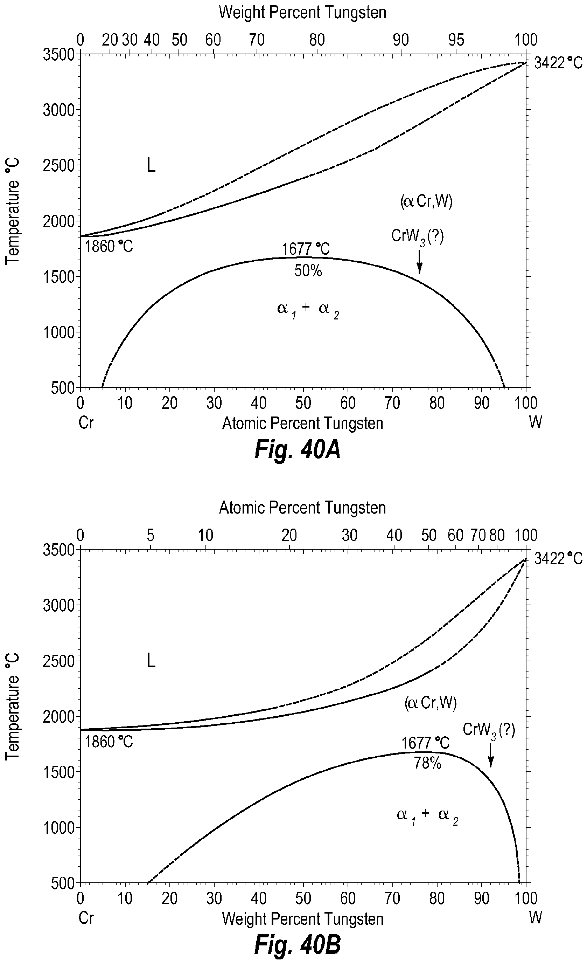

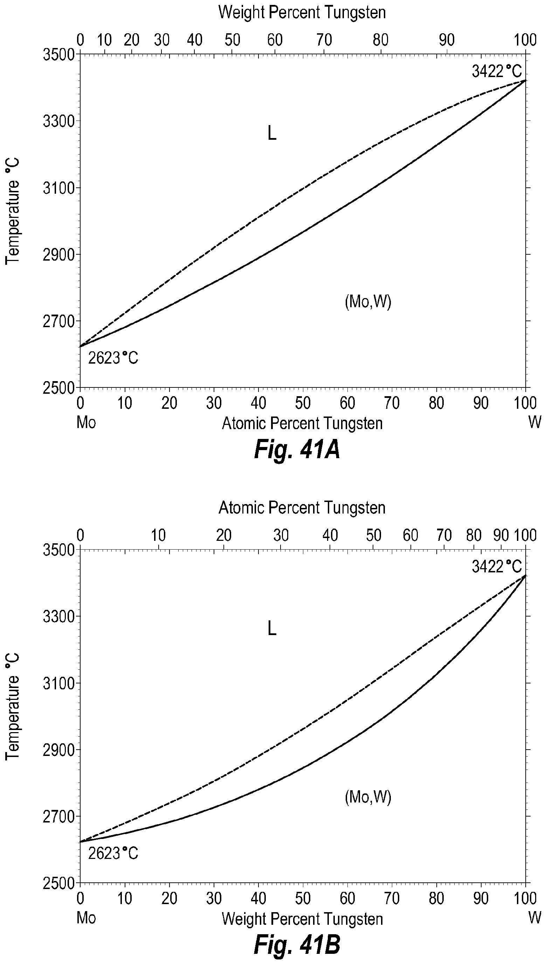

For a disclosed alloy containing W, the following may be observed:
- Co-W is an extremely complex phase diagram with eutectic, eutectoid, peritectoid, and allotropic transformations featuring two different ordered phases at the temperatures of interest. HCP Co₃W forms from about 48 to about 51.5W. Rhombohedral Co₇W₆ forms from about 70 to about 74.5W. Up to about 48W the microstructure is a mixture of HCP (εCo) and HCP Co₃W phases. From about 51.5 to about 70W, the microstructure is a mixture of HCP Co₃W and rhombohedral Co₇W₆ phases. Above about 74.5W is a mixture of rhombohedral Co₇W₆ and BCC (W) phases. Based on the L-605 information and the similar MP-35N Co-Mo information, alloys that fall outside of the ordered phases allow for a workable alloy (**FIGS. 39A-39B**).
- Cr-W is a phase diagram with a large miscibility gap starting around 15 weight percent W. Note that the miscibility gap most likely starts closer to 0-1W at the temperatures of interest, based on the shape of the curve. Below the start of the miscibility gap is a solid solution of BCC (Cr,W). Within the miscibility gap the two BCC (α₁+α₂) Cr and W phases exist (**FIGS. 40A-40B**).
- Mo-W forms a continuous BCC (Mo,W) solid solution at all compositions (**FIGS. 41A-41B**).

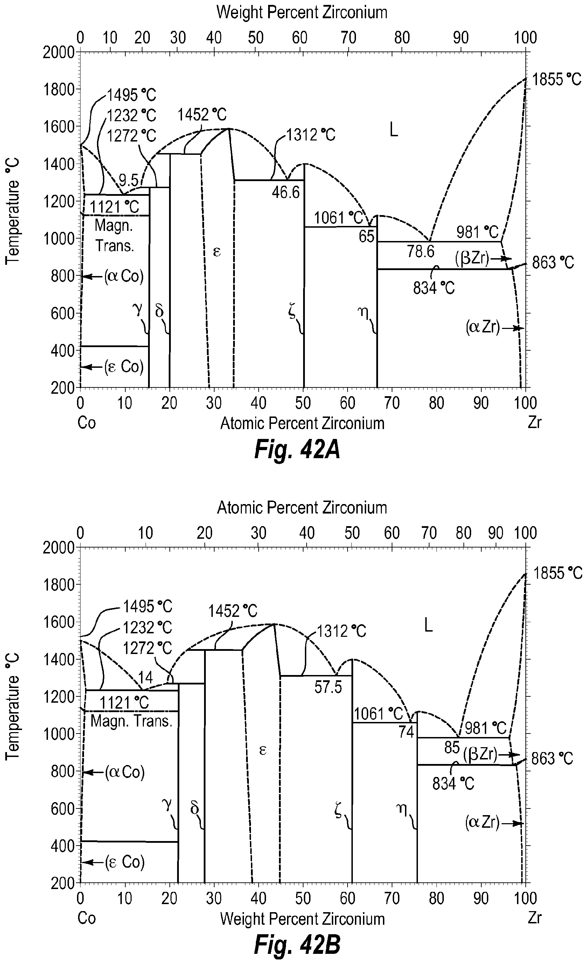

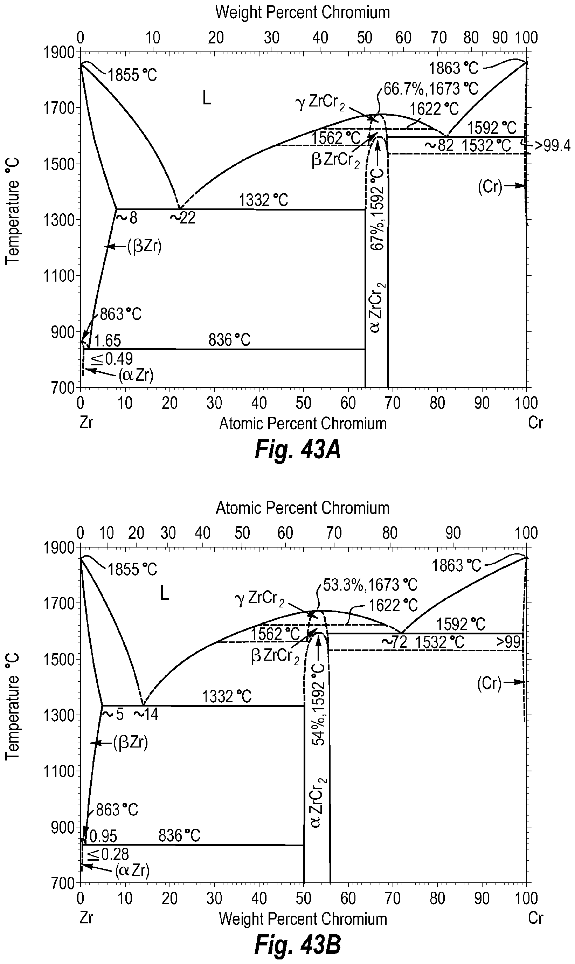

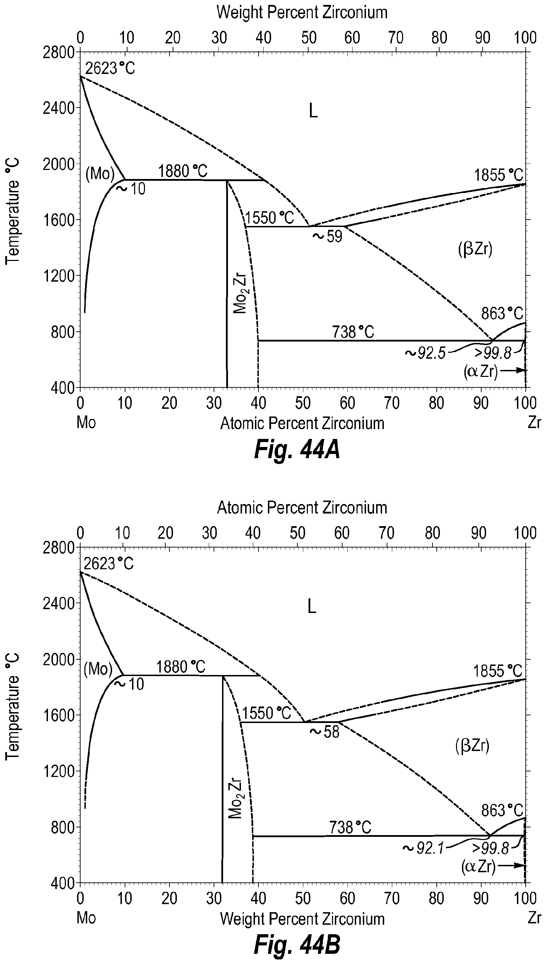

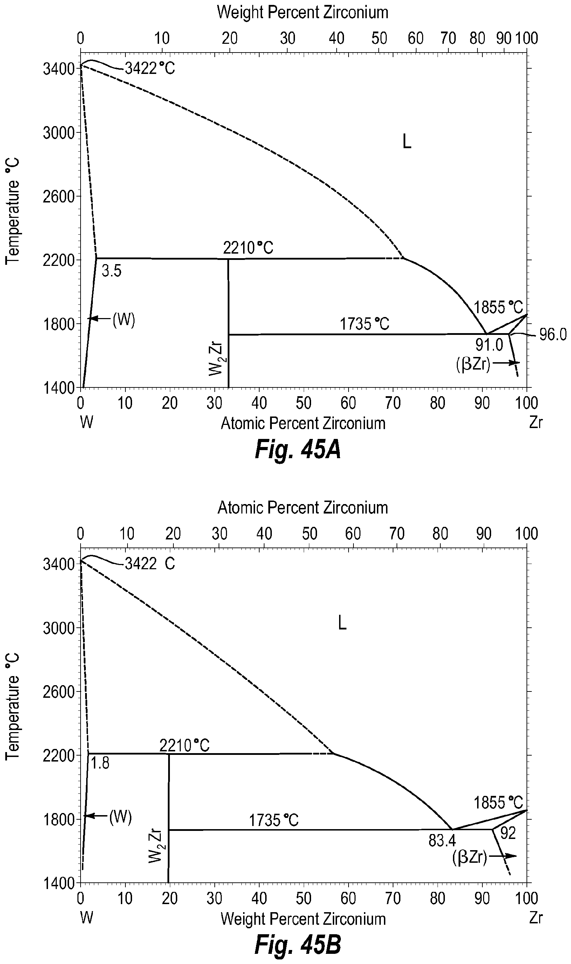

For a disclosed alloy containing Zr, the following may be observed:
- Co-Zr is a complex phase diagram with eutectic, eutectoid, peritectic, and allotropic transformations. The formation of five different ordered phases are shown. The γ phase, of unknown structure, is at about 22Zr. The FCC δ phase appears at about 28Zr. The FCC ε phase appears roughly from about 39 to about 45Zr. The cubic ξ phase appears at about 61Zr. The BCT η phase appears at about 75.5Zr. Up to about 22Zr, the microstructure is a combination of HCP (εCo) and FCC γ phases. From about 22 to about 28Zr is a mixture of FCC γ and FCC δ phases. From about 28 to about 39Zr is a mixture of FCC δ and FCC ε phases. From about 45 to about 61Zr is a mixture of FCC ε and cubic ξ phases. From about 61 to about 75.5Zr is a mixture of cubic ξ and BCT η phases. From about 75.5 to about 99Zr is a mixture of BCT η phase and HCP (αZr) phases. Above 99Zr is a solid solution HCP (αZr) phase (**FIGS. 42A-****42B**).
- Cr-Zr is a complex phase diagram with eutectic, eutectoid, congruent and allotropic transformation features. One intermediate ordered phase exists at the temperature of interest. That FCC αZrCr₂ phase falls from about 44 to about 50Zr. Up to about 44Zr is a combination of BCC (Cr) and FCC αZrCr₂ phases. Above about 50Zr is a combination of FCC αZrCr₂ and HCP (αZr) phases (**FIGS. 43A-43B**).
- Mo-Zr is a phase diagram with eutectic, peritectic, eutectoid, and allotropic transformation features. A single ordered FCC Mo₂Zr phase exists from about 32 to about 39Zr. Up to about 32Zr is a mixture of BCC (Mo) and FCC Mo₂Zr phases. Above about 39Zr is a mixture of FCC Mo₂Zr and HCP (αZr) phases (**FIGS. 44A-44B**).
- W-Zr is a phase diagram with eutectic features, showing a single ordered FCC W₂Zr phase at around 20Zr. Up to about 20Zr is a mixture of BCC (W) and FCC W₂Zr phases. Above about 20Zr is a mixture of FCC W₂Zr and HCP (αZr) phases (**FIGS. 45A-45B**).

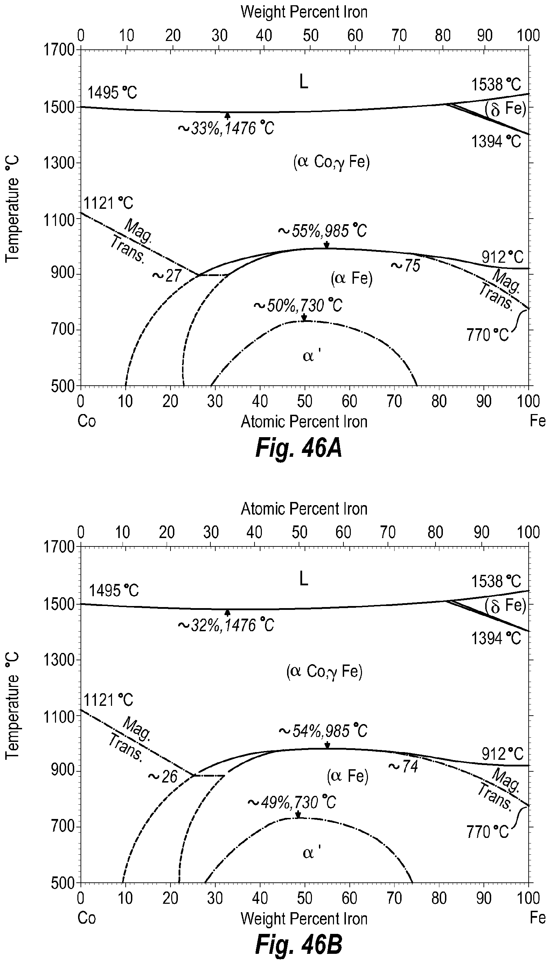

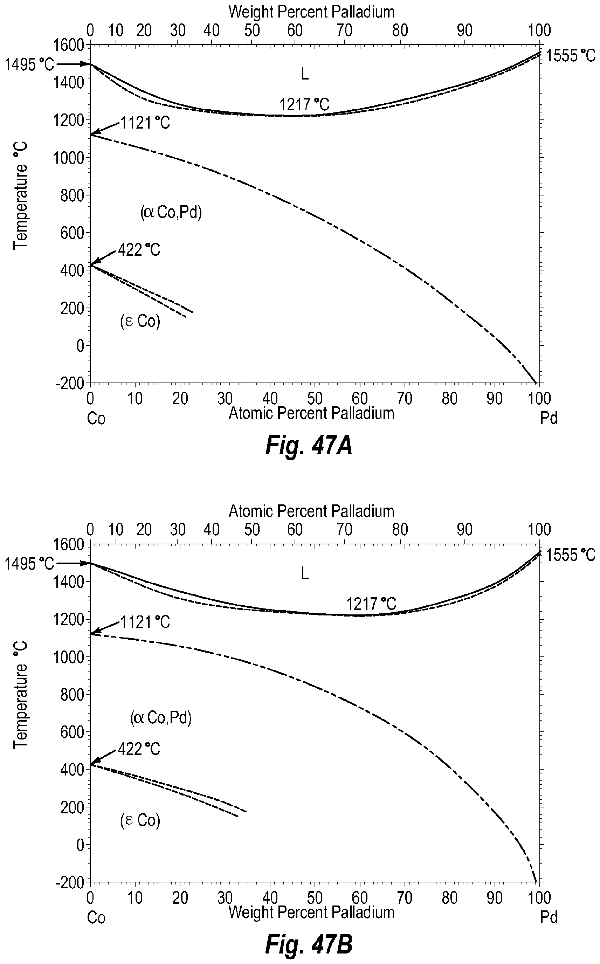

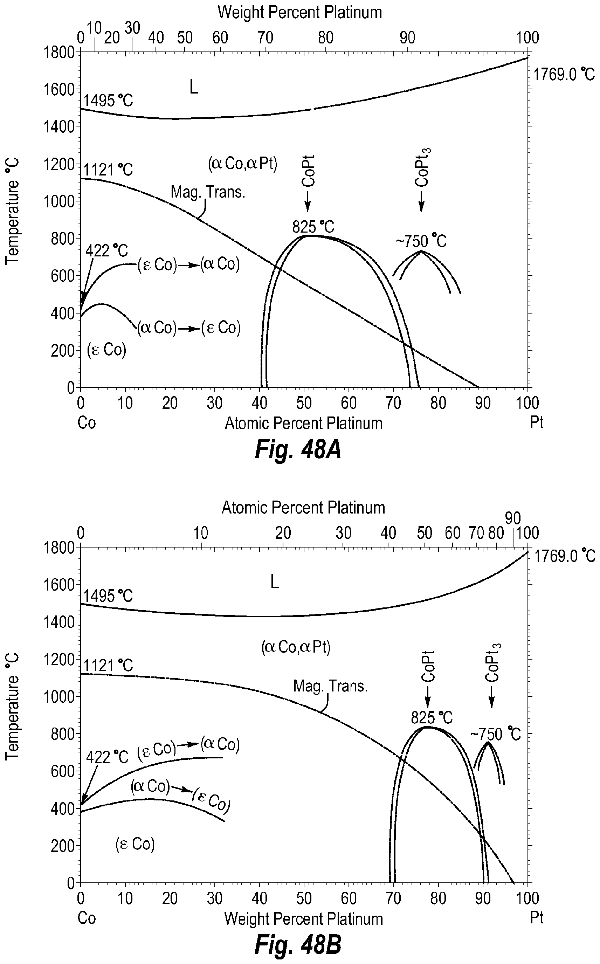

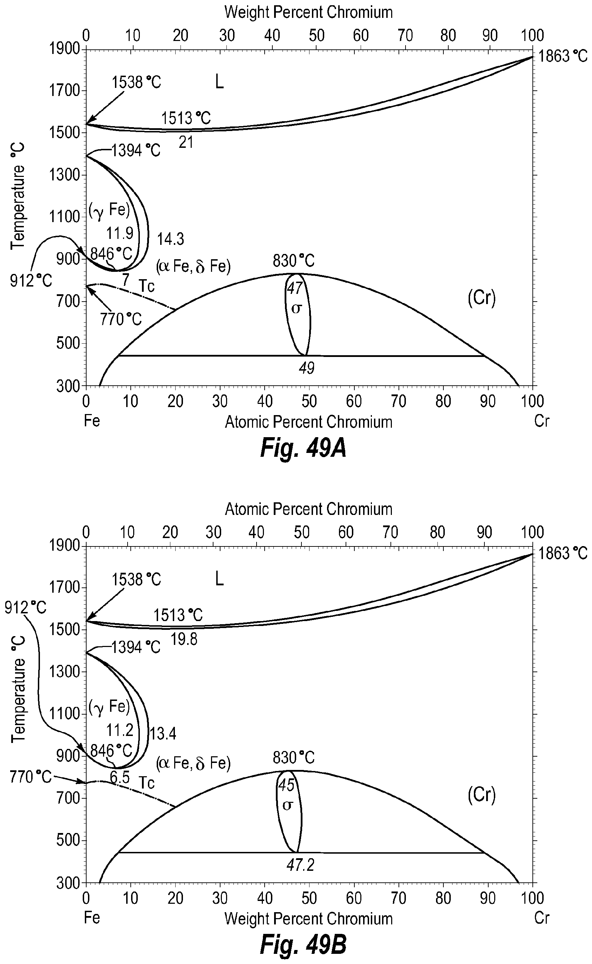

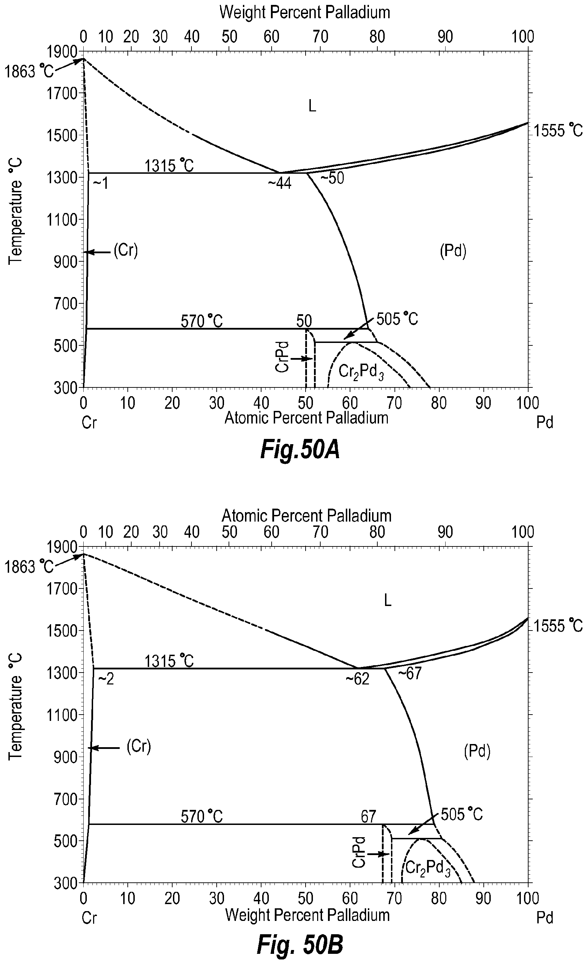

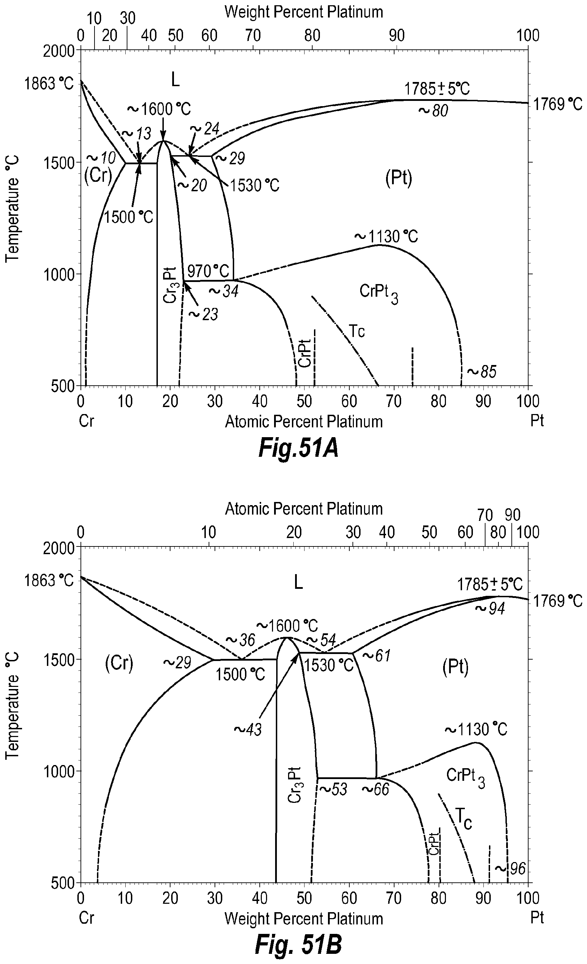

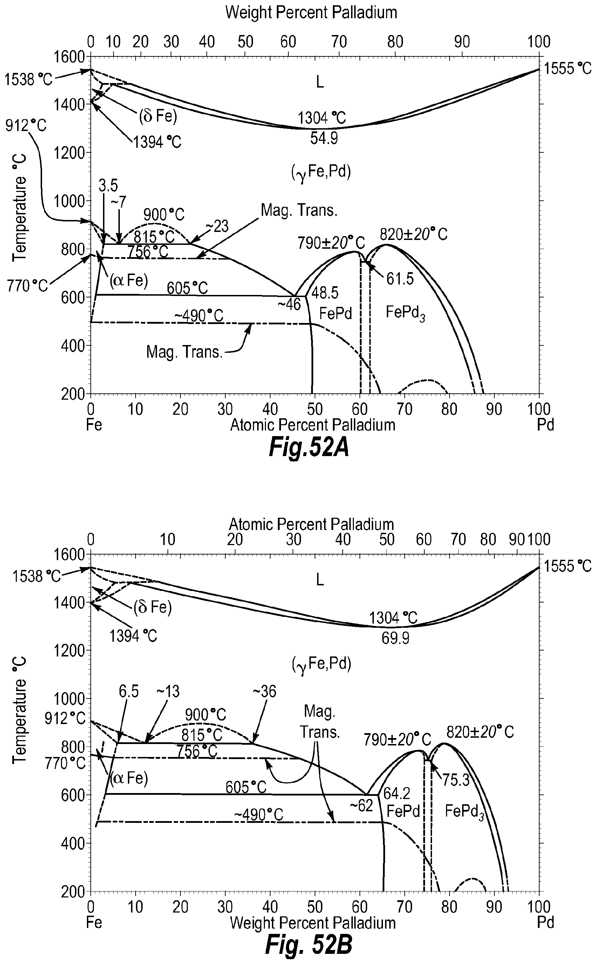

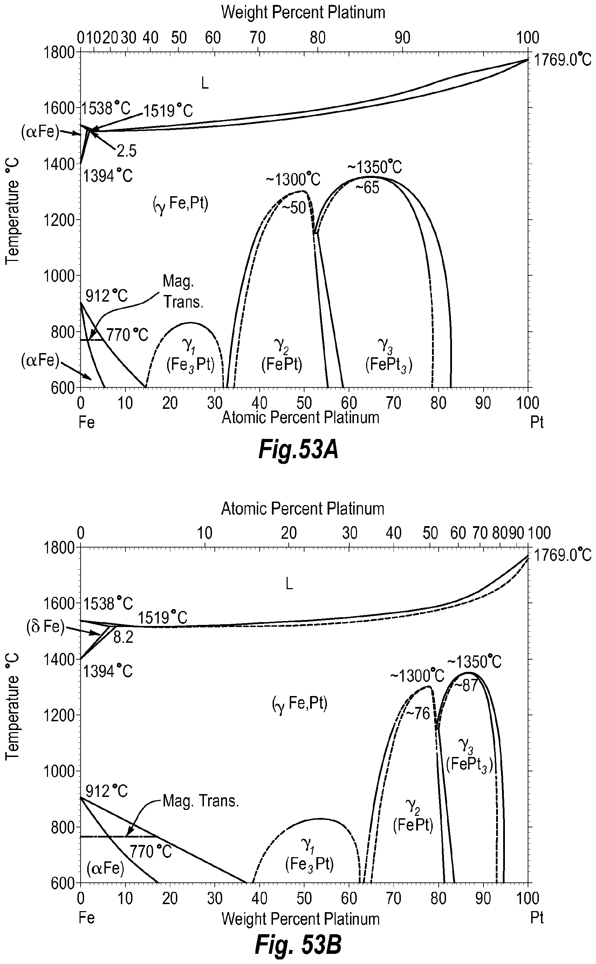

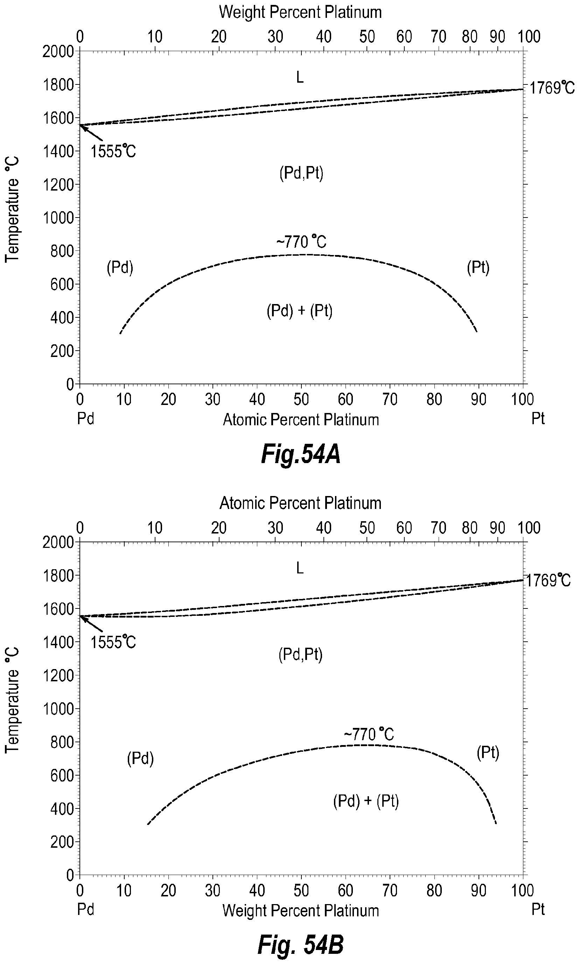

For a disclosed alloy containing Co, Cr, Fe and Pd or Pt (e.g., as discussed in more detail in conjunction with Examples 78-89, and particularly Example 85, below), the following may be observed:
- Co-Fe is a phase diagram showing a miscibility gap between about 28 and about 74Fe, with a basic cubic structure of the two intermixed Co and Fe phases. Above and below these compositions the structure is a solid solution Co and Fe mixture with a BCC structure (**FIGS. 46A-46B**).
- Co-Pd phase diagram shows a continuous solid solution with an HCP (εCo) structure (**FIGS. 47A-47B**).
- Co-Pt phase diagram shows a solid solution structure with two miscibility gaps and two focal phase formations. Up to about 69Pt the structure is HCP (εCo) and FCC α(Co,Pt). Between about 69 and about 91Pt there is a miscibility gap, with a focal tetragonal CoPt structure at about 76Pt. Above 91Pt there is again FCC α(Co,Pt) (**FIGS. 48A-48B**).
- Cr-Fe is a somewhat complex phase diagram with a variety of features. This system displays a BCC eutectic structure between about 3 and about 97Fe. Above 97Fe, the structure is primarily FCC, while below 3Fe the structure is primarily BCC (**FIGS. 49A-49B**).
- Cr-Pd is a fairly complex phase diagram. Up to about 67Pd, the structure is a mixture of FCC (Cr) and tetragonal CrPd. Between about 67 and about 69Pd the structure is tetragonal CrPd. Between about 69 and about 71Pd, the structure is a mixture of tetragonal CrPd and FCC Cr₂Pd₃. Between about 71 and about 88Pd the structure is FCC Cr₂Pd₃. Above about 88Pd, the structure is FCC (Pd) (**FIGS. 50A-50B**).
- Cr-Pt is a fairly complex phase diagram. Up to about 5Pt, the structure is FCC (Cr). From about 5 to about 43Pt the structure is a mix of FCC (Cr) and cubic Cr₃Pt. From about 43 to about 51Pt the structure is cubic Cr₃Pt. From about 51 to about 78Pt the structure is a mix of cubic Cr₃Pt and tetragonal CrPt. From about 78 to about 80Pt the structure is tetragonal CrPt. From about 80 to about 96Pt the structure is primarily cubic CrPt₃. Above about 96Pt the structure is FCC (Pt) (**FIGS.** 51A-51B)**.**
- Fe-Pd is a complex phase diagram. Up to about 65Pd is a combination of FCC (γFe,Pd) and tetragonal FePd. From about 65 to about75Pd is tetragonal FePd. From about 75 to about 93Pd is cubic FePd₃. Above around 93Pd is FCC (γFe,Pd) (**FIGS. 52A-52B**).
- Fe-Pt phase diagram shows a eutectic with multiple intermediate phases. Up to about 18Pt is a BCC solid solution. Between about 18 and about 38Pt is an FCC solid solution. Between about 38 and about 62Pt is cubic γ₁ (Fe₃Pt). Between about 63 and about 82Pt is tetragonal γ₂ (FePt). Between about 82 and about 94Pt is cubic γ₃ (FePt₃). Above about 94Pt, the structure is a continuous solid solution with a FCC structure (**FIGS. 53A-53B****).**
- Pd-Pt phase diagram shows a continuous solid solution that is a mixture of FCC (Pd) + (Pt) across the majority of compositions. At some undefined lower and upper compositions the phase is primarily FCC (Pd) or FCC (Pt) (**FIGS. 54A-54B**).

In considering the possible disclosed alloys from both a radiopacity and a compatibility perspective, the following possible compositions come to the forefront. Note that minor and trace elements are not considered in these calculations. It is important to avoid the Co-Cr σ intermetallic phase, because this phase is quite brittle. The examples below will explicitly discuss avoidance of this phase by remaining below the 51-64 weight percent Cr in the Co-Cr regime. It is also envisioned that viable alloy compositions may be provided above this regime (i.e., greater than about 64Cr in the Co-Cr regime) that would provide workable alloys. For example, 10Co and 20Cr would give a relative composition of 33.3Co and 66.7Cr, moving above the σ intermetallic regime.

Reference Examples 25-30 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, silver. Example 31 further replaces the tungsten of L-605 or the molybdenum of MP-35N with silver so that the alloy is essentially a ternary Co-Cr-Ag alloy. Trace elements such as beryllium, boron, carbon, iron, manganese, phosphorus, silicon, sulfur, and titanium are not listed.

### Reference Example 25 - ASTM F90 L-605 Alloy With Weight Substitution of Nickel With Silver

| Element | Weight Percent |
|---|---|
| Cobalt | 55 |
| Chromium | 20 |
| Tungsten | 15 |
| Silver | 10 |

From a metallurgical perspective, the Co-Cr, Co-W, and Cr-W will fall in the same regions as for L-605. Otherwise the alloy will also include a series of eutectic immiscible phases of FCC Ag, HCP εCo, BCC Cr, and BCC W. This alloy would otherwise behave similarly to L-605, but with increased relatively radiopacity (to about 4.7 barnes/cc).

### Reference Example 26 - ASTM F90 L-605 Alloy With Weight Substitution of Nickel and a Portion of the Cobalt With Silver

| Element | Weight Percent |
|---|---|
| Cobalt | 35 |
| Chromium | 20 |
| Tungsten | 15 |
| Silver | 30 |

In order to increase the radiopacity, the silver may be increased (beyond simply substituting for Ni) to also substitute for a portion of the cobalt. For example, increasing the silver to 30 weight percent would lower cobalt to 35 weight percent, leaving the other elements the same. This results in a calculated radiopacity of 7.0 barnes/cc. Similar to Example 25, the silver would result in a series of immiscible phases with Co, Cr, and W. Co-Cr would shift to be in the same region as MP-35N. Co-W would shift higher but still remain in the same phase region as L-605. Cr-W would remain the same as L-605. This would result in a workable alloy.

### Reference Example 27 - ASTM F90 L-605 Alloy With Weight Substitution of Nickel and a Portion of the Cobalt With Silver

| Element | Weight Percent |
|---|---|
| Cobalt | 25 |
| Chromium | 20 |
| Tungsten | 15 |
| Silver | 40 |

If even higher radiopacity is desired, the silver may be increased to 40 weight percent. This results in a calculated radiopacity of 8.2 barnes/cc. Similar to Example 26, the silver would remain a series of immiscible phases with Co, Cr, and W. Co-Cr would shift to be in the same region as MP-35N, closer to the middle of the phase. Co-W shifts higher but remains in the same phase region as L-605. Cr-W would remain the same as for L-605. This would result in a workable alloy.

### Reference Example 28 - ASTM F90 L-605 Alloy With Weight Substitution of Nickel and a Portion of the Cobalt With Silver

| Element | Weight Percent |
|---|---|
| Cobalt | at least 20 |
| Chromium | 20 |
| Tungsten | 15 |
| Silver | up to 45 |

Assuming that Cr and W are not changed, Ag should remain at or below about 45 weight percent (e.g., 10 to 45 weight percent) in order to avoid the Co-Cr σ intermetallic phase. At 45 weight percent Ag, calculated radiopacity would be 8.8 barnes/cc.

### Reference Example 29 - ASTM F562 MP-35N Alloy With Weight Substitution of Nickel With Silver

| Element | Weight Percent |
|---|---|
| Cobalt | 35 |
| Chromium | 20 |
| Molybdenum | 10 |
| Silver | 35 |

Utilizing a straight substitution of silver for nickel on a weight percentage basis results in the calculated radiopacity increasing from 3.4 barnes/cc to 7.0 barnes/cc. From a metallurgical perspective, the alloy includes a series of eutectic immiscible phases of FCC Ag, HCP εCo, BCC Cr, and BCC Mo with Co-Cr, Co-Mo, and Cr-Mo falling in the same regions as MP-35N. This alloy would otherwise behave similarly to MP-35N, but with increased relatively radiopacity (to about 7.0 barnes/cc).

### Reference Example 30 - ASTM F562 MP-35N Alloy With Weight Substitution of Nickel and a Portion of the Cobalt With Silver

| Element | Weight Percent |
|---|---|
| Cobalt | at least 20 |
| Chromium | 20 |
| Molybdenum | 10 |
| Silver | up to 50 |

Assuming that Cr and Mo are not changed, Ag should remain at or below about 50 weight percent (e.g., 35 to 50 weight percent) in order to avoid the Co-Cr σ intermetallic phase. At 50 weight percent Ag, calculated radiopacity would be 8.7 barnes/cc.

### Reference Example 31 - ASTM F90 L-605 Alloy or ASTM F562 MP-35N Alloy With Weight Substitution of Nickel and Tungsten or Molybdenum With Silver

| Element | Weight Percent |
|---|---|
| Cobalt | at least 20 |
| Chromium | 20 |
| Silver | up to 60 |

When considering the disparity in melting temperature between Ag and Mo and W, the alloy may be considered without either of these elements (the Mo or W), i.e., consisting of Ag-Co-Cr only. In this instance, the Ag may range up to about 60 weight percent to avoid the Co-Cr σ intermetallic phase. At 60 weight percent Ag, calculated radiopacity would be 9.0 barnes/cc. Ag-Co and Ag-Cr both fall into the eutectic regimes of these phase diagrams.

Examples 32-35 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, gold. Example 36 further replaces the tungsten of L-605 or the molybdenum of MP-35N with gold so that the alloy is essentially a ternary Co-Cr-Au alloy. Trace elements such as beryllium, boron, carbon, iron, manganese, phosphorus, silicon, sulfur, and titanium are not listed.

### Reference Example 32 - ASTM F90 L-605 Alloy With Weight Substitution of Nickel With Gold

| Element | Weight Percent |
|---|---|
| Cobalt | 55 |
| Chromium | 20 |
| Tungsten | 15 |
| Gold | 10 |

Substituting Au for Ni on a weight percentage basis increases the calculated radiopacity from 3.6 to 4.6 barnes/cc. From a metallurgical perspective, the Co-Cr, CoW, and Cr-W will fall in the same regions as for L-605. Otherwise the alloy will also include a series of eutectic immiscible phases of FCC Au, BCC α' and HCP εCo, BCC Cr, and BCC W. This alloy would otherwise behave similarly to L-605, but with increased relatively radiopacity (to about 4.6 barnes/cc).

### Reference Example 33 - ASTM F90 L-605 Alloy With Weight Substitution of Nickel and a Portion of the Cobalt With Gold

| Element | Weight Percent |
|---|---|
| Cobalt | at least 20 |
| Chromium | 20 |
| Tungsten | 15 |
| Gold | up to 45 |

As with Ag, assuming that Cr and W are not changed, Au should remain at or below about 45 weight percent (e.g., 10 to 45 weight percent) in order to avoid the Co-Cr σ intermetallic phase. At 45 weight percent Au, calculated radiopacity would be 8.9 barnes/cc.

### Reference Example 34 - ASTM F562 MP-35N Alloy With Weight Substitution of Nickel With Gold

| Element | Weight Percent |
|---|---|
| Cobalt | 35 |
| Chromium | 20 |
| Molybdenum | 10 |
| Gold | 35 |

Utilizing a straight substitution of gold for nickel on a weight percentage basis results in the calculated radiopacity increasing from 3.4 barnes/cc to 6.8 barnes/cc. From a metallurgical perspective, the alloy includes a series of eutectic immiscible phases of FCC Au, BCC α' and HCP εCo, BCC Cr, and BCC Mo with Co-Cr, Co-Mo, and Cr-Mo falling in the same regions as MP-35N. Similar to the MP-35N alloy, this would result in a workable alloy.

### Reference Example 35 - ASTM F562 MP-35N Alloy With Weight Substitution of Nickel and a Portion of the Cobalt With Gold

| Element | Weight Percent |
|---|---|
| Cobalt | at least 20 |
| Chromium | 20 |
| Molybdenum | 10 |
| Gold | up to 50 |

Assuming that Cr and Mo are not changed, Au should remain at or below about 50 weight percent (e.g., 35 to 50 weight percent) in order to avoid the Co-Cr σ intermetallic phase. At 50 weight percent Au, calculated radiopacity would be 8.7 barnes/cc.

### Reference Example 36 - ASTM F90 L-605 Alloy or ASTM F562 MP-35N Alloy With Weight Substitution of Nickel and Tungsten or Molybdenum With Gold

| Element | Weight Percent |
|---|---|
| Cobalt | at least 20 |
| Chromium | 20 |
| Gold | up to 60 |

When considering the disparity in melting temperature between Au and Mo and W, the alloy may be considered without either of these elements (the Mo or W), i.e., consisting of Au-Co-Cr only. In this instance, the Au may range up to about 60 weight percent to avoid the Co-Cr σ intermetallic phase. At 60 weight percent Au, calculated radiopacity would be 9.2 barnes/cc. Au-Co and Au-Cr both fall into the eutectic regimes of these phase diagrams.

Examples 37-40 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, hafnium. Example 41 further replaces the tungsten of L-605 or the molybdenum of MP-35N with hafnium so that the alloy is essentially a ternary Co-Cr-Hf alloy. Trace elements such as beryllium, boron, carbon, iron, manganese, phosphorus, silicon, sulfur, and titanium are not listed.

When considering such systems, a few items should be taken into account. As Hf falls in the refractory metal category and L605 and MP-35N Co alloys already include tungsten or Mo, respectively, which also fall into this category, the metallurgical behavior of Hf may be inferred from Co-Mo and Co-W phase diagrams. It is noted that Hf has a large effect on other refractory metals and on Ni and Fe based alloys in small amounts. The grain boundary pinning effect that is experienced in smaller additions will not continue with larger additions. Furthermore, the Co-Hf mixture may be partially paramagnetic as early as around 47 weight percent Hf. As with other alloys, it is likely that this behavior is repressed by the other elements in the alloy, resulting in a workable alloy.

### Reference Example 37 - ASTM F90 L-605 Alloy With Weight Substitution of Nickel With Hafnium

| Element | Weight Percent |
|---|---|
| Cobalt | 55 |
| Chromium | 20 |
| Tungsten | 15 |
| Hafnium | 10 |

Substituting Hf for Ni on a weight percentage basis increases the calculated radiopacity from 3.6 to 4.3 barnes/cc. From a metallurgical perspective, the Co-Cr, CoW, and Cr-W will fall in the same regions as for L-605. For Hf-Co, the alloy will fall within the mixed HCP εCo and tetragonal Co₇Hf₂ regime. For Hf-Cr, the alloy will fall within the FCC Cr₂Hf plus BCC Cr phases. For Hf-Mo the mixture would include BCC W and FCC HfW₂. It is expected that this alloy may be stronger than L-605.

### Reference Example 38 - ASTM F90 L-605 Alloy With Weight Substitution of Nickel and a Portion of the Cobalt With Hafnium

| Element | Weight Percent |
|---|---|
| Cobalt | at least 20 |
| Chromium | 20 |
| Tungsten | 15 |
| Hafnium | up to 45 |

As with Ag and Au, here it is assumed that Cr and W are not changed. At around 30 weight percent Hf, radiopacity would be 5.8 barnes/cc. Phases would include a combination of HCP εCo and tetragonal Co₇Hf₂, Cr₂Hf, HCP αHf, and FCC HfW₂. If the suppression of the paramagnetism is successful and strengthening is not too much to allow workability, Hf may be increased up to 45 weight percent. At 45 weight percent Hf, the radiopacity is 7.2 barnes/cc, and metallurgically would result in a mix of FCC Co₂Hf and cubic CoHf, FCC Cr₂Hf and some HCP αHf, and FCC HfW₂ and HCP αHf phases.

### Reference Example 39 - ASTM F562 MP-35N Alloy With Weight Substitution of Nickel With Hafnium

| Element | Weight Percent |
|---|---|
| Cobalt | 35 |
| Chromium | 20 |
| Molybdenum | 10 |
| Hafnium | 35 |

Utilizing a straight substitution of hafnium for nickel on a weight percentage basis results in the calculated radiopacity increasing from 3.4 barnes/cc to 5.7 barnes/cc. From a metallurgical perspective, Co-Cr, Co-Mo, and Cr-Mo will fall in the same regions as MP-35N. Co-Hf falls into the possible partially paramagnetic regime with the combination of tetragonal Co₇Hf₂ and FCC Co₂Hf. Presuming the suppression of the paramagnetism by the presence of the other elements in the alloy, this alloy would also include a combination of FCC Cr₂Hf, FCC αMo₂Hf and HCP αHf phases.

### Reference Example 40 - ASTM F562 MP-35N Alloy With Weight Substitution of Nickel and a Portion of the Cobalt With Hafnium

| Element | Weight Percent |
|---|---|
| Cobalt | at least 20 |
| Chromium | 20 |
| Molybdenum | 10 |
| Hafnium | up to 50 |

If the suppression of the paramagnetism is successful and strengthening is not too much to still allow workability, Hf may be included up to about 50 weight percent. This would result in a radiopacity of 6.9 barnes/cc. Phases in this alloy beyond the MP-35N Co-Cr, Co-Mo and Cr-Mo phase mixtures would include FCC Co₂Hf and cubic CoHf, FCC Cr₂Hf and HCP αHf, and FCC αMo₂Hf and HCP αHf phases.

### Reference Example 41 - ASTM F90 L-605 Alloy or ASTM F562 MP-35N Alloy With Weight Substitution of Nickel and Tungsten or Molybdenum With Hafnium

| Element | Weight Percent |
|---|---|
| Cobalt | at least 20 |
| Chromium | 20 |
| Hafnium | up to 60 |

When considering a simpler mixture, the alloy may be considered without either Mo or W, i.e., consisting of Hf-Co-Cr only. In this instance, the Hf may range up to about 60 weight percent to avoid the Co-Cr σ intermetallic phase. At 60 weight percent Hf, calculated radiopacity would be 6.9 barnes/cc. Co-Hf falls in the cubic CoHf phase and Cr-Hf falls in the FCC Cr₂Hf plus HCP αHf regime.

Examples 42-44 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, molybdenum. Example 45 further replaces the tungsten of L-605 (or is relative to MP-35N, which already includes some molybdenum) with molybdenum so that the alloy is essentially a ternary Co-Cr-Mo alloy. Trace elements such as beryllium, boron, carbon, iron, manganese, phosphorus, silicon, sulfur, and titanium are not listed.

### Reference Example 42 - ASTM F90 L-605 Alloy With Weight Substitution of Nickel With Molybdenum

| Element | Weight Percent |
|---|---|
| Cobalt | 55 |
| Chromium | 20 |
| Tungsten | 15 |
| Molybdenum | 10 |

Substituting Mo for Ni on a weight percentage basis increases the calculated radiopacity from 3.6 to 4.4 barnes/cc. From a metallurgical perspective, the Co-Cr, CoW, and Cr-W will fall in the same regions as for L-605. Otherwise the alloy will contain a mixture of HCP εCo and HCP κ phases, BCC Cr and BCC Mo phases, and solid solution Mo and W phases.

### Reference Example 43 - ASTM F90 L-605 Alloy With Weight Substitution of Nickel and a Portion of the Cobalt With Molybdenum

| Element | Weight Percent |
|---|---|
| Cobalt | at least 20 |
| Chromium | 20 |
| Tungsten | 15 |
| Molybdenum | up to 45 |

As with the above alloys of Ag, Au, and Hf, here it is assumed that Cr and W are not changed, thus Co-Cr, Co-W, and Cr-W are the same as for L-605. From a metallurgical phase diagram perspective, Mo may be included up to about 45 weight percent. At 45 weight percent Mo, this gives a radiopacity of 7.2 barnes/cc and metallurgically would result in a mix of rhombohedral ε and BCC Mo, BCC Cr and BCC Mo, and BCC Mo and BCC W phases.

### Reference Example 44 - ASTM F562 MP-35N Alloy With Weight Substitution of Nickel With Molybdenum

| Element | Weight Percent |
|---|---|
| Cobalt | 35 |
| Chromium | 20 |
| Molybdenum | 45 |

Utilizing a straight substitution of molybdenum for nickel on a weight percentage basis increases the radiopacity from 3.4 barnes/cc to 5.8 barnes/cc. From a metallurgical perspective, Co-Cr falls in the same region as MP-35N. Co-Mo and Cr-Mo shift, as the Mo has increased by 35 weight percentage points. Co-Mo shifts into the rhombohedral ε phase region, and Cr-Mo stays in the BCC Cr and BCC Mo regime, shifting closer to the Mo side of the phase diagram.

### Reference Example 45 - ASTM F90 L-605 Alloy or ASTM F562 MP-35N Alloy With Weight Substitution of Nickel and Tungsten or a Portion of the Cobalt With Molybdenum

| Element | Weight Percent |
|---|---|
| Cobalt | at least 20 |
| Chromium | 20 |
| Molybdenum | up to 60 |

When considering a simpler mixture, the alloy may be considered with even more Mo, without W (in the case of L-605), i.e., consisting of Mo-Co-Cr only. In the case of a modified MP-35N alloy, some of the cobalt may be replaced to further increase Mo content. Avoiding the Co-Cr σ intermetallic phase, Mo may be used up to 60 weight percent. This increases the radiopacity to 7.0 barnes/cc. This would shift the Co-Mo interaction into the rhombohedral ε and BCC Mo phase regime. The Cr-Co would remain in the BCC Cr and BCC Mo regime, shifting further toward the Mo side of the phase diagram.

Examples 46-49 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, niobium. Example 50 further replaces the tungsten of L-605 or the molybdenum of MP-35N with niobium so that the alloy is essentially a ternary Co-Cr-Nb alloy. Trace elements such as beryllium, boron, carbon, iron, manganese, phosphorus, silicon, sulfur, and titanium are not listed.

### Reference Example 46 - ASTM F90 L-605 Alloy With Weight Substitution of Nickel With Niobium

| Element | Weight Percent |
|---|---|
| Cobalt | 55 |
| Chromium | 20 |
| Tungsten | 15 |
| Niobium | 10 |

Substituting Nb for Ni on a weight percentage basis increases the calculated radiopacity from 3.6 to 4.2 barnes/cc. From a metallurgical perspective, the Co-Cr, CoW, and Cr-W will fall in the same regions as for L-605. Otherwise the alloy will contain a mixture of HCP εCo and HCP NbCo₃ phases, BCC Cr and BCC Cr₂Nb phases, and solid solution Nb and W phases.

### Reference Example 47 - ASTM F90 L-605 Alloy With Weight Substitution of Nickel and a Portion of the Cobalt With Niobium

| Element | Weight Percent |
|---|---|
| Cobalt | at least 20 |
| Chromium | 20 |
| Tungsten | 15 |
| Niobium | up to 45 |

As with the above alloys of Ag, Au, Hf, and Mo, here it is assumed that Cr and W are not changed, thus Co-Cr, Co-W, and Cr-W are the same as for L-605. From a metallurgical phase diagram perspective, Nb may be included up to about 45 weight percent. At 45 weight percent Nb, this gives a radiopacity of 6.3 barnes/cc and metallurgically would result in a mix of rhombohedral Nb₆Co₇ and BCC Nb, FCC Cr₂Nb and BCC Nb, and BCC Nb and BCC W phases.

### Reference Example 48 - ASTM F562 MP-35N Alloy With Weight Substitution of Nickel With Niobium

| Element | Weight Percent |
|---|---|
| Cobalt | 35 |
| Chromium | 20 |
| Molybdenum | 10 |
| Niobium | 35 |

Utilizing a straight substitution of niobium for nickel on a weight percentage basis increases the radiopacity from 3.4 barnes/cc to 5.3 barnes/cc. From a metallurgical perspective, Co-Cr, Co-Mo, and Cr-Mo fall in the same regions as MP-35N. Other phases will include rhombohedral Nb₆Co₇ and BCC Nb phases, FCC Cr₂Nb and BCC Nb phases, and BCC Nb and BCC Mo phases.

### Reference Example 49 - ASTM F562 MP-35N Alloy With Weight Substitution of Nickel and a Portion of the Cobalt With Niobium

| Element | Weight Percent |
|---|---|
| Cobalt | at least 20 |
| Chromium | 20 |
| Molybdenum | 10 |
| Niobium | up to 50 |

While avoiding the Co-Cr σ intermetallic phase Nb may be included up to 50 weight percent. This increases radiopacity to 6.1 barnes/cc. Metallurgically, this results in the presence of rhombohedral Nb₆Co₇ and BCC Nb phases, FCC Cr₂Nb and BCC Nb phases, and BCC Nb and BCC Mo phases.

### Reference Example 50 - ASTM F90 L-605 Alloy or ASTM F562 MP-35N Alloy With Weight Substitution of Nickel and Tungsten or Molybdenum With Niobium

| Element | Weight Percent |
|---|---|
| Cobalt | at least 20 |
| Chromium | 20 |
| Niobium | up to 60 |

When considering a simpler mixture, the alloy may be considered without either Mo or W, i.e., consisting of Nb-Co-Cr only. In this instance, the Nb may range up to about 60 weight percent to avoid the Co-Cr σ intermetallic phase. At 60 weight percent Nb, calculated radiopacity would be 5.9 barnes/cc. Co-Nb falls in the rhombohedral Nb₆Co₇ and BCC Nb regime, while the Cr-Nb falls in the FCC Cr₂Nb and BCC Nb regime.

Examples 51-54 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, rhenium. Example 55 further replaces the tungsten of L-605 or the molybdenum of MP-35N with rhenium so that the alloy is essentially a ternary Co-Cr-Re alloy. Trace elements such as beryllium, boron, carbon, iron, manganese, phosphorus, silicon, sulfur, and titanium are not listed.

### Reference Example 51 - ASTM F90 L-605 Alloy With Weight Substitution of Nickel With Rhenium

| Element | Weight Percent |
|---|---|
| Cobalt | 55 |
| Chromium | 20 |
| Tungsten | 15 |
| Rhenium | 10 |

Substituting Re for Ni on a weight percentage basis increases the calculated radiopacity from 3.6 to 4.4 barnes/cc. From a metallurgical perspective, the Co-Cr, CoW, and Cr-W will fall in the same regions as for L-605. Otherwise the alloy will contain a mixture of HCP εCo and HCP Re phases, BCC Cr and BCC Re phases, and the Re-W will contain tetragonal σ phase and BCC W phase.

### Reference Example 52 - ASTM F90 L-605 Alloy With Weight Substitution of Nickel and a Portion of the Cobalt With Rhenium

| Element | Weight Percent |
|---|---|
| Cobalt | at least 20 |
| Chromium | 20 |
| Tungsten | 15 |
| Rhenium | up to 45 |

As with the above alloys of Ag, Au, Hf, Mo, and Nb, here it is assumed that Cr and W are not changed, thus Co-Cr, Co-W, and Cr-W are the same as for L-605. From a metallurgical phase diagram perspective, Re may be included up to about 45 weight percent. At 45 weight percent Re, this gives a radiopacity of 8.0 barnes/cc and metallurgically would result in a mix of HCP (εCo,Re), BCC Cr and tetragonal σ(Cr₂Re₃), and BCC χ and HCP Re phases.

### Reference Example 53 - ASTM F562 MP-35N Alloy With Weight Substitution of Nickel With Rhenium

| Element | Weight Percent |
|---|---|
| Cobalt | 35 |
| Chromium | 20 |
| Molybdenum | 10 |
| Rhenium | 35 |

Utilizing a straight substitution of rhenium for nickel on a weight percentage basis increases the radiopacity from 3.4 barnes/cc to 6.1 barnes/cc. From a metallurgical perspective, Co-Cr, Co-Mo, and Cr-Mo fall in the same regions as MP-35N. Other phases will include HCP (εCo,Re), BCC Cr and BCC Re phases, and BCC Mo and BCC χ phases.

### Reference Example 54 - ASTM F562 MP-35N Alloy With Weight Substitution of Nickel and a Portion of the Cobalt With Rhenium

| Element | Weight Percent |
|---|---|
| Cobalt | at least 20 |
| Chromium | 20 |
| Molybdenum | 10 |
| Rhenium | up to 50 |

While avoiding the Co-Cr σ intermetallic phase Re may be included up to 50 weight percent. This increases radiopacity to 7.8 barnes/cc. Metallurgically, this results in the presence of HCP (εCo,Re), BCC Cr and tetragonal σ(Cr₂Re₃), and BCC Mo and BCC χ phases.

### Reference Example 55 - ASTM F90 L-605 Alloy or ASTM F562 MP-35N Alloy With Weight Substitution of Nickel and Tungsten or Molybdenum With Rhenium

| Element | Weight Percent |
|---|---|
| Cobalt | at least 20 |
| Chromium | 20 |
| Rhenium | up to 60 |

When considering a simpler mixture, the alloy may be considered without either Mo or W, i.e., consisting of Re-Co-Cr only. In this instance, the Re may range up to about 60 weight percent to avoid the Co-Cr σ intermetallic phase. At 60 weight percent Re, calculated radiopacity would be 8.1 barnes/cc. Co-Re falls in the HCP (εCo,Re) regime while Cr-Re falls into the BCC Cr and tetragonal σ(Cr₂Re₃) regime.

Examples 56-59 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, tantalum. Example 60 further replaces the tungsten of L-605 or the molybdenum of MP-35N with tantalum so that the alloy is essentially a ternary Co-Cr-Ta alloy. Trace elements such as beryllium, boron, carbon, iron, manganese, phosphorus, silicon, sulfur, and titanium are not listed.

### Reference Example 56 - ASTM F90 L-605 Alloy With Weight Substitution of Nickel With Tantalum

| Element | Weight Percent |
|---|---|
| Cobalt | 55 |
| Chromium | 20 |
| Tungsten | 15 |
| Tantalum | 10 |

Substituting Ta for Ni on a weight percentage basis increases the calculated radiopacity from 3.6 to 4.4 barnes/cc. From a metallurgical perspective, the Co-Cr, CoW, and Cr-W will fall in the same regions as for L-605. Otherwise the alloy will contain a mixture of HCP εCo and Co₇Ta₂, BCC Cr and FCC Cr₂Ta, and BCC (Ta,W) phases.

### Reference Example 57 - ASTM F90 L-605 Alloy With Weight Substitution of Nickel and a Portion of the Cobalt With Tantalum

| Element | Weight Percent |
|---|---|
| Cobalt | at least 20 |
| Chromium | 20 |
| Tungsten | 15 |
| Tantalum | up to 45 |

As with the above alloys of Ag, Au, Hf, Mo, Nb and Re, here it is assumed that Cr and W are not changed, thus Co-Cr, Co-W, and Cr-W are the same as for L-605. From a metallurgical phase diagram perspective, Ta may be included up to about 45 weight percent. At 45 weight percent Ta, this gives a radiopacity of 7.9 barnes/cc and metallurgically would result in a mix of FCC λ₂ (Co₂Ta) and rhombohedral Co₆Ta₇, FCC Cr₂Ta and BCC Ta, and BCC (Ta,W) phases.

### Reference Example 58 - ASTM F562 MP-35N Alloy With Weight Substitution of Nickel With Tantalum

| Element | Weight Percent |
|---|---|
| Cobalt | 35 |
| Chromium | 20 |
| Molybdenum | 10 |
| Tantalum | 35 |

Utilizing a straight substitution of tantalum for nickel on a weight percentage basis increases the radiopacity from 3.4 barnes/cc to 6.1 barnes/cc. From a metallurgical perspective, Co-Cr, Co-Mo, and Cr-Mo fall in the same regions as MP-35N. Other phases will include Co₇Ta₂ and HCP λ₃, FCC Cr₂Ta, and BCC Mo and BCC Ta phases.

### Reference Example 59 - ASTM F562 MP-35N Alloy With Weight Substitution of Nickel and a Portion of the Cobalt With Tantalum

| Element | Weight Percent |
|---|---|
| Cobalt | at least 20 |
| Chromium | 20 |
| Molybdenum | 10 |
| Tantalum | up to 50 |

While avoiding the Co-Cr σ intermetallic phase Ta may be included up to 50 weight percent. This increases radiopacity to 7.7 barnes/cc. Metallurgically, this results in the presence of Co₆Ta_{7,} FCC Cr₂Ta and BCC Ta, and BCC Mo and BCC Ta phases.

### Reference Example 60 - ASTM F90 L-605 Alloy or ASTM F562 MP-35N Alloy With Weight Substitution of Nickel and Tungsten or Molybdenum With Tantalum

| Element | Weight Percent |
|---|---|
| Cobalt | at least 20 |
| Chromium | 20 |
| Tantalum | up to 60 |

When considering a simpler mixture, the alloy may be considered without either Mo or W, i.e., consisting of Ta-Co-Cr only. In this instance, the Ta may range up to about 60 weight percent to avoid the Co-Cr σ intermetallic phase. At 60 weight percent Ta, calculated radiopacity would be 7.9 barnes/cc. Co-Ta falls in the rhombohedral Co₆Ta₇ regime and Cr-Ta falls in the FCC Cr₂Ta and BCC Ta regime.

Examples 61-64 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, tungsten. The modified L-605 alloys are essentially ternary Co-Cr-W alloys. Trace elements such as beryllium, boron, carbon, iron, manganese, phosphorus, silicon, sulfur, and titanium are not listed.

### Reference Example 61 - ASTM F90 L-605 Alloy With Weight Substitution of Nickel With Tungsten

| Element | Weight Percent |
|---|---|
| Cobalt | 55 |
| Chromium | 20 |
| Tungsten | 25 |

Substituting W for Ni on a weight percentage basis increases the calculated radiopacity from 3.6 to 4.4 barnes/cc. From a metallurgical perspective, Co-Cr will fall in the same region as for L-605. Otherwise, the alloy will contain a mixture of HCP εCo and HCP Co₃W, and BCC (α₁+α₂) phases.

### Reference Example 62 - ASTM F90 L-605 Alloy With Weight Substitution of Nickel and a Portion of the Cobalt With Tungsten

| Element | Weight Percent |
|---|---|
| Cobalt | at least 35 |
| Chromium | 20 |
| Tungsten | up to 45 |

It is assumed that Cr is not changed, thus Co-Cr is the same as for L-605. From a metallurgical phase diagram perspective, W may be included up to about 45 weight percent. At 45 weight percent W, this gives a radiopacity of 7.9 barnes/cc and metallurgically would result in a mix of rhombohedral Co₇W₆ and HCP Co₃W, and BCC (α₁+α₂) phases.

### Reference Example 63 - ASTM F562 MP-35N Alloy With Weight Substitution of Nickel With Tungsten

| Element | Weight Percent |
|---|---|
| Cobalt | 35 |
| Chromium | 20 |
| Molybdenum | 10 |
| Tungsten | 35 |

Utilizing a straight substitution of tungsten for nickel on a weight percentage basis increases the radiopacity from 3.4 barnes/cc to 6.1 barnes/cc. From a metallurgical perspective, Co-Cr, Co-Mo, and Cr-Mo fall in the same regions as MP-35N. Other phases will include HCP Co₃W, BCC (α₁+α₂), and BCC Mo and BCC W phases.

### Reference Example 64 - ASTM F562 MP-35N Alloy With Weight Substitution of Nickel and a Portion of the Cobalt With Tungsten

| Element | Weight Percent |
|---|---|
| Cobalt | at least 20 |
| Chromium | 20 |
| Molybdenum | 10 |
| Tungsten | up to 50 |

While avoiding the Co-Cr σ intermetallic phase W may be included up to 50 weight percent. This increases radiopacity to 7.7 barnes/cc. Metallurgically, this results in the presence of rhombohedral Co₇W₆ BCC (α₁+α₂) and BCC Mo and BCC W phases. Another example may further increase the tungsten to up to 60% by replacing the molybdenum with tungsten. This would further increase radiopacity, and result in the same phases present as described relative to an alloy including up to 50 weight percent tungsten. Of course, such a modified alloy could also be relative to (or derived from) L-605, as both would include at least 20% Co, 20% Cr, and up to 60% W.

Examples 65-68 below are based on the nominal compositions of the ASTM F90 L-605 Alloy of Table 1 and the ASTM F562 MP-35N Alloy of Table 3 in which at least the nickel, and in some examples, some of the cobalt has been replaced on an weight substitution basis with a refractory metal, zirconium. Example 69 further replaces the tungsten of L-605 or the molybdenum of MP-35N with zirconium so that the alloy is essentially a ternary Co-Cr-Zr alloy. Trace elements such as beryllium, boron, carbon, iron, manganese, phosphorus, silicon, sulfur, and titanium are not listed.

### Reference Example 65 - ASTM F90 L-605 Alloy With Weight Substitution of Nickel With Zirconium

| Element | Weight Percent |
|---|---|
| Cobalt | 55 |
| Chromium | 20 |
| Tungsten | 15 |
| Zirconium | 10 |

Substituting Zr for Ni on a weight percentage basis increases the calculated radiopacity from 3.6 to 4.1 barnes/cc. From a metallurgical perspective, the Co-Cr, CoW, and Cr-W will fall in the same regions as for L-605. Otherwise the alloy will contain a mixture of HCP εCo and γ(CoZr), FCC αZrCr₂ and BCC Cr, FCC W₂Zr, and HCP (αZr) phases.

### Reference Example 66 - ASTM F90 L-605 Alloy With Weight Substitution of Nickel and a Portion of the Cobalt With Zirconium

| Element | Weight Percent |
|---|---|
| Cobalt | at least 20 |
| Chromium | 20 |
| Tungsten | 15 |
| Zirconium | up to 45 |

As with the above alloys of Ag, Au, Hf, Mo, Nb, Re, and Ta, here it is assumed that Cr and W are not changed, thus Co-Cr, Co-W, and Cr-W are the same as for L-605. From a metallurgical phase diagram perspective, Zr may be included up to about 45 weight percent. At 45 weight percent Zr, this gives a radiopacity of 5.5 barnes/cc and metallurgically would result in a mix of cubic ζ(CoZr) and BCT η(CoZr), FCC αZrCr₂ and HCP αZr, and FCC W₂Zr and HCP αZr phases.

### Reference Example 67 - ASTM F562 MP-35N Alloy With Weight Substitution of Nickel With Zirconium

| Element | Weight Percent |
|---|---|
| Cobalt | 35 |
| Chromium | 20 |
| Molybdenum | 10 |
| Zirconium | 35 |

Utilizing a straight substitution of zirconium for nickel on a weight percentage basis increases the radiopacity from 3.4 barnes/cc to 4.8 barnes/cc. From a metallurgical perspective, Co-Cr, Co-Mo, and Cr-Mo fall in the same regions as MP-35N. Other phases will include FCC ε(CoZr) and cubic ζ(CoZr), FCC αZrCr₂ and HCP αZr, and FCC Mo₂Zr, and BCC Mo phases.

### Reference Example 68 - ASTM F562 MP-35N Alloy With Weight Substitution of Nickel and a Portion of the Cobalt With Zirconium

| Element | Weight Percent |
|---|---|
| Cobalt | at least 20 |
| Chromium | 20 |
| Molybdenum | 10 |
| Zirconium | up to 50 |

While avoiding the Co-Cr σ intermetallic phase Zr may be included up to 50 weight percent. This increases radiopacity to 5.3 barnes/cc. Metallurgically, this results in the presence of cubic ζ(CoZr) and BCT η(CoZr), FCC αZrCr₂ and HCP αZr, FCC Mo₂Zr, and HCP αZr phases.

### Reference Example 69 - ASTM F90 L-605 Alloy or ASTM F562 MP-35N Alloy With Weight Substitution of Nickel and Tungsten or Molybdenum With Zirconium

| Element | Weight Percent |
|---|---|
| Cobalt | at least 20 |
| Chromium | 20 |
| Zirconium | up to 60 |

When considering a simpler mixture, the alloy may be considered without either Mo or W, i.e., consisting of Zr-Co-Cr only. In this instance, the Zr may range up to about 60 weight percent to avoid the Co-Cr σ intermetallic phase. At 60 weight percent Zr, calculated radiopacity would be 5.0 barnes/cc. Co-Zr falls close to the BCT η(CoZr) phase, but would also likely include some cubic ζ(CoZr) phase as well. The Cr-Zr falls in the FCC αZrCr₂ and HCP αZr phase regime.

Disclosed alloys of Examples 25-69, even those with relatively complex phase diagrams, are expected to provide viable alloys for use in stent fabrication. Enhancing the radiopacity of the alloy while remaining viable from metallurgical and engineering perspectives allows the fabrication of improved medical devices, particularly stents where greater radiopacity is beneficial.

Reference Examples 70-73 describe particular ternary cobalt-chromium-iridium alloys that are somewhat similar to Examples 7-8, but which are essentially ternary alloys and do not include elements other than cobalt, chromium, and iridium. Such an embodiment may include chromium at a level from about 10 to about 25 atomic percent, more particularly from about 15 to about 25 (e.g., about 20 atomic percent) atomic percent to impart the desired tightly adhering Cr₂O₃ oxide layer that provides excellent corrosion resistance. The cobalt and iridium levels may be varied in virtually any ratio, depending on desired radiopacity, degree of ferromagnetism, and mechanical strength because cobalt and iridium are mutually soluble in one another and therefore do not form undesirable intermetallic phases. In order to avoid ferromagnetism at room temperature, the ratio of iridium to cobalt may be selected so as to be greater than about 1:1 on an atomic percentage basis. The result is a radiopaque, corrosion resistant, and relatively inexpensive ternary alloy system for stents. Iridium is attractive because it has slightly better radiopacity than platinum and has a cost approximately half that of platinum and similar to that of palladium. Similar ternary alloys including only cobalt, chromium, and a metal other than iridium (e.g., Pt, Pd, Ru, Rh, or Os) could similarly be provided. Such ternary alloys may include similar atomic fractions as those described above and in Examples 70-73 but in which the Iridium is substituted with Pt, Pd, Ru, Rh, or Os. Similar ternary alloys could also be formed from Co, Cr, and a refractory metal or precious metal (i.e., silver or gold).

According to a disclosed embodiment, such a ternary alloy is not based on any existing commercially available alloy composition such as L605 or MP-35N so that it does not contain extra elements such as tungsten, molybdenum, iron, manganese, etc. and thus minimizes the complexity in terms of intermetallics, precipitate formation, etc.

### Reference Example 70 - Ternary Cobalt-Chromium-Iridium Alloy

| Element | Atomic Percent |
|---|---|
| Cobalt | 35 |
| Chromium | 20 |
| Iridium | 45 |

### Example 71- Ternary Cobalt-Chromium-Iridium Alloy

| Element | Atomic Percent |
|---|---|
| Cobalt | 30 |
| Chromium | 20 |
| Iridium | 50 |

### Reference Example 72 - Ternary Cobalt-Chromium-Iridium Alloy

| Element | Atomic Percent |
|---|---|
| Cobalt | 25 |
| Chromium | 20 |
| Iridium | 55 |

### Reference Example 73 - Ternary Cobalt-Chromium-Iridium Alloy

| Element | Atomic Percent |
|---|---|
| Cobalt | 20 |
| Chromium | 20 |
| Iridium | 60 |

As mentioned previously, a platinum group metal, refractory metal, or precious metal may replace elements in addition to the nickel of an existing commercially available alloy such as L605, MP-35N, Elgiloy, or Phynox. For example, in L605, the nickel is included as an austenitic stabilizer. Nickel and the platinum group elements palladium and platinum are group 10 elements. Thus, platinum and palladium may also be expected to act as austentic stabilizers in cobalt. In addition, cobalt and each of the three group 10 elements (nickel, palladium, and platinum) are mutually dissolvable in each other. Platinum and/or palladium may be substituted for nickel in L605 to improve radiopacity. Where insufficient radiopacity is provided under atomic percentage substitution as described in Examples 1 and 3, at least some of the cobalt of an alloy such as L605 may also be replaced with the platinum group metal to further increase the radiopacity. Reference Examples 74-75 and Reference Examples 76-77 describe such examples that are similar to 1 and 3, respectively, but also replace at least a portion of the cobalt to further increase radiopacity. Similar reference examples could be provided based on alloys other than L605 (e.g., MP-35N, Elgiloy, or Phynox)

### Reference Example 74 - ASTM F90 L-605 Alloy With Atomic Substitution of Nickel and a Portion of the Cobalt With Platinum

| Element | Atomic Percent |
|---|---|
| Cobalt | 48.9 |
| Chromium | 24.4 |
| Tungsten | 5.2 |
| Platinum | 15.8 |
| Manganese (maximum) | 2.3 |
| Iron (maximum) | 3.4 |

### Reference Example 75 - ASTM F90 L-605 Alloy With Atomic Substitution of Nickel and a Portion of the Cobalt With Platinum

| Element | Atomic Percent |
|---|---|
| Cobalt | 43.9 |
| Chromium | 24.4 |
| Tungsten | 5.2 |
| Platinum | 20.8 |
| Manganese (maximum) | 2.3 |
| Iron (maximum) | 3.4 |

### Reference Example 76 - ASTM F90 L-605 Alloy With Atomic Substitution of Nickel and a Portion of the Cobalt With Palladium

| Element | Atomic Percent |
|---|---|
| Cobalt | 48.9 |
| Chromium | 24.4 |
| Tungsten | 5.2 |
| Palladium | 15.8 |
| Manganese (maximum) | 2.3 |
| Iron (maximum) | 3.4 |

### Reference Example 77 - ASTM F90 L-605 Alloy With Atomic Substitution of Nickel and a Portion of the Cobalt With Palladium

| Element | Atomic Percent |
|---|---|
| Cobalt | 43.9 |
| Chromium | 24.4 |
| Tungsten | 5.2 |
| Palladium | 20.8 |
| Manganese (maximum) | 2.3 |
| Iron (maximum) | 3.4 |

As mentioned previously, a platinum group metal, refractory metal, or precious metal may replace elements in addition to the nickel of an existing commercially available alloy such as L605, MP-35N, Elgiloy, or Phynox. Several of the above disclosed embodiments include substitution of a portion of the cobalt in addition to substitution of the nickel. Another disclosed embodiment may specifically substitute the refractory metal (e.g., molybdenum or tungsten) present within the commercial alloy, replacing this refractory metal with a platinum group metal (particularly preferred substituting platinum group metals include Pt, Pd, Ir, and Rh. Another disclosed embodiment may substitute with another refractory metal (e.g., substitute Mo with Re). Another disclosed embodiment may substitute the refractory metal with iron, which if done, is may be present in a small amount, as described herein. Many of these disclosed alloy embodiments may also replace the nickel, and optionally a portion of the cobalt, chromium, or even both, with a platinum group metal, particularly preferred of which would be Pt.

For example, a base disclosed research alloy which still includes the refractory metal (e.g., molybdenum) is herein designated MP1 has a composition as shown below in Table 5. MP1 may be considered to be a more heavily substituted variant of MP-35N, as the amount of chromium is lower (13.5% vs. 20% by weight) and the amount of molybdenum is lower (6.7% vs. 10%). Some of the chromium and molybdenum, as well as all the nickel and a portion of the cobalt has been replaced with platinum.

**Table 5 - MP1 Alloy**

| Element | Weight Percent | Atomic Percent |
|---|---|---|
| Cobalt | 23.3 | 39.0 |
| Chromium | 13.5 | 25.6 |
| Molybdenum | 6.7 | 6.9 |
| Platinum | 56.5 | 28.5 |

In evaluating various of the earlier disclosed cobalt-chromium-platinum group alloys described above in conjunction with Reference Examples 1-24, it was found that as ingots were prepared by melting and then worked to evaluate their workability, a greater understanding of ambient temperature workability (i.e., cold workability) of these alloys was gained. Ordinarily significant reduction in workability when platinum is added to 316L stainless steel at greater than 33 weight percent is expected. However, this reduction in workability did not occur in at least some of the present alloy systems described herein. Rather, some of the initial platinum substituted alloy melts with greater than 33 weight percent platinum were workable as evidenced by being rolled at up to 50% deformation.

One of the most promising precious metal substitution alloys melted and processed was MP1, described in Table 5 above. Other alloys with greater percentages of Pd that were investigated with off stoichiometric alloy compositions showed good workability, but resulted in lower than desired densities. Pd (density of 12.02 g/cm³) is significantly less dense than Pt (density of 21.45 g/cm³). Thus, substitution with platinum is the substitution according to the present invention. Alloy MP1 maintains a high density (12.8 g/cm³) and a relative radiopacity of 9.8 barnes/cm³. While this alloy showed good workability after casting, microstructural changes resulted in somewhat lower workability after heat treatment.

In order to further improve the workability of the MP1 alloy, additional quaternary (and in some cases pentenary) alloys were investigated by replacing the molybdenum in the MP1 alloy, as described above. Example 87 is a ternary alloy in which the molybdenum is replaced with additional cobalt. Examples 78-89 in Table 6 below show the resulting compositions. Refractory metal elements such as molybdenum and tungsten are typically used for strengthening, but can also reduce workability. The specific substitutions shown in Examples 78-89 of Table 6 are based on phase diagram analysis. Examples 81, 85, and 87 were actually formed by melting, and then worked to evaluate their workability characteristics. Of these three, Examples 85 and 87 showed the best cast workability in rolling, with Example 85 being slightly better.

**Table 6 - Variants of MP1 With Substitution of Molybdenum**

| Alloy | Pt wt % at % | Co wt % at % | Cr wt % at % | Fe wt % at % | Ir wt % at % | Pd wt % at % | Re wt % at % | Rh wt % at % | Density (g/cm³) | RR (barnes/cm³) |
|---|---|---|---|---|---|---|---|---|---|---|
| Ex. 78 | 56.5 (28.7) | 23.3 (39.2) | 13.5 (25.8) | | | 6.7 (6.2) | | | 13.0 | 10.1 |
| Ex. 79 | 52 (24.9) | 23.3 (36.9) | 18 (32.3) | | | 6.7 (5.9) | | | 12.3 | 9.2 |
| Ex. 80 | 54 (26.5) | 23.3 (37.9) | 16 (29.5) | | | 6.7 (6.0) | | | 12.6 | 9.6 |
| Ex. 81 | 54.2 (27.3) | 23.3 (38.9) | 13.5 (25.5) | | | 9.0 (8.3) | | | 12.8 | 10.1 |
| Ex. 82 | 43.7 (20.6) | 23.3 (36.4) | 18 (31.9) | | | 10 (8.6) | 5 (2.5) | | 12.1 | 9.1 |
| Ex. 83 | 56.5 (29.5) | 23.3 (40.3) | 13.5 (26.5) | | | | 6.7 (3.7) | | 13.4 | 10.0 |
| Ex. 84 | 52.9 (26.2) | 23.1 (37.9) | 16 (29.8) | | | 5 (4.5) | 3 (1.6) | | 12.7 | 9.0 |
| Ex. 85 | 56.5 (27.2) | 23.3 (37.1) | 13.5 (24.4) | 6.7 (11.3) | | | | | 12.5 | 8.9 |
| Ex. 86 | 56.5 (28.7) | 23.3 (39.2) | 13.5 (25.7) | | | | | 6.7 (6.4) | 13.0 | 10.1 |
| Ex. 87 | 56.5 (27.4) | 30.0 (48.1) | 13.5 (24.5) | | | | | | 12.7 | 9.0 |
| Ex. 88 | 56.5 (29.6) | 23.3 (40.4) | 13.5 (26.5) | | 6.7 (3.6) | | | | 13.5 | 10.2 |
| Ex. 89 | 40 (19.0) | 23.3 (36.6) | 13.5 (24.0) | | | 13.2 (11.5) | | 10 (9.0) | 12.1 | 10.0 |

Generally, Examples 78-89 are nickel-free, molybdenum-free cobalt-based alloys comprising from about 18 weight percent to about 39 weight percent cobalt, from about 10 weight percent to about 25 weight percent chromium, from about 40 weight percent to about 65 weight percent platinum, and from about 5 weight percent to about 25 weight percent of one or more other platinum group metals, refractory metals, or combinations thereof. In another embodiment, and as will be apparent from Examples 78-89, the cobalt-based alloy may comprise from about 18 weight percent to about 30 weight percent cobalt, from about 10 weight percent to about 20 weight percent chromium, and from about 45 weight percent to about 60 weight percent platinum. In another embodiment, and as will be apparent from Examples 78-89, the cobalt-based alloy may comprise from about 18 weight percent to about 25 weight percent cobalt, from about 10 weight percent to about 15 weight percent chromium, and from about 50 weight percent to about 60 weight percent platinum. Example 85 substitutes with iron rather than a platinum group metal or refractory metal, while Example 87 substitutes the molybdenum with additional cobalt to result in a ternary alloy. In an embodiment, the alloy may be substantially free or completely free of other refractory metals, other than molybdenum (e.g., tungsten).

Examples 78-81, 83, and 86-87 are quaternary alloys comprising cobalt, chromium, platinum, and one other platinum group metal or refractory metal. Examples 82, 84, and 89 are pentenary alloy comprising cobalt, chromium, platinum, and one or more other platinum group metals, refractory metals, or combinations thereof (i.e., two additional elements are selected from platinum group metals, refractory metals, or combinations).

Example 85 can more generally be described as a molybdenum-free cobalt-based alloy comprising from about 18 weight percent to about 39 weight percent cobalt, from about 10 weight percent to about 25 weight percent chromium, from about 40 weight percent to about 65 weight percent platinum, and from about 5 weight percent to about 10 weight percent iron. In one embodiment, iron is present at less than 10 weight percent by weight, or less than 8 weight percent.

With respect to an embodiment including iron, (e.g., Example 85), it is noted that the Fe-Cr phase diagram does include an intermetallic σ phase between about 43 and about 48 weight percent chromium (and 52-57 weight percent iron). Although this phase is only formed at elevated temperatures (e.g., from about 440°C to about 830°C), such a phase may possibly remain even after such an alloy is cooled to room temperatures. As such, in some embodiments, it may be desired to maintain the ratio of iron to chromium outside of the intermetallic σ phase. This phase occurs just below a 1:1 weight ratio of Cr:Fe. In one embodiment, the weight fraction of chromium is greater than the weight fraction of iron so as to clearly avoid this phase. Because Chromium is typically present at a significantly higher level (e.g., 10% plus, 15% plus, etc.) than the iron, this will typically not be an issue. For example, Example 85 includes a Cr:Fe weight ratio of about 2:1. Even where the weight ratio of Cr to Fe is lower (e.g., 1:1, or even 0.8:1) such alloys may still be suitable if no intermetallic is present at the conditions of use (e.g., where the intermetallic phase does not persist at ambient temperature (e.g., near 20°C)).

Example 87 can more generally be described as a molybdenum-free cobalt-based alloy comprising from about 18 weight percent to about 39 weight percent cobalt, from about 10 weight percent to about 25 weight percent chromium, from about 40 weight percent to about 65 weight percent platinum. The alloy is ternary, the molybdenum of MP1 having been replaced with additional cobalt.

Although the compositions shown in Table 6 is based on MP1 (which is based on MP-35N), it will be understood that alloy variants of L-605, Elgiloy, and Phynox can also be provided by substituting for nickel, for refractory elements (e.g., tungsten in L-605, molybdenum for Elgiloy and Phynox), as well as substituting for some cobalt, some chromium, and iron or other low fraction elements. When these alloys are reduced to quaternary or pentenary form with relative radiopacities greater than 5.5 barnes/cm³, the possible elemental compositions may be similar to those shown above in Table 6, as well as various examples discussed previously. As such, these variants of L-605, Elgiloy, and Phynox are also within the scope of the present disclosure.

Although described for use in manufacturing stents, it will be understood that any of the disclosed alloys may also be used in the manufacture of the following disclosed medical devices: guide wires, guide wire tip coils, balloon markers, or other structures associated with catheter use, and other implantable structures in which improved radiopacity would be desirable.

The present invention can be embodied in other specific forms without departing from its spirit or essential characteristics. Thus, the described implementations are to be considered in all respects only as illustrative and not restrictive. The scope of the invention is, therefore, indicated by the appended claims rather than by the foregoing description. All changes that come within the meaning and range of equivalency of the claims are to be embraced within their scope.

## Claims

1. A radiopaque stent, comprising:
a cylindrical main body comprising a cobalt-based alloy including cobalt, chromium and one or more platinum group metals selected from the group consisting of platinum, palladium, rhodium, iridium, osmium, ruthenium, silver and gold the cobalt-based alloy being substantially free of nickel and comprising no more than about 20 percent by weight iron;
wherein the cobalt-based alloy comprises from about 18 weight percent to about 39 weight percent cobalt, from about 10 weight percent to about 25 weight percent chromium, from about 40 weight percent to about 65 weight percent platinum, and wherein the alloy is substantially free of molybdenum.

2. The radiopaque stent of claim 1, wherein the cobalt-based alloy further comprises from about 5 weight percent to about 10 weight percent iron.

3. The radiopaque stent of claim 2, wherein the cobalt-based alloy is a quaternary alloy consisting essentially of cobalt, chromium, platinum, and iron;
or
wherein the cobalt-based alloy comprises from about 18 weight percent to about 30 weight percent cobalt, from about 10 weight percent to about 20 weight percent chromium, from about 45 weight percent to about 60 weight percent platinum;
or
wherein the cobalt-based alloy comprises from about 18 weight percent to about 25 weight percent cobalt, from about 10 weight percent to about 15 weight percent chromium, from about 50 weight percent to about 60 weight percent platinum.

4. The radiopaque stent of claim 1, wherein the cobalt-based alloy is substantially free of tungsten;
or
wherein the cobalt-based alloy is entirely free of nickel.

5. The radiopaque stent of claim 1, wherein the cobalt-based alloy is a ternary alloy consisting essentially of cobalt, chromium, and platinum;
or
wherein the cobalt-based alloy is formed by providing each constituent metal in solid form, powder form, or both, and melting the constituent metals so as to form the cobalt-based alloy.

6. The radiopaque stent of claim 1, wherein melting the constituent metals is achieved by arc melting, electron beam melting, induction melting, radiant heat melting, microwave melting, or combinations thereof;
or
wherein the cobalt-based alloy is formed by providing each constituent metal in powder form, mixing the powders together, and compacting and sintering the mixture of constituent metals in powder form so as to form the cobalt-based alloy.

7. The radiopaque stent of claim 1, wherein the cobalt-based alloy comprises from about 5 weight percent to about 25 weight percent of one or more other platinum group metals, refractory metals, or combinations thereof.

8. The radiopaque stent of claim 7, wherein the cobalt-based alloy is a quaternary alloy comprising cobalt, chromium, platinum, and one other platinum group metal or refractory metal.

9. The radiopaque stent of claim 7, wherein the cobalt-based alloy is a pentenary alloy comprising cobalt, chromium, platinum, and two additional metals selected from the group consisting of platinum group metals other than platinum, refractory metals, and combinations thereof.

## Patentansprüche

1. Radiodichter Stent, umfassend:
einen zylindrischen Hauptkörper, umfassend eine Legierung auf Kobaltbasis, die Kobalt, Chrom und ein oder mehrere Platingruppenmetalle, ausgewählt aus der Gruppe, bestehend aus Platin, Palladium, Rhodium, Iridium, Osmium, Ruthenium, Silber und Gold, beinhaltet, wobei die Legierung auf Kobaltbasis im Wesentlichen frei von Nickel ist und höchstens etwa 20 Gewichtsprozent Eisen umfasst;
wobei die Legierung auf Kobaltbasis von etwa 18 Gewichtsprozent bis etwa 39 Gewichtsprozent Kobalt, von etwa 10 Gewichtsprozent bis etwa 25 Gewichtsprozent Chrom, von etwa 40 Gewichtsprozent bis etwa 65 Gewichtsprozent Platin umfasst und wobei die Legierung im Wesentlichen frei von Molybdän ist.

2. Radiodichter Stent nach Anspruch 1, wobei die Legierung auf Kobaltbasis ferner von etwa 5 Gewichtsprozent bis etwa 10 Gewichtsprozent Eisen umfasst.

3. Radiodichter Stent nach Anspruch 2, wobei die Legierung auf Kobaltbasis eine quaternäre Legierung ist, die im Wesentlichen aus Kobalt, Chrom, Platin und Eisen besteht;
oder
wobei die Legierung auf Kobaltbasis von etwa 18 Gewichtsprozent bis etwa 30 Gewichtsprozent Kobalt, von etwa 10 Gewichtsprozent bis etwa 20 Gewichtsprozent Chrom, von etwa 45 Gewichtsprozent bis etwa 60 Gewichtsprozent Platin umfasst;
oder
wobei die Legierung auf Kobaltbasis von etwa 18 Gewichtsprozent bis etwa 25 Gewichtsprozent Kobalt, von etwa 10 Gewichtsprozent bis etwa 15 Gewichtsprozent Chrom, von etwa 50 Gewichtsprozent bis etwa 60 Gewichtsprozent Platin umfasst.

4. Radiodichter Stent nach Anspruch 1, wobei die Legierung auf Kobaltbasis im Wesentlichen frei von Wolfram ist;
oder
wobei die Legierung auf Kobaltbasis völlig frei von Nickel ist.

5. Radiodichter Stent nach Anspruch 1, wobei die Legierung auf Kobaltbasis eine ternäre Legierung ist, die im Wesentlichen aus Kobalt, Chrom und Platin besteht;
oder
wobei die Legierung auf Kobaltbasis durch Bereitstellen jedes metallischen Bestandteils in fester Form, Pulverform oder beidem und Schmelzen der metallischen Bestandteile zum Bilden der Legierung auf Kobaltbasis gebildet ist.

6. Radiodichter Stent nach Anspruch 1, wobei das Schmelzen der metallischen Bestandteile durch Lichtbogenschmelzen, Elektronenstrahlschmelzen, Induktionsschmelzen, Strahlungshitzeschmelzen, Mikrowellenschmelzen oder Kombinationen davon erreicht ist;
oder
wobei die Legierung auf Kobaltbasis durch Bereitstellen jedes metallischen Bestandteils in Pulverform, Zusammenmischen der Pulver und Verdichten und Sintern des Gemisches aus metallischen Bestandteilen in Pulverform gebildet ist, um die Legierung auf Kobaltbasis zu bilden.

7. Radiodichter Stent nach Anspruch 1, wobei die Legierung auf Kobaltbasis von etwa 5 Gewichtsprozent bis etwa 25 Gewichtsprozent eines oder mehrerer anderer Platingruppenmetalle, Refraktärmetalle oder Kombinationen davon umfasst.

8. Radiodichter Stent nach Anspruch 7, wobei die Legierung auf Kobaltbasis eine quaternäre Legierung ist, die Kobalt, Chrom, Platin und ein anderes Platingruppenmetall oder Refraktärmetall umfasst.

9. Radiodichter Stent nach Anspruch 7, wobei die Legierung auf Kobaltbasis eine quinäre Legierung ist, die Kobalt, Chrom, Platin und zwei zusätzliche Metalle, ausgewählt aus der Gruppe, bestehend aus anderen Platingruppenmetallen als Platin, Refraktärmetallen und Kombinationen davon, umfasst.

## Revendications

1. Stent radio-opaque, comprenant :
un corps principal cylindrique comprenant un alliage à base de cobalt contenant du cobalt, du chrome et un ou plusieurs métaux du groupe du platine sélectionnés dans le groupe composé du platine, du palladium, du rhodium, de l'iridium, de l'osmium, du ruthénium, de l'argent et de l'or, l'alliage à base de cobalt étant sensiblement exempt de nickel et ne contenant pas plus d'environ 20 % en poids de fer ;
dans lequel l'alliage à base de cobalt contient environ 18 % à environ 39 % en poids de cobalt, environ 10 % en poids à environ 25 % en poids de chrome, environ 40 % en poids à environ 65 % en poids de platine et dans lequel l'alliage est sensiblement exempt de molybdène.

2. Stent radio-opaque selon la revendication 1, dans lequel l'alliage à base de cobalt comprend en outre environ 5 % en poids à environ 10 % en poids de fer.

3. Stent radio-opaque selon la revendication 2, dans lequel l'alliage à base de cobalt est un alliage quaternaire composé essentiellement de cobalt, de chrome, de platine et de fer ;
ou
dans lequel l'alliage à base de cobalt contient environ 18 % en poids à environ 30 % en poids de cobalt, environ 10 % en poids à environ 20 % en poids de chrome, environ 45 % à environ 60 % en poids de platine ;
ou
dans lequel l'alliage à base de cobalt contient environ 18 % en poids à environ 25 % en poids de cobalt, environ 10 % en poids à environ 15 % en poids de chrome, environ 50 % à environ 60 % en poids de platine.

4. Stent radio-opaque selon la revendication 1, dans lequel l'alliage à base de cobalt est sensiblement exempt de tungstène ;
ou
dans lequel l'alliage à base de cobalt est entièrement exempt de nickel.

5. Stent radio-opaque selon la revendication 1, dans lequel l'alliage à base de cobalt est un alliage ternaire composé essentiellement de cobalt, de chrome et de platine ;
ou
dans lequel l'alliage à base de cobalt est formé en prévoyant chaque métal constitutif sous forme solide, sous forme de poudre ou les deux et en faisant fondre les métaux constitutifs pour former l'alliage à base de cobalt.

6. Stent radio-opaque selon la revendication 1, dans lequel la fusion des métaux constitutifs est réalisée par fusion à l'arc, fusion au faisceau électronique, fusion par induction, fusion par chaleur radiante, fusion par micro-ondes ou leurs combinaisons ;
ou
dans lequel l'alliage à base de cobalt est formé en prévoyant chaque métal constitutif sous forme de poudre, en mélangeant les poudres ensemble et en compactant et en frittant le mélange de métaux constitutifs sous forme de poudres de manière à former l'alliage à base de cobalt.

7. Stent radio-opaque selon la revendication 1, dans lequel l'alliage à base de cobalt contient environ 5 % en poids environ 25 % en poids d'un ou plusieurs autres métaux du groupe du platine, métaux réfractaires ou leurs combinaisons.

8. Stent radio-opaque selon la revendication 7, dans lequel l'alliage à base de cobalt est un alliage quaternaire contenant du cobalt, du chrome, du platine et un autre métal du groupe du platine ou métal réfractaire.

9. Stent radio-opaque selon la revendication 7, dans lequel l'alliage à base de cobalt est un alliage penténaire contenant du cobalt, du chrome, du platine et deux métaux supplémentaires sélectionnés dans le groupe composé des métaux du groupe du platine autres que le platine, les métaux réfractaires et leurs combinaisons.
